(19) Europäisches Patentamt / European Patent Office / Office européen des brevets

(11) EP 2 594 566 A1

(12) EUROPEAN PATENT APPLICATION
published in accordance with Art. 153(4) EPC

(43) Date of publication:
**22.05.2013 Bulletin 2013/21**

(21) Application number: **11806893.1**

(22) Date of filing: **15.07.2011**

(51) Int Cl.:
***C07D 401/12*** (2006.01) ***A61K 31/4709*** (2006.01)
***A61P 35/00*** (2006.01) ***A61P 43/00*** (2006.01)
***C07D 401/14*** (2006.01) ***C07D 413/14*** (2006.01)
***C07D 417/14*** (2006.01) ***C07D 495/04*** (2006.01)

(86) International application number:
**PCT/JP2011/066185**

(87) International publication number:
**WO 2012/008563 (19.01.2012 Gazette 2012/03)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **16.07.2010 JP 2010161272**

(71) Applicant: **Kyowa Hakko Kirin Co., Ltd.**
**Tokyo 100-8185 (JP)**

(72) Inventors:
- **FURUTA, Takayuki (JP)**
- **KUBO, Kazuo (JP)**
- **ATSUMI, Toshiyuki (JP)**
- **YAMANOUCHI, Maasa (JP)**

(74) Representative: **Harding, Charles Thomas**
**D Young & Co LLP**
**120 Holborn**
**London**
**EC1N 2DY (GB)**

# (54) NITROGENATED AROMATIC HETEROCYCLIC RING DERIVATIVE

(57) The present invention provides a nitrogen-containing aromatic heterocyclic derivative represented by formula (I):

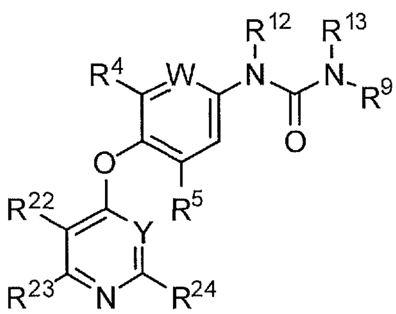

(I)

[wherein W represents $CR^3$ (wherein $R^3$ represents a hydrogen atom or the like) or the like, $R^4$ represents a hydrogen atom or the like, $R^5$ represents a hydrogen atom or the like, $R^9$ represents substituted imidazolyl, $R^{12}$ and $R^{13}$ may be the same or different, each represent a hydrogen atom or the like, $R^{22}$ and $R^{23}$ may be the same or different, each represent a hydrogen atom or the like, $R^{24}$ represents a hydrogen atom or the like, Y represents C-$R^{25}$ (wherein $R^{25}$ represents a hydrogen atom or the like) or the like] or a pharmaceutically acceptable salt thereof and a FGFR inhibitor comprising, as an active ingredient, the nitrogen-containing aromatic heterocyclic derivative or the pharmaceutically acceptable salt thereof, or the like.

EP 2 594 566 A1

## Description

TECHNICAL FIELD

The present invention relates to a nitrogen-containing aromatic heterocyclic derivative or a pharmaceutically acceptable salt thereof having an activity inhibiting a fibroblast growth factor receptor (FGFR), or the like.

BACKGROUND ART

The FGFR is a receptor-type protein tyrosine kinase and is activated by binding with its ligand, a fibroblast growth factor (FGF). The FGF signal has many physiological functions such as angiogenesis and wound repair, and the FGFR is expressed in cells of many tissues to regulate a cellular phenotype, such as proliferation, differentiation, or survival. Accordingly, it is believed that aberrant FGF signal is involved in tumor growth by stimulating the proliferation and survival of a cancer cell. In mammals, the FGFR consists of four subtypes: FGFR1, FGFR2, FGFR3, and FGFR4 (Nature Reviews Cancer, vol. 10, pp. 116-129, 2010).

Recently, it has been reported that abnormality in regulation of the FGF signal is involved in cancer. For example, there are reports that amplification of FGFR1 is observed in approximately 10% of estrogen receptor positive breast cancer patients (Cancer Res., vol. 57, pp. 4360-4367, 1997), that FGFR2 is overexpressed in approximately 50% of poorly differentiated malignant gastric cancer patients and is a poor-prognosis factor (Clin. Cancer Res., vol. 13, pp. 3051-3057, 2007), that somatic mutation of FGFR2 occurs in 12% of endometrial cancer patients and cell lines carrying the mutation are highly sensitive to FGFR inhibitors (Proc. Natl. Acad. Sci. USA, vol. 105, pp. 8713-8717, 2008), that activating mutation of FGFR3 occurs in approximately 50% of bladder cancer patients (Nature Genetics, vol. 23, pp. 18-19, 1999), and that the FGFR4 Arg388 allele correlates with prognosis and metastasis of cancer patients (Cancer Res., vol. 62, pp. 840-847, 2002).

Furthermore, overexpression and activating mutation of FGF or FGFR are reported in various cancers (for example, pituitary tumor, myeloproliferative disorder, renal cancer, bladder cancer, colon cancer, head and neck cancer, skin cancer, non-Hodgkin's lymphoma, brain tumor, breast cancer, ovarian cancer, multiple myeloma, osteosarcoma, and the like) (Expert Opinion on Therapeutic Targets, vol. 6, pp. 469-482, 2002; and Nature, vol. 411, pp. 355-365, 2001).

FGFR inhibitors, AZD2171 (Clin. Cancer Res., vol. 13, pp. 3051-3057, 2007) and 4-[3-chloro-4-(cyclopropylaminocarbonyl)aminophenoxy]-7-methoxy-6-quinolinecarboxamide (see Patent Literature 8), show antitumor activities in human gastric cancer tumor models.

Furthermore, it has been reported that inhibition of the FGFR suppresses the downstream signal, resulting in poly(ADP-ribose) polymerase (PARP) cleavage (Journal of Hepatology, vol. 50, pp. 118-127, 2009). In addition, it has been reported that the cleavage of PARP induces apoptosis (The Journal of Biological Chemistry, vol. 273, No. 50, pp. 33533-33539, 1998). The induction of apoptosis is believed to lead to therapy of cancer, virus infection, an autoimmune disease such as systemic lupus erythematosus, rheumatoid arthritis, hepatitis, and a renal disease, or the like (Protein, Nucleic acid and Enzyme, vol. 38, No. 2, pp. 102-108, 1993; and Protein, Nucleic acid and Enzyme, vol. 44, No. 10, pp. 1477-1486, 1999).

From the above, it is believed that an FGFR inhibitor is useful as a therapeutic and/or preventive agent for various cancers (for example, gastric cancer, endometrial cancer, pituitary tumor, myeloproliferative disorder, renal cancer, bladder cancer, colon cancer, head and neck cancer, skin cancer, non-Hodgkin's lymphoma, brain tumor, breast cancer, ovarian cancer, multiple myeloma, osteosarcoma, and the like), virus infection, an autoimmune disease such as systemic lupus erythematosus, rheumatoid arthritis, hepatitis, a renal disease, and the like.

Incidentally, as a kinase inhibitor, quinoline derivatives having aromatic heterocyclic urea structures are known (see Patent Literature 1 to 9).

Furthermore, as VEGF receptor tyrosine kinase inhibitors, quinoline derivatives having imidazolyl urea structures are known (see Non Patent Literature 1).

PRIOR ART

PATENT LITERATURE

Patent Literature 1: WO 97/17329
Patent Literature 2: WO 00/43366
Patent Literature 3: WO 02/088110
Patent Literature 4: U.S. Patent No. 7329670

Patent Literature 5: WO 99/32106
Patent Literature 6: WO 99/32455
Patent Literature 7 : WO 02/32872
Patent Literature 8: WO 2008/026748
Patent Literature 9: WO 2007/061127

NON PATENT LITERATURE

Non Patent Literature 1: American Association for Cancer Research (AACR), July 11 to 14, 2003 "#36 Azolyl Quinoline-Urea Derivatives: A Novel Series of Potent Orally Active VEGF Receptor Tyrosine Kinase Inhibitors"

SUMMARY OF INVENTION

PROBLEMS TO BE SOLVED BY INVENTION

An object of the present invention is to provide a nitrogen-containing aromatic heterocyclic derivative or a pharmaceutically acceptable salt thereof having an activity inhibiting FGFR, or the like.

MEANS FOR SOLVING THE PROBLEM

The present invention relates to the following (1) to (32).

(1) A nitrogen-containing aromatic heterocyclic derivative represented by Formula (I):

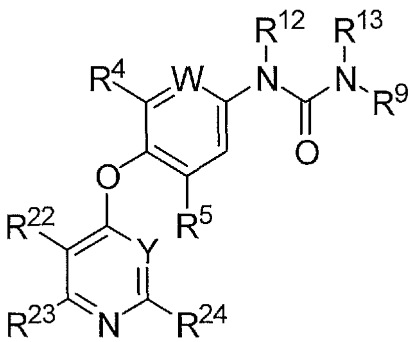

(I)

[wherein W represents $CR^3$ (wherein $R^3$ represents a hydrogen atom, lower alkyl, lower alkoxy, or halogen) or a nitrogen atom;
$R^4$ represents a hydrogen atom, lower alkyl, or halogen;
$R^5$ represents a hydrogen atom or lower alkyl;
$R^9$ represents substituted imidazolyl;
$R^{12}$ and $R^{13}$ may be the same or different and each represent a hydrogen atom or lower alkyl;
$R^{22}$ and $R^{23}$ may be the same or different and each represent a hydrogen atom, optionally substituted aryl, an optionally substituted aromatic heterocyclic group, or $-C(=O)NR^{26}R^{27}$ (wherein $R^{26}$ and $R^{27}$ may be the same or different and each represent a hydrogen atom, lower alkyl, or an aromatic heterocyclic group), or $R^{22}$ and $R^{23}$ are combined together with the respective adjacent carbon atoms to form an optionally substituted benzene ring or an optionally substituted thiophene ring;
$R^{24}$ represents a hydrogen atom or lower alkyl; and
Y represents $C-R^{25}$ (wherein $R^{25}$ represents a hydrogen atom or lower alkyl) or a nitrogen atom] or a pharmaceutically acceptable salt thereof.

(2) The nitrogen-containing aromatic heterocyclic derivative or the pharmaceutically acceptable salt thereof according to (1), wherein in Formula (I),

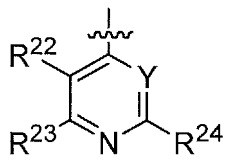

is

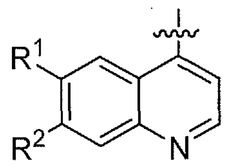

[wherein $R^1$ and $R^2$ may be the same or different and each represent a hydrogen atom, halogen, hydroxy, optionally substituted lower alkylsulfonyloxy, optionally substituted lower alkoxy, $-NR^{31}R^{32}$ (wherein $R^{31}$ and $R^{32}$ may be the same or different and each represent a hydrogen atom or optionally substituted lower alkyl), $-C(=O)NR^{33}R^{34}$ (wherein $R^{33}$ and $R^{34}$ may be the same or different and each represent a hydrogen atom or optionally substituted lower alkyl), or optionally substituted lower alkoxycarbonyl].

(3) The nitrogen-containing aromatic heterocyclic derivative or the pharmaceutically acceptable salt thereof according to (2), wherein $R^1$ and $R^2$ may be the same or different and each are a hydrogen atom, hydroxy, optionally substituted lower alkylsulfonyloxy, optionally substituted lower alkoxy, or $-NR^{31}R^{32}$ (wherein $R^{31}$ and $R^{32}$ may be the same or different and each represent a hydrogen atom or optionally substituted lower alkyl).

(4) The nitrogen-containing aromatic heterocyclic derivative or the pharmaceutically acceptable salt thereof according to (2), wherein $R^1$ and $R^2$ may be the same or different and each are optionally substituted lower alkoxy.

(5) The nitrogen-containing aromatic heterocyclic derivative or the pharmaceutically acceptable salt thereof according to (1), wherein in Formula (I),

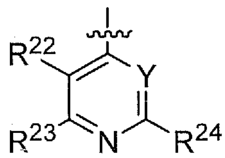

is

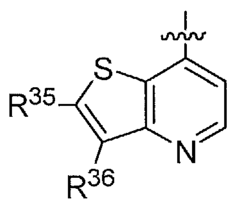

[wherein $R^{35}$ and $R^{36}$ may be the same or different and each represent a hydrogen atom, halogen, optionally substituted aryl, an optionally substituted aromatic heterocyclic group, or $-(C=O)R^{37}$ (wherein $R^{37}$ represents an optionally substituted aliphatic heterocyclic group)].

(6) The nitrogen-containing aromatic heterocyclic derivative or the pharmaceutically acceptable salt thereof according

to (5), wherein $R^{35}$ is optionally substituted aryl or an optionally substituted aromatic heterocyclic group, and $R^{36}$ is a hydrogen atom.

(7) The nitrogen-containing aromatic heterocyclic derivative or the pharmaceutically acceptable salt thereof according to any one of (1) to (6), wherein W is $CR^{3A}$ (wherein $R^{3A}$ represents a hydrogen atom, lower alkoxy, or halogen) or a nitrogen atom.

(8) The nitrogen-containing aromatic heterocyclic derivative or the pharmaceutically acceptable salt thereof according to any one of (1) to (6), wherein W is $CR^{3A}$ (wherein $R^{3A}$ has the same meaning as defined above).

(9) The nitrogen-containing aromatic heterocyclic derivative or the pharmaceutically acceptable salt thereof according to any one of (1) to (8), wherein $R^4$ is a hydrogen atom or halogen.

(10) The nitrogen-containing aromatic heterocyclic derivative or the pharmaceutically acceptable salt thereof according to any one of (1) to (9), wherein $R^5$ is a hydrogen atom.

(11) The nitrogen-containing aromatic heterocyclic derivative or the pharmaceutically acceptable salt thereof according to any one of (1) to (10), wherein $R^9$ is

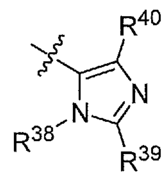

[wherein $R^{38}$ represents a hydrogen atom or lower alkyl; $R^{39}$ represents lower alkyl; and $R^{40}$ represents a hydrogen atom, optionally substituted lower alkyl, optionally substituted lower alkenyl, optionally substituted lower alkynyl, optionally substituted aryl, an optionally substituted aromatic heterocyclic group, or $-C(=O)NR^{41}R^{42}$ (wherein $R^{41}$ and $R^{42}$ may be the same or different and each represent a hydrogen atom or an aromatic heterocyclic group)].

(12) The nitrogen-containing aromatic heterocyclic derivative or the pharmaceutically acceptable salt thereof according to Claim 11, wherein $R^{40}$ is optionally substituted aryl or an optionally substituted aromatic heterocyclic group.

(13) The nitrogen-containing aromatic heterocyclic derivative or the pharmaceutically acceptable salt thereof according to any one of (1) to (10), wherein $R^9$ is

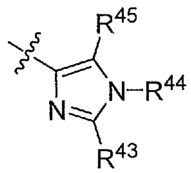

(wherein $R^{43}$ represents a hydrogen atom or lower alkyl, $R^{44}$ represents lower alkyl, and $R^{45}$ represents a hydrogen atom or lower alkyl).

(14) The nitrogen-containing aromatic heterocyclic derivative or the pharmaceutically acceptable salt thereof according to (13), wherein $R^{43}$ is a hydrogen atom, $R^{44}$ is lower alkyl, and $R^{45}$ is a hydrogen atom.

(15) The nitrogen-containing aromatic heterocyclic derivative or the pharmaceutically acceptable salt thereof according to any one of (1) to (14), wherein $R^{12}$ and $R^{13}$ are hydrogen atoms.

(16) A pharmaceutical composition comprising, as an active ingredient, the nitrogen-containing aromatic heterocyclic derivative or the pharmaceutically acceptable salt thereof according to any one of (1) to (15).

(17) A fibroblast growth factor receptor inhibitor comprising, as an active ingredient, the nitrogen-containing aromatic heterocyclic derivative or the pharmaceutically acceptable salt thereof according to any one of (1) to (15).

(18) A therapeutic and/or preventive agent for a disease in which a fibroblast growth factor receptor is involved, comprising, as an active ingredient, the nitrogen-containing aromatic heterocyclic derivative or the pharmaceutically acceptable salt thereof according to any one of (1) to (15).

(19) An antitumor agent comprising, as an active ingredient, the nitrogen-containing aromatic heterocyclic derivative or the pharmaceutically acceptable salt thereof according to any one of (1) to (15).

(20) A therapeutic and/or preventive agent for gastric cancer comprising, as an active ingredient, the nitrogen-containing aromatic heterocyclic derivative or the pharmaceutically acceptable salt thereof according to any one of (1) to (15).
(21) A method for inhibiting a fibroblast growth factor receptor, comprising a step of administering an effective amount of the nitrogen-containing aromatic heterocyclic derivative or the pharmaceutically acceptable salt thereof according to any one of (1) to (15).
(22) A method for treating and/or preventing a disease in which a fibroblast growth factor receptor is involved, comprising a step of administering an effective amount of the nitrogen-containing aromatic heterocyclic derivative or the pharmaceutically acceptable salt thereof according to any one of (1) to (15).
(23) A method for treating and/or preventing cancer, comprising a step of administering an effective amount of the nitrogen-containing aromatic heterocyclic derivative or the pharmaceutically acceptable salt thereof according to any one of (1) to (15).
(24) A method for treating and/or preventing gastric cancer, comprising a step of administering an effective amount of the nitrogen-containing aromatic heterocyclic derivative or the pharmaceutically acceptable salt thereof according to any one of (1) to (15).
(25) Use of the nitrogen-containing aromatic heterocyclic derivative or the pharmaceutically acceptable salt thereof according to any one of (1) to (15) for the manufacture of a fibroblast growth factor receptor inhibitor.
(26) Use of the nitrogen-containing aromatic heterocyclic derivative or the pharmaceutically acceptable salt thereof according to any one of (1) to (15) for the manufacture of a therapeutic and/or preventive agent for a disease in which a fibroblast growth factor receptor is involved.
(27) Use of the nitrogen-containing aromatic heterocyclic derivative or the pharmaceutically acceptable salt thereof according to any one of (1) to (15) for the manufacture of an antitumor agent.
(28) Use of the nitrogen-containing aromatic heterocyclic derivative or the pharmaceutically acceptable salt thereof according to any one of (1) to (15) for the manufacture of a therapeutic and/or preventive agent for gastric cancer.
(29) The nitrogen-containing aromatic heterocyclic derivative or the pharmaceutically acceptable salt thereof according to any one of (1) to (15) for use in inhibiting a fibroblast growth factor receptor.
(30) The nitrogen-containing aromatic heterocyclic derivative or the pharmaceutically acceptable salt thereof according to any one of (1) to (15) for use in treating and/or preventing a disease in which a fibroblast growth factor receptor is involved.
(31) The nitrogen-containing aromatic heterocyclic derivative or the pharmaceutically acceptable salt thereof according to any one of (1) to (15) for use in treating and/or preventing cancer.
(32) The nitrogen-containing aromatic heterocyclic derivative or the pharmaceutically acceptable salt thereof according to any one of (1) to (15) for use in treating and/or preventing gastric cancer.

## EFFECTS OF INVENTION

The present invention provides, for example, a nitrogen-containing aromatic heterocyclic derivative or a pharmaceutically acceptable salt thereof having an activity inhibiting FGFR and being effective for treating and/or preventing a disease in which the FGFR is involved (for example, gastric cancer, endometrial cancer, pituitary tumor, myeloproliferative disorder, renal cancer, bladder cancer, colon cancer, head and neck cancer, skin cancer, non-Hodgkin's lymphoma, brain tumor, breast cancer, ovarian cancer, multiple myeloma, osteosarcoma, virus infection, an autoimmune disease such as systemic lupus erythematosus, rheumatoid arthritis, hepatitis, a renal disease, or the like), or the like.

## MODE FOR CARRYING OUT THE INVENTION

Hereinafter, compounds represented by Formula (I) are referred to as Compound (I), and the same applies to compounds represented by other formula numbers.
In the definition of each group of Formula (I),
examples of the lower alkyl and the lower alkyl moiety of the lower alkoxy, the lower alkoxycarbonyl, and the lower alkylsulfonyloxy include linear or branched alkyl having 1 to 10 carbon atoms. More specific examples thereof include methyl, ethyl, propyl, isopropyl, butyl, isobutyl, sec-butyl, tert-butyl, pentyl, isopentyl, neopentyl, hexyl, heptyl, octyl, nonyl, decyl, and the like.
Examples of the lower alkenyl include linear or branched alkenyl having 2 to 10 carbon atoms. More specific examples thereof include vinyl, allyl, 1-propenyl, 1-butenyl, 3-butenyl, 2-pentenyl, 4-pentenyl, 2-hexenyl, 5-hexenyl, 2-decenyl, 9-decenyl, and the like. Preferred are linear or branched alkenyl having two or three carbon atoms, and the like. More preferred are vinyl, and the like.
Examples of the lower alkynyl include linear or branched alkynyl having 2 to 10 carbon atoms. More specific examples thereof include ethynyl, 1-propynyl, 2-propynyl, 3-butynyl, 4-pentynyl, 5-hexynyl, 9-decynyl, and the like.

Preferred are linear or branched alkynyl having two or three carbon atoms, and the like. More preferred are ethynyl, and the like.
Examples of the aryl include monocyclic aryl and condensed aryl formed by condensation of two or more rings. More specific examples thereof include aryl having 6 to 14 ring carbon atoms such as phenyl, naphthyl, azulenyl, and anthryl. Preferred are aryl having 6 to 10 carbon atoms, and the like. More preferred are phenyl, and the like.

Examples of the aromatic heterocyclic group include a 5- or 6-membered monocyclic aromatic heterocyclic group containing at least one atom selected from a nitrogen atom, an oxygen atom, and a sulfur atom; a bicyclic or tricyclic condensed aromatic heterocyclic group formed by condensation of 3- to 8-membered rings and containing at least one hetero atom selected from a nitrogen atom, an oxygen atom, and a sulfur atom, and the like. More specific examples thereof include furyl, thienyl, pyrrolyl, imidazolyl, pyrazolyl, oxazolyl, isoxazolyl, oxadiazolyl, thiazolyl, isothiazolyl, thiadiazolyl, triazolyl, tetrazolyl, pyridyl, pyridazinyl, pyrimidinyl, pyrazinyl, triazinyl, benzofuranyl, benzothiophenyl, benzoxazolyl, benzothiazolyl, isoindolyl, indolyl, indazolyl, benzimidazolyl, benzotriazolyl, oxazolopyrimidinyl, thiazolopyrimidinyl, pyrrolopyridinyl, pyrrolopyrimidinyl, imidazopyridinyl, purinyl, quinolinyl, isoquinolinyl, cinnolinyl, phthalazinyl, quinazolinyl, quinoxalinyl, naphthyridinyl, and the like.

Examples of the aliphatic heterocyclic group include a 5- or 6-membered monocyclic aliphatic heterocyclic group containing at least one atom selected from a nitrogen atom, an oxygen atom, and a sulfur atom; a bicyclic or tricyclic condensed aliphatic heterocyclic group formed by condensation of 3- to 8-membered rings and containing at least one atom selected from a nitrogen atom, an oxygen atom, and a sulfur atom, and the like. More specific examples thereof include aziridinyl, azetidinyl, pyrrolidinyl, piperidino, piperidinyl, azepanyl, 1,2,5,6-tetrahydropyridyl, imidazolidinyl, pyrazolidinyl, piperazinyl, homopiperazinyl, pyrazolyl, oxiranyl, tetrahydrofuranyl, tetrahydro-2H-pyranyl, 5,6-dihydro-2H-pyranyl, oxazolidinyl, morpholino, morpholinyl, thioxazolidinyl, thiomorpholinyl, 2H-oxazolyl, 2H-thioxazolyl, dihydroindolyl, dihydroisoindolyl, dihydrobenzofuranyl, benzimidazolidinyl, dihydrobenzoxazolyl, dihydrobenzothioxazolyl, benzodioxolinyl, tetrahydroquinolyl, tetrahydroisoquinolyl, dihydro-2H-chromanyl, dihydro-1H-chromanyl, dihydro-2H-thiochromanyl, dihydro-1H-thiochromanyl, tetrahydroquinoxalinyl, tetrahydroquinazolinyl, dihydrobenzodioxanyl, and the like.

Halogen means each atom of fluorine, chlorine, bromine, and iodine.

(i) The substituents of the optionally substituted lower alkyl, the optionally substituted lower alkenyl, the optionally substituted lower alkynyl, the optionally substituted lower alkoxy, the optionally substituted lower alkoxycarbonyl, and the optionally substituted lower alkylsulfonyloxy may be the same or different, and, for example, the number of the substituents may be from 1 to 3. Examples of the substituents include substituents selected from the group consisting of
halogen, hydroxy, sulfanyl, nitro, cyano, carboxy, carbamoyl, $C_{3-8}$ cycloalkyl, $C_{6-14}$ aryl, an optionally substituted aliphatic heterocyclic group (examples of the substituents of the optionally substituted aliphatic heterocyclic group include $C_{1-10}$ alkyl), aliphatic heterocyclic oxy, an aromatic heterocyclic group,
$C_{1-10}$ alkoxy, $C_{3-8}$ cycloalkoxy, $C_{6-14}$ aryloxy, $C_{7-16}$ aralkyloxy, $C_{2-11}$ alkanoyloxy, $C_{7-15}$ aroyloxy,
$C_{1-10}$ alkylsulfanyl, $-NR^XR^Y$ (wherein $R^X$ and $R^Y$ may be the same or different and each represent a hydrogen atom, $C_{1-10}$ alkyl, $C_{3-8}$ cycloalkyl, $C_{6-14}$ aryl, an aromatic heterocyclic group, $C_{7-16}$ aralkyl, $C_{2-11}$ alkanoyl, $C_{7-15}$ aroyl, $C_{1-10}$ alkoxycarbonyl, or $C_{7-16}$ aralkyloxycarbonyl),
$C_{2-11}$ alkanoyl, $C_{7-15}$ aroyl, $C_{1-10}$ alkoxycarbonyl, $C_{6-14}$ aryloxycarbonyl, $C_{1-10}$ alkylcarbamoyl, di-$C_{1-10}$ alkylcarbamoyl, and tri-$C_{1-10}$ alkylsilyloxy.
(ii) The substituents of the substituted imidazolyl, the optionally substituted aryl, the optionally substituted aromatic heterocyclic group, the optionally substituted benzene ring, and the optionally substituted thiophene ring may be the same or different, and, for example, the number of the substituents may be from 1 to 3. Examples of the substituents include substituents selected from the group consisting of
halogen, hydroxy, sulfanyl, nitro, cyano, carboxy, carbamoyl, optionally substituted $C_{1-10}$ alkyl (examples of the substituents of the optionally substituted $C_{1-10}$ alkyl include the groups exemplified in the substituent (i) of the optionally substituted lower alkyl, and the like), optionally substituted $C_{2-10}$ alkenyl (examples of the substituents of the optionally substituted $C_{2-10}$ alkenyl include the groups exemplified in the substituent (i) of the optionally substituted lower alkyl, and the like), optionally substituted $C_{2-10}$ alkynyl (examples of the substituents of the optionally substituted $C_{2-10}$ alkynyl include the groups exemplified in the substituent (i) of the optionally substituted lower alkyl, and the like), optionally substituted $C_{3-8}$ cycloalkyl (examples of the substituents (a) of the optionally substituted $C_{3-8}$ cycloalkyl include $C_{1-10}$ alkyl, trifluoromethyl, and the like in addition to the groups exemplified in the substituent (i) of the optionally substituted lower alkyl, and the like), optionally substituted $C_{6-14}$ aryl (examples of the substituents of the optionally substituted $C_{6-14}$ aryl include the substituent (a) of the optionally substituted $C_{3-8}$ cycloalkyl, and the like),
an aliphatic heterocyclic group,
an optionally substituted aromatic heterocyclic group (examples of the substituents of the optionally substituted

aromatic heterocyclic group include the substituent (a) of the optionally substituted $C_{3-8}$ cycloalkyl, and the like), optionally substituted $C_{1-10}$ alkoxy (examples of the substituents of the optionally substituted $C_{1-10}$ alkoxy include the substituent (a) of the optionally substituted $C_{3-8}$ cycloalkyl, and the like),

$C_{3-8}$ cycloalkoxy, $C_{6-14}$ aryloxy, $C_{7-16}$ aralkyloxy, $C_{2-11}$ alkanoyloxy, $C_{7-15}$ aroyloxy,

$C_{1-10}$ alkylsulfanyl, $C_{1-10}$ alkylsulfonyl, $-NR^{X1}R^{Y1}$ (wherein $R^{X1}$ and $R^{Y1}$ may be the same or different and each represent a hydrogen atom, $C_{1-10}$ alkyl, $C_{3-8}$ cycloalkyl, $C_{6-14}$ aryl, an aromatic heterocyclic group, $C_{7-16}$ aralkyl, $C_{2-11}$ alkanoyl, $C_{7-15}$ aroyl, $C_{1-10}$ alkoxycarbonyl, or $C_{7-16}$ aralkyloxycarbonyl),

$C_{2-11}$ alkanoyl, $C_{7-15}$ aroyl, $C_{1-10}$ alkoxycarbonyl, $C_{6-14}$ aryloxycarbonyl, optionally substituted $C_{1-10}$ alkylcarbamoyl (examples of the substituents of the optionally substituted $C_{1-10}$ alkylcarbamoyl include the groups exemplified in the substituent (i) of the optionally substituted lower alkyl, and the like), di-$C_{1-10}$ alkylcarbamoyl, and $-(C{=}O)NHR^{x2}$ (wherein $R^{x2}$ represents $C_{6-14}$ aryl or an aromatic heterocyclic group).

(iii) The substituents of the optionally substituted aliphatic heterocyclic group may be the same or different, and, for example, the number of the substituents may be from 1 to 3. Examples of the substituents of the optionally substituted aliphatic heterocyclic group include substituents selected from the group consisting of oxo, halogen, hydroxy, sulfanyl, nitro, cyano, carboxy, carbamoyl, $C_{1-10}$ alkyl, trifluoromethyl, $C_{3-8}$ cycloalkyl,

$C_{6-14}$ aryl, an aliphatic heterocyclic group, an aromatic heterocyclic group,

$C_{1-10}$ alkoxy, $C_{3-8}$ cycloalkoxy, $C_{6-14}$ aryloxy, $C_{7-16}$ aralkyloxy, $C_{2-11}$ alkanoyloxy, $C_{7-15}$ aroyloxy,

$C_{1-10}$ alkylsulfanyl, $-NR^{X3}R^{Y3}$ (wherein $R^{X3}$ and $R^{Y3}$ nay be the same or different and each represent a hydrogen atom, $C_{1-10}$ alkyl, $C_{3-8}$ cycloalkyl, $C_{6-14}$ aryl, an aromatic heterocyclic group, $C_{7-16}$ aralkyl, $C_{2-11}$ alkanoyl, $C_{7-15}$ aroyl, $C_{1-10}$ alkoxycarbonyl, or $C_{7-16}$ aralkyloxycarbonyl),

$C_{2-11}$ alkanoyl, $C_{7-15}$ aroyl, $C_{1-10}$ alkoxycarbonyl, $C_{6-14}$ aryloxycarbonyl, $C_{1-10}$ alkylcarbamoyl, and di-$C_{1-10}$ alkylcarbamoyl.

Examples of the $C_{1-10}$ alkyl and the $C_{1-10}$ alkyl moieties of the $C_{1-10}$ alkoxy, the $C_{2-11}$ alkanoyloxy, the $C_{1-10}$ alkylsulfanyl, the $C_{1-10}$ alkylsulfonyl, the $C_{2-11}$ alkanoyl, the $C_{1-10}$ alkoxycarbonyl, the $C_{1-10}$ alkylcarbamoyl, the di-$C_{1-10}$ alkylcarbamoyl, and the tri-$C_{1-10}$ alkylsilyloxy include groups exemplified in the examples of the lower alkyl. The two $C_{1-10}$ alkyl groups of the di-$C_{1-10}$ alkylcarbamoyl may be the same or different. The three $C_{1-10}$ alkyl groups of the tri-$C_{1-10}$ alkylsilyloxy may be the same or different.

Examples of the $C_{3-8}$ cycloalkyl and the cycloalkyl moiety of the $C_{3-8}$ cycloalkoxy include cycloalkyl having 3 to 8 carbon atoms, and more specific examples thereof include cyclopropyl, cyclobutyl, cyclopentyl, cyclohexyl, cycloheptyl, cyclooctyl, and the like.

Examples of the $C_{6-14}$ aryl and the aryl moieties of the $C_{6-14}$ aryloxy, the $C_{7-15}$ aroyl, the $C_{7-15}$ aroyloxy, and the $C_{6-14}$ aryloxycarbonyl include groups, for example, exemplified in the examples of the aryl. More specific examples thereof include phenyl, naphthyl, azulenyl, anthryl, and the like.

Examples of the aryl moieties of the $C_{7-16}$ aralkyloxy, the $C_{7-16}$ aralkyl, and the $C_{7-16}$ aralkyloxycarbonyl include groups exemplified in the examples of the $C_{6-14}$ aryl; and examples of the alkylene moieties include $C_{1-10}$ alkylene, and more specific examples thereof include groups obtained by removing one hydrogen atom from each group exemplified in the examples of the lower alkyl.

The aliphatic heterocyclic group and the aliphatic heterocyclic group moiety of the aliphatic heterocyclic oxy have the same meaning as the above-mentioned aliphatic heterocyclic group.

The aromatic heterocyclic group has the same meaning as the above-mentioned aromatic heterocyclic group. The halogen has the same meaning as the above-mentioned halogen.

Examples of the pharmaceutically acceptable salt of Compound (I) include a pharmaceutically acceptable acid addition salt, a metal salt, an ammonium salt, an organic amine addition salt, an amino acid addition salt, and the like. Examples of the pharmaceutically acceptable acid addition salt of Compound (I) include an inorganic acid salt such as a hydrochloride, a hydrobromide, a nitrate, a sulfate, or a phosphate; an organic acid salt such as an acetate, an oxalate, a maleate, a fumarate, a citrate, a benzoate, or a methanesulfonate, and the like. Examples of the pharmaceutically acceptable metal salt include an alkali metal salt such as a sodium salt or a potassium salt; an alkaline earth metal salt such as a magnesium salt or a calcium salt; an aluminum salt; a zinc salt, and the like. Examples of the pharmaceutically acceptable ammonium salt include salts such as an ammonium and a tetramethylammonium. Examples of the pharmaceutically acceptable organic amine addition salt include addition salts such as morpholine and piperidine. Examples of the pharmaceutically acceptable amino acid addition salt include addition salts such as lysine, glycine, phenylalanine, aspartic acid, and glutamic acid.

A production method of Compound (I) will be described.

In the production method described below, in cases where a defined group changes under the conditions of the production method or is not suitable for carrying out the production method, the target compound can be produced by using a method for introducing and removing a protecting group, and the like commonly used in synthetic organic chemistry, (for example, the method described in Protective Groups in Organic Synthesis, Third edition, T.W. Greene, John Wiley

& Sons Inc. (1999), or the like). Furthermore, the order of reaction steps such as the introduction of a substituent may be changed, if necessary.

Intermediate (V-1) of Compound (1-2) among Compound (I) wherein W is $CR^3$ (wherein $R^3$ has the same meaning as defined above), can be produced by, for example, the following Production method 1.

Production method 1

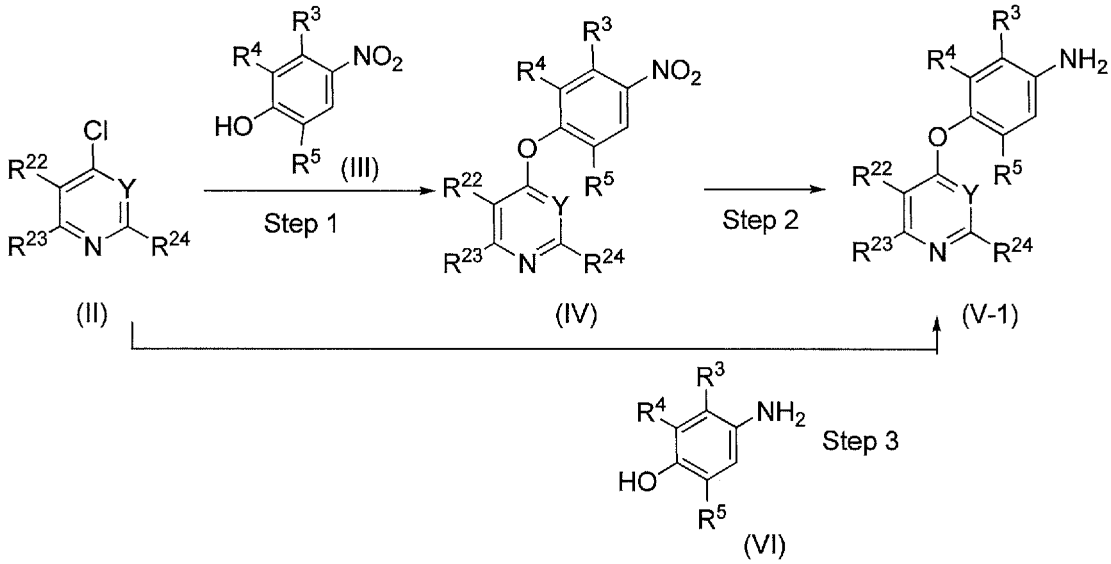


(wherein $R^3$, $R^4$, $R^5$, $R^{22}$, $R^{23}$, $R^{24}$, and Y have the same respective meanings as defined above).

Step 1

Compound (IV) can be produced by reacting Compound (II) with preferably 1 to 100 equivalents of Compound (III) in a solvent in the presence of preferably 1 to 100 equivalents of a base, if necessary, at a temperature between -10°C and the boiling point of the solvent used for 5 minutes to 72 hours.

Examples of the base include potassium carbonate, sodium carbonate, diisopropylethylamine, 2,6-lutidine, and the like. These bases may be used alone or as a mixture thereof.

Examples of the solvent include N-methylpyrrolidone (NMP), N,N-dimethylformamide (DMF), N,N-dimethylacetamide (DMA), dimethylsulfoxide (DMSO), chlorobenzene, and the like. These solvents may be used alone or as a mixture thereof.

Compound (III) can be obtained, for example, as a commercial product.

Compound (II) can be obtained, for example, as a commercial product or can be produced by a method described in, for example, WO 97/017329, JP-A-11-158149, WO 99/24440, WO 00/41698, WO 00/42012, WO 00/43366, WO 01/47890, WO 02/088110, WO 03/000194, WO 03/000660, WO 03/033472, WO 2004/018430, WO 2004/039782, WO 2006/010264, US 5773449A, J. Med. Chem., 2005, 48(5), 1359-1366, J. Med. Chem., 2006, 49(8), 2440-2455, or the like, or according to such a method.

The Compound (IV) can also be produced by a method described in, for example, WO 03/000194, WO 2006010264, or the like, or according to such a method.

Step 2

Compound (V-1) can be produced by treating Compound (IV) in a solvent in the presence of 0.1 to 100 wt%, preferably 0.1 to 50 wt%, of a metal catalyst with respect to Compound (IV) at a temperature between -10°C and the boiling point of the solvent used, preferably at a temperature between 20°C and the boiling point of the solvent used, under a normal or increased pressure in a hydrogen atmosphere or in the presence of one equivalent to large excess of a suitable hydrogen source for 5 minutes to 72 hours.

At this step, 0.01 to 30 equivalents of an acid can be added to proceed the reaction.

Examples of the solvent include ethanol, methanol, ethyl acetate, THF, diethyl ether, water, acetonitrile, and the like. These solvents may be used alone or as a mixture.
Examples of the metal catalyst include palladium on carbon, palladium on alumina, palladium hydroxide, palladium hydroxide on carbon, palladium chloride, Wilkinson's catalyst, and the like.
Examples of the hydrogen source include formic acid, ammonium formate, sodium formate, and the like.
Examples of the acid include hydrochloric acid, sulfuric acid, nitric acid, acetic acid, trifluoroacetic acid, trifluoromethanesulfonic acid, and the like.
Compound (V-1) can also be produced by treating Compound (IV) with preferably 1 to 10 equivalents of a reducing agent with respect to Compound (IV) in a solvent at a temperature between -10°C and the boiling point of the solvent used, preferably at a temperature between 20°C and the boiling point of the solvent used, for 5 minutes to 72 hours.
Examples of the solvent include ethanol, methanol, ethyl acetate, THF, diethyl ether, water, acetonitrile, and the like. These solvents may be used alone or as a mixture.
Examples of the reducing agent include sodium dithionite, tin, tin dichloride, iron, zinc, nickel borohydride, lithium aluminum hydride, and the like.

Step 3

Compound (V-1) can also be produced by reacting Compound (II) with preferably 1 to 10 equivalents of Compound (VI) in a solvent used in the presence of preferably 1 to 10 equivalents of a base with respect to Compound (II) at a temperature between -10°C and the boiling point of the solvent used for 5 minutes to 72 hours. At this step, 0.01 to 30 equivalents of a suitable additive may be added thereto.
Examples of the solvent include DMF, DMA, DMSO, chloroform, acetone, methyl ethyl ketone, water, and the like. These solvents may be used alone or as a mixture.
Examples of the base include sodium hydroxide, sodium hydride, and the like.
Examples of the additive include tetrabutylammonium bromide, and the like.
Compound (VI) can be obtained, for example, as a commercial product.

Production method 2

Intermediate (V-2) of Compound (I-3) among Compound (I), wherein W is a nitrogen atom, can be produced by, for example, a method described in US 20080312232 (in particular, scheme 10), WO 2005121125, or the like, or according to such a method.
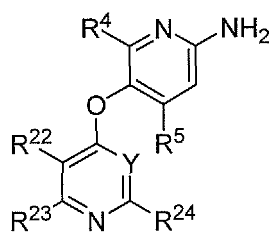

(V-2)

(wherein $R^4$, $R^5$, $R^{22}$, $R^{23}$, $R^{24}$, and Y have the same respective meanings as defined above).

Production method 3

Compound (I) can be produced using Compound (V-1) obtained by Production method 1 or Compound (V-2) obtained by Production method 2 (collectively referred to as Compound (V)) by the production method of the urea moiety described in WO 00/43366 or WO 02/088110, or according to such a method.
Specifically, Compound (I) can be produced by, for example, the following production method or according to the method.
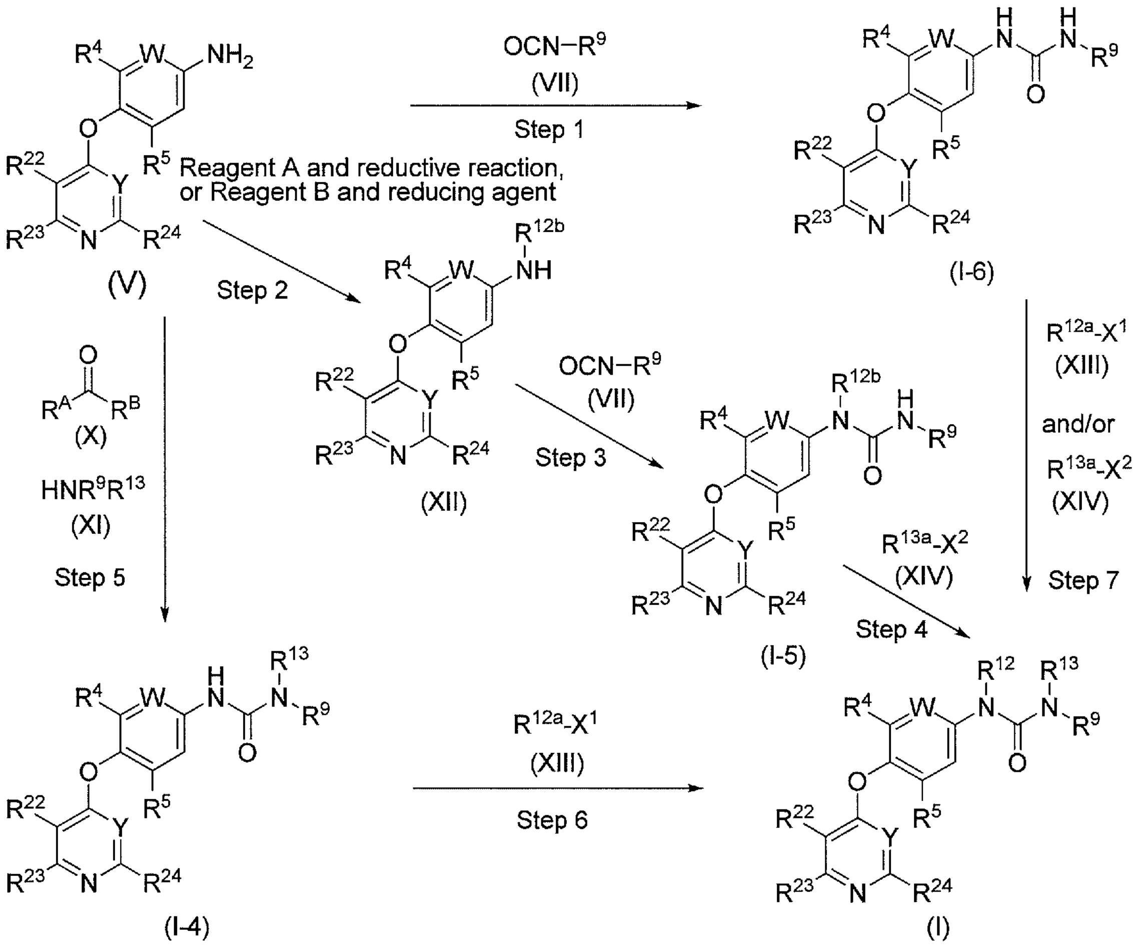


Reagent A: $R^{14}COX$ (VIII) or $(R^{14}CO)_2O$ (IX-1)

Reagent B: 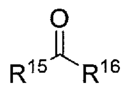 (IX-2)

(wherein $X^1$ and $X^2$ may be the same or different and each represent a chlorine atom, bromine atom, iodine atom, p-toluenesulfonyloxy, methanesulfonyloxy, or trifluoromethanesulfonyloxy; X represents halogen; $R^4$, $R^5$, $R^9$, $R^{12}$, $R^{13}$, $R^{22}$, $R^{23}$, $R^{24}$, Y, and W have the same respective meanings as defined above; $R^{12a}$ and $R^{13a}$ may be the same or different and each represent the lower alkyl in the definition of $R^{12}$ and $R^{13}$; $R^{12b}$ represents a primary or secondary lower alkyl in the definition of $R^{12}$; $R^{14}$ represents a group obtained from lower alkyl having methylene at the end in the definition of $R^{12}$ by removing the methylene or represents a hydrogen atom; $R^{15}$ and $R^{16}$ are combined together with the adjacent carbon atom to obtain group formed by removing a hydrogen atom from the carbon atom binding to the nitrogen atom of $R^{12b}$ ; and $R^A$ and $R^B$ may be the same or different and each represent a chlorine atom, bromine atom, or trichloromethyloxy).

Step 1

Compound (I-6) can be produced by reacting Compound (V) with preferably 1 to 10 equivalents of Compound (VII) in a solvent at a temperature between -10°C and the boiling point of the solvent used for 5 minutes to 72 hours.

Examples of the solvent include dichloromethane, chloroform, 1,2-dichloroethane, toluene, ethyl acetate, acetonitrile, diethyl ether, THF, 1,2-dimethoxyethane (DME), dioxane, NMP, DMF, DMA, pyridine, and the like. These solvents may be used alone or as a mixture thereof.

Compound (VII) can be obtained, for example, as a commercial product or can be produced by a known method (for example, "4th edition, Jikken Kagaku Koza (Experimental Chemistry Course) 20 Yuki Gosei (Organic Synthesis) II", p. 473, Maruzen, (2001)) or according to the method or can be produced by the following production method A. Production method A

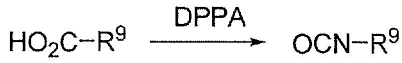


(XV) (VII)

(wherein $R^9$ has the same meaning as defined above).

Compound (VII) can be produced by, for example, the method described in Synth. Commun., 1993, 23(3), 335 or according to the method.

Specifically, Compound (VII) can be produced by reacting Compound (XV) with preferably 1 to 10 equivalents of diphenylphosphoryl azide (DPPA) in a solvent at a temperature between -10°C and the boiling point of the solvent used for 5 minutes to 72 hours.

Examples of the solvent include toluene, ethyl acetate, acetonitrile, diethyl ether, THF, DME, dioxane, NMP, DMF, DMA, pyridine, and the like. These solvents may be used alone or as a mixture thereof.

Pyrazolecarboxylic acid (XV-1) among Compound (XV) can be produced by a method described in, for example, 1) "Jikken Kagaku Koza (Experimental Chemistry Course), 5th edition, vol. 16, Chapter 1, Synthesis of carboxylic acid (pp. 1-34), Maruzen; 2) J. Heterocyclic Chem., 26(5), 1389-1392 (1989), or the like. Imidazolecarboxylic acid (XV-2) can be produced by a method described in, for example, 1) J. Org. Chem., 1977, 42(7), 1153-1159; 2) Heterocycles, 2006, 68 (6), 1149-1162; 3) Tetrahedron Lett., 35(11), 1635-1638 (1994), or the like. Isoxazolecarboxylic acid (XV-3) can be produced by, for example, a method described in US 20100048545, or the like.

Step 2

Compound (XII) can be produced by reacting Compound (V) with preferably 1 to 10 equivalents of Compound (VIII) or preferably 1 to 10 equivalents of Compound (IX-1) in a solvent in the presence of preferably 1 to 5 equivalents of a base at a temperature between -10°C and the boiling point of the solvent used, preferably a temperature between 0°C and the boiling point of the solvent used, for 5 minutes to 72 hours and then treating with preferably 1 to 10 equivalents of a reducing agent at a temperature between - 10°C and the boiling point of the solvent used, preferably a temperature between 0°C and the boiling point of the solvent used, for 5 minutes to 72 hours.

Examples of the solvent include NMP, DMF, DMA, benzene, toluene, hexane, ethyl acetate, THF, dioxane, diethyl ether, dichloromethane, chloroform, and the like. These solvents may be used alone or as a mixture thereof.

Examples of the base include dimethylaminopyridine (DMAP), 1,4-diazabicyclo[2.2.2]octane (DABCO), triethylamine, diisopropylethylamine, and the like.

Examples of the reducing agent include lithium aluminum hydride, diisobutylaluminum hydride (DIBAL), lithium borohydride, and the like.

Compound (VIII) and Compound (IX-1) can be obtained, for example, as commercial products.

Compound (XII) can also be produced by reacting Compound (V) with preferably 1 to 10 equivalents of a reducing agent and preferably 1 to 10 equivalents of Compound (IX-2) in a solvent, in the presence of preferably 1 to 10 equivalents of an additive, if necessary, for 5 minutes to 72 hours.

Examples of the solvent include methanol, ethanol, dichloromethane, chloroform, 1,2-dichloroethane, toluene, ethyl acetate, acetonitrile, diethyl ether, THF, DME, dioxane, DMF, DMA, NMP, water, and the like. These solvents may be used alone or as a mixture thereof.

Examples of the additive include hydrochloric acid, sulfuric acid, formic acid, acetic acid, trifluoroacetic acid, p-toluenesulfonic acid, tetraisopropoxy titanium, and the like.

Examples of the reducing agent include sodium cyanoborohydride, sodium triacetoxyborohydride, pyridine borane, 2-picoline borane, and the like.

Compound (IX-2) can be obtained, for example, as a commercial product.

Step 3

Compound (I-5) can be produced using Compound (XII) by the method described in Step 1 or according to the method.

Step 4

Compound (I) in which $R^{12}$ is primary or secondary lower alkyl and $R^{13}$ is lower alkyl can be produced by reacting Compound (I-5) with preferably 1 to 10 equivalents of Compound (XIV) in a solvent in the presence of preferably

1 to 10 equivalents of a base at a temperature preferably between 0°C and the boiling point of the solvent used for 5 minutes to 72 hours.

Examples of the base include potassium carbonate, potassium hydroxide, sodium hydroxide, sodium methoxide, potassium tert-butoxide, 1,8-diazabicyclo[5.4.0]-7-undecene (DBU), sodium hydride, and the like.
Examples of the solvent include DMF, DMA, NMP, benzene, toluene, hexane, ethyl acetate, DME, THF, dioxane, diethyl ether, and the like. These solvents may be used alone or as a mixture thereof.

Compound (XIV) can be obtained, for example, as a commercial product.

Step 5

Compound (I-4) can be produced by reacting Compound (V) with preferably 1 to 10 equivalents of Compound (X) in a solvent in the presence of preferably 1 to 10 equivalents of a base at a temperature between -10°C and the boiling point of the solvent used, preferably a temperature between 0°C and the boiling point of the solvent used, for 5 minutes to 72 hours and then reacting with preferably 1 to 10 equivalents of Compound (XI) in a solvent in the presence of preferably 1 to 10 equivalents of a base at a temperature between -10°C and the boiling point of the solvent used, preferably a temperature between 0°C and the boiling point of the solvent used, for 5 minutes to 72 hours.

Examples of the solvent include dichloromethane, chloroform, 1,2-dichloroethane, toluene, ethyl acetate, acetonitrile, diethyl ether, THF, DME, DMF, DMA, NMP, pyridine, and the like. These solvents may be used alone or as a mixture thereof.
Compound (X) can be obtained, for example, as a commercial product.
Compound (XI) can be obtained, for example, as a commercial product or can be produced by the method described in WO 1999/032106 or in Step 2 of Production method 3, or according to the method.

Step 6

Compound (I) in which $R^{12}$ is lower alkyl can be produced using Compound (I-4) and Compound (XIII) by the method described in Step 4 or according to the method.

Compound (XIII) can be obtained, for example, as a commercial product.

Step 7

Compound (I) in which at least one of $R^{12}$ and $R^{13}$ is lower alkyl can be produced using Compound (I-6), Compound (XIII), and/or Compound (XIV) by the method described in Step 4 or according to the method.

Compound (XIII) and Compound (XIV) can be obtained, for example, as commercial products.
The intermediate and the target compound in each production method described above can be isolated and purified by a separation and purification method commonly used in synthetic organic chemistry, for example, filtration, extraction, washing, drying, concentration, recrystallization, various chromatographies, and the like. Further, the intermediate may be subjected to the subsequent reaction without any particularly purification.

In Compound (I), a stereoisomer such as a geometrical isomer or an optical isomer, a tautomer, and the like may be present. All possible isomers including these isomers and mixtures thereof are used in the present invention or are included in the present invention.
Compounds in which one or more hydrogen atoms in Compound (I) are substituted by deuterium atoms are also included in Compound (I), and these compounds and mixtures thereof are used in the present invention or are included in the present invention.

In the case where a salt of Compound (I) is desired, and when Compound (I) is obtained in a salt form, the salt may be simply purified as it is; or when Compound (I) is obtained in a free form, the Compound (I) may be allowed to form a salt by adding an acid or a base thereto after dissolved or suspended in a suitable solvent, and the resulting salt may be isolated and purified.
Compound (I) or a pharmaceutically acceptable salt thereof can also exist in an adduct form with water or various solvents, and such an adduct is used in the present invention or is included in the present invention.

Specific examples of Compound (I) are shown in Tables 1 to 10, but examples of Compound (I) of the present invention are not limited thereto.
In the tables, Me represents methyl, Et represents ethyl, i-Pr represents isopropyl, and t-Bu represents tert-butyl.

Table 1

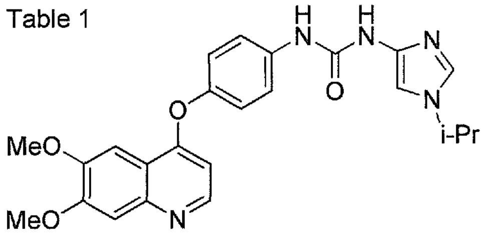

Compound 1

Table 2

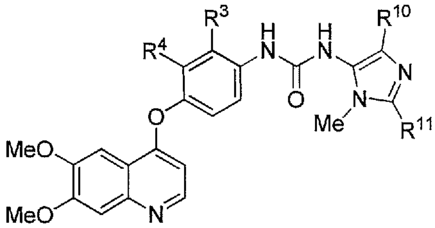

| Compound No. | $R^3$ | $R^4$ | $R^{10}$ | $R^{11}$ | Compound No. | $R^3$ | $R^4$ | $R^{10}$ | $R^{11}$ |
|---|---|---|---|---|---|---|---|---|---|
| 2 | H | H | 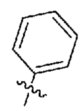 | t-Bu | 11 | H | H | 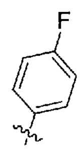 | t-Bu |
| 3 | H | H | 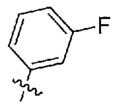 | i-Pr | 12 | H | F | 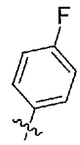 | t-Bu |
| 4 | H | F | 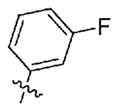 | i-Pr | 13 | H | F | 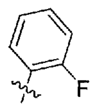 | i-Pr |
| 5 | F | H | 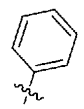 | t-Bu | 14 | F | H | 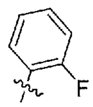 | i-Pr |
| 6 | F | H | 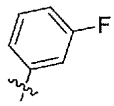 | t-Bu | 15 | H | F | 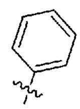 | t-Bu |
| 7 | F | H | 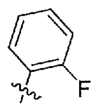 | t-Bu | 16 | F | H | 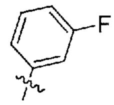 | i-Pr |
| 8 | H | F | 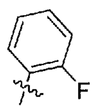 | t-Bu | 17 | H | H | 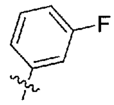 | t-by |
| 9 | H | F | 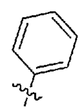 | i-Pr | 18 | H | F | 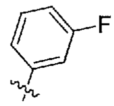 | t-Bu |
| 10 | H | F | 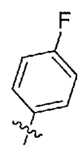 | i-Pr | 19 | H | H | 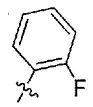 | t-Bu |

Table 3

le 3

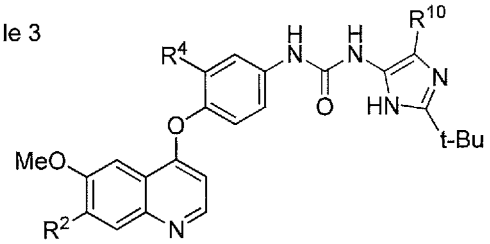

| Compound No. | $R^2$ | $R^4$ | $R^{10}$ |
|---|---|---|---|
| 20 | MeO | H | 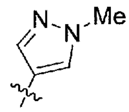 |
| 21 | MeO | H | 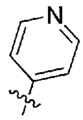 |
| 22 | MeO | H | 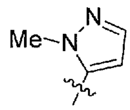 |
| 23 | MeO | F | 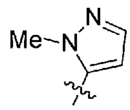 |
| 24 | MeO | H | 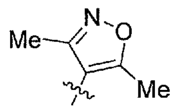 |
| 25 | MeO | H | 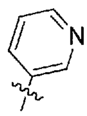 |
| 26 | MeO | H | 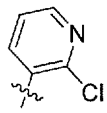 |
| 27 | 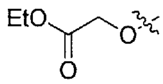 | H | 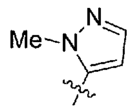 |
| 28 | 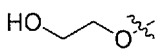 | H | 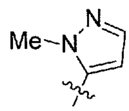 |

Table 4

Table 4

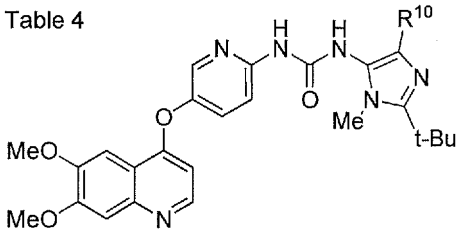

| Compound No. | $R^{10}$ |
|---|---|
| 29 | 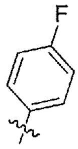 |
| 30 | 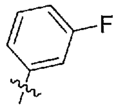 |
| 31 | 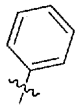 |
| 32 | 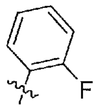 |

Table 5

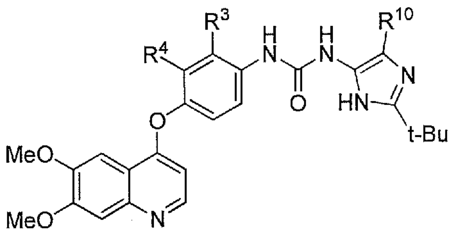

| Compound No. | $R^3$ | $R^4$ | $R^{10}$ | Compound No. | $R^3$ | $R^4$ | $R^{10}$ |
|---|---|---|---|---|---|---|---|
| 33 | H | H | 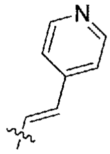 | 41 | H | F | 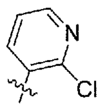 |
| 34 | H | H | 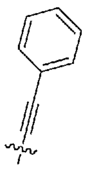 | 42 | H | H | 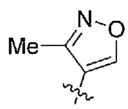 |
| 35 | H | H | 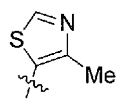 | 43 | H | H | 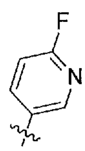 |

(continued)

Table 5

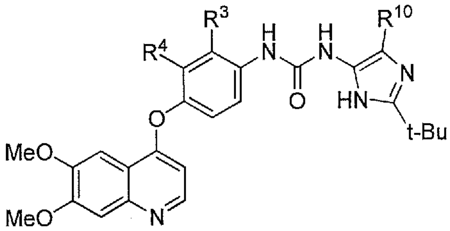

| Compound No. | $R^3$ | $R^4$ | $R^{10}$ | Compound No. | $R^3$ | $R^4$ | $R^{10}$ |
|---|---|---|---|---|---|---|---|
| 36 | H | H | 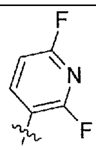 | 44 | Cl | H | 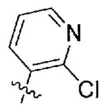 |
| 37 | H | H | 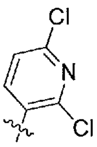 | 45 | Me | H | 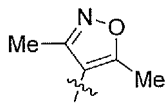 |
| 38 | H | H | 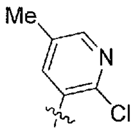 | 46 | H | Cl | 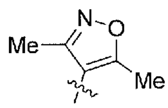 |
| 39 | H | H | 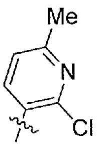 | 47 | H | F | 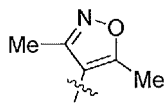 |
| 40 | H | H | 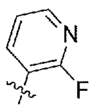 | 48 | Cl | H | 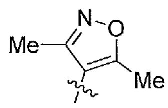 |

Table 6

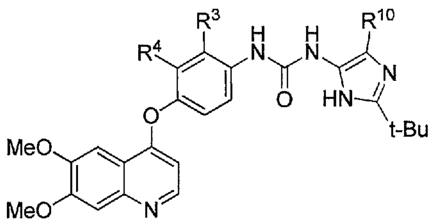

| Compound No. | $R^3$ | $R^4$ | $R^{10}$ | Compound No. | $R^3$ | $R^4$ | $R^{10}$ |
|---|---|---|---|---|---|---|---|
| 49 | F | H | 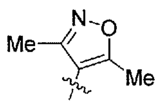 | 58 | H | Cl | 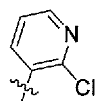 |
| 50 | H | H | 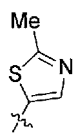 | 59 | H | H | 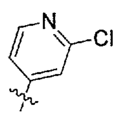 |
| 51 | H | H | 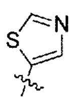 | 60 | OMe | H | 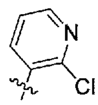 |

(continued)

Table 6

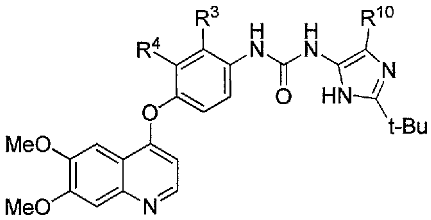

| Compound No. | $R^3$ | $R^4$ | $R^{10}$ | Compound No. | $R^3$ | $R^4$ | $R^{10}$ |
|---|---|---|---|---|---|---|---|
| 52 | H | H | 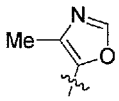 | 61 | Me | H | 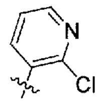 |
| 53 | H | H | 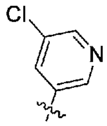 | 62 | F | H | 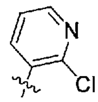 |
| 54 | H | H | 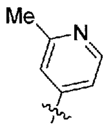 | 63 | H | H | 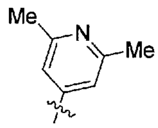 |
| 55 | H | H | 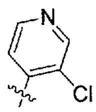 | 64 | H | H | 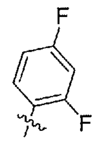 |
| 56 | H | H | 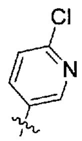 | 65 | H | H | 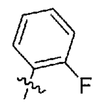 |
| 57 | H | H | 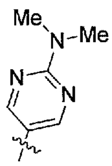 | 66 | H | H | H |

Table 7

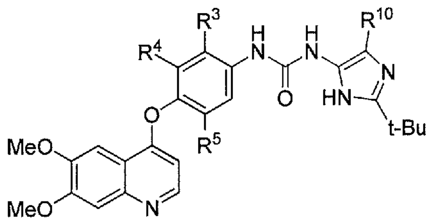

| Compound No. | $R^3$ | $R^4$ | $R^5$ | $R^{10}$ |
|---|---|---|---|---|
| 67 | Me | H | Me | 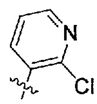 |
| 68 | Me | Me | H | 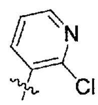 |

(continued)

| Table 7 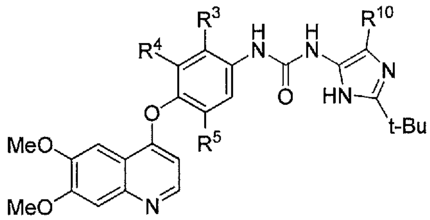 | | | | |
|---|---|---|---|---|
| Compound No. | $R^3$ | $R^4$ | $R^5$ | $R^{10}$ |
| 69 | H | H | H | 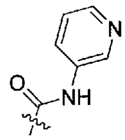 |
| 70 | H | H | H | 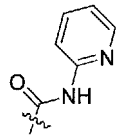 |

Table 8

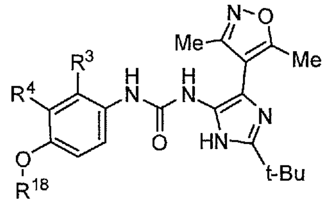

| Compound No. | $R^3$ | $R^4$ | $R^{18}$ | Compound No. | $R^3$ | $R^4$ | $R^{18}$ |
|---|---|---|---|---|---|---|---|
| 71 | H | H | 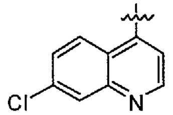 | 77 | H | F | 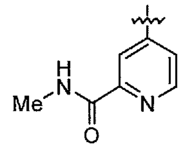 |
| 72 | Cl | H | 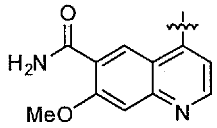 | 78 | H | H | 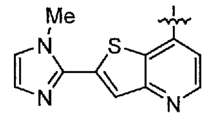 |
| 73 | H | F | 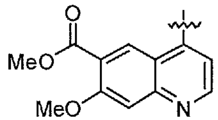 | 79 | H | H | 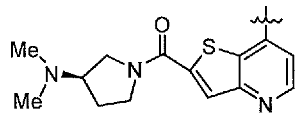 |
| 74 | H | F | 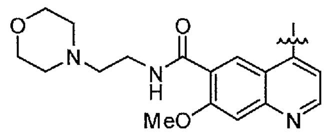 | 80 | H | H | 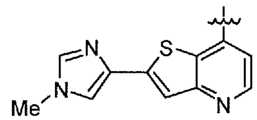 |
| 75 | H | H | 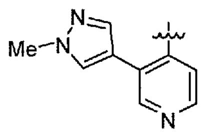 | 81 | H | H | 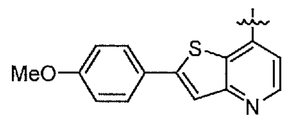 |
| 76 | H | H | 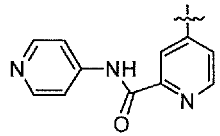 | 82 | H | H | 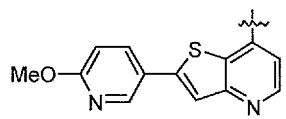 |

Table 9

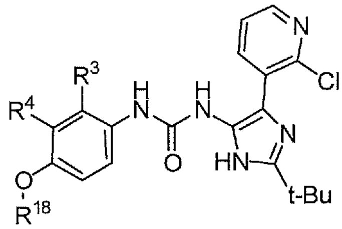

| Compound No. | $R^3$ | $R^4$ | $R^{18}$ |
|---|---|---|---|
| 83 | Cl | H | 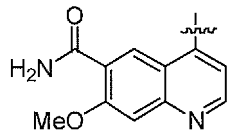 |
| 84 | OMe | H | 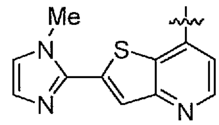 |
| 85 | H | F | 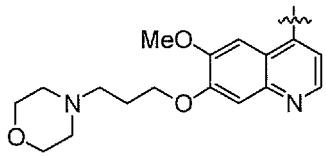 |

Next, pharmacological effects of typical Compound (I) will be specifically described with reference to test examples.

Test Example 1: Measurement of inhibitory activity of human FGFR2 enzymatic activity

FGFR2 (FGFR2, active, 14-617, Millipore) diluted with an assay buffer [100 mmol/L of 4-(2-hydroxyethyl)-1-piperazineethanesulfonic acid (HEPES), 10 mmol/L of magnesium chloride, 10 mmol/L of manganese chloride, 0.003 vol% of Brij-35 (BRIJ (registered trademark) 35 Detergent, 30% aqueous solution, Calbiochem), 0.004 vol% of Tween 20 (PlusOne™ Tween™ 20 (trade name), Amersham Biosciences), and 1 mmol/L of dithiothreitol, pH 7.5], a fluorescently labeled substrate (FL-Peptide 22, 760366, Caliper Life Sciences), ATP (A7699, Sigma-Aldrich), and a test compound dissolved in DMSO were added to a 384-well polypropylene plate (3657, Corning) in the total amount of 25 μL and were subjected to a reaction at 28°C for 240 minutes. The final concentrations of FGFR2, fluorescently labeled substrate, ATP, and DMSO were 2 nmol/L, 1.5 μmol/L, 84 μmol/L, and 1%, respectively. Then, 45 μL of a termination buffer [100 mmol/L of HEPES, 15 mmol/L of ethylenediaminetetraacetic acid (EDTA), 0.022 vol% of Brij-35, 0.17% of Coating Reagent 3 (LabChip Coating Reagent 3, Caliper Life Sciences), and 7.2% of DMSO, pH 7.5] was added to the plate to stop the reaction. Subsequently, the reaction solution was aspirated with a mobility shift assay system (LabChip EZ Reader II, Caliper Life Sciences), and a voltage and a pressure were added to the flow path to separate the fluorescently labeled substrate phosphorylated by the enzyme reaction from the unchanged fluorescently labeled substrate based on the charge difference. The fluorescence intensities at 530 nm of the phosphorylated and the unchanged fluorescently labeled substrates were measured at an excitation wavelength of 488 nm. Enzymatic activity is measured as follows. The conversion ratio under each condition was calculated by the following Equation 1:

Equation 1

$$\text{Conversion ratio (\%)} = \frac{\text{peak of phosphorylated fluorescently labeled substrate}}{\begin{pmatrix}\text{peak of phosphorylated}\\ \text{fluorescently labeled substrate}\end{pmatrix} + \begin{pmatrix}\text{peak of unchanged}\\ \text{fluorescently labeled substrate}\end{pmatrix}} \times 100$$

The inhibition ratio of FGFR2 phosphorylation inhibition ratio was determined by the following Equation 2 based

on the resulting conversion ratios.

Equation 2

$$\text{Inhibition ratio of FGFR2 phosphorylation (\%)} = \frac{\begin{pmatrix}\text{conversion ratio of reaction solution containing}\\ \text{FGFR2, FL-peptide 22, ATP, and 1\% DMSO}\end{pmatrix} - \begin{pmatrix}\text{conversion ratio of reaction solution}\\ \text{containing FGFR2, FL-peptide 22,}\\ \text{ATP, Compound, and 1\% DMSO}\end{pmatrix}}{\begin{pmatrix}\text{conversion ratio of reaction solution containing}\\ \text{FGFR2, FL-peptide 22, ATP, and 1\% DMSO}\end{pmatrix} - \begin{pmatrix}\text{conversion ratio of reaction solution}\\ \text{containing FL-peptide 22, ATP,}\\ \text{and 1\% DMSO}\end{pmatrix}} \times 100$$

The inhibition ratios of FGFR2 phosphorylation at various concentrations of the test compound were determined, and the 50% inhibitory concentration ($IC_{50}$) of FGFR2 phosphorylation of the test compound was calculated using the inhibition ratios at various concentrations. As a result, Compounds 2 to 26, 28 to 70, 72 to 74, 78 to 80, and 83 to 85 each showed an $IC_{50}$ value less than 100 nmol/L.

It was confirmed by the results of the above-mentioned test that Compound (I) has an FGFR2 phosphorylation inhibition activity. Accordingly, it is believed that Compound (I) or a pharmaceutically acceptable salt thereof inhibits FGFR and is useful as a therapeutic and/or preventive agent for a disease in which FGFR is involved, for example, various cancers (for example, stomach cancer, endometrial cancer, pituitary tumor, myeloproliferative disorder, renal cancer, bladder cancer, colon cancer, head and neck cancer, skin cancer, non-Hodgkin's lymphoma, brain tumor, breast cancer, ovarian cancer, multiple myeloma, osteosarcoma, and the like), virus infection, an autoimmune disease such as systemic lupus erythematosus, rheumatoid arthritis, hepatitis, a renal disease, or the like. Test Example 2: Growth inhibition test using human scirrhous gastric cancer cell line

OCUM-2MD3 cells (human scirrhous gastric cancer cell line) were cultured in a Dulbecco's modified Eagle medium (DMEM) 1640 medium (Invitrogen, catalog No. 11995-065) supplemented with 10% fetal calf serum (Invitrogen, catalog No. 10091-148) and penicillin-streptomycin (Invitrogen, catalog No. 15140-122).

OCUM-2MD3 cells were prepared in an amount of $1.25 \times 10^4$ cells per 1 mL of the medium, and 80 $\mu$L of the medium containing the cells was seeded in each well of a Nunc 96-Microwell plate (Thermo Scientific, catalog No. 167008). The cells were cultured at 37°C for 24 hours in an incubator with 5% carbon dioxide. Twenty microliters of a diluted solution of a test compound was added to each well (to give a final concentration of DMSO 0.1 vol% in the plate), followed by culturing in the incubator with 5% carbon dioxide at 37°C for 72 hours. Subsequently, 20 $\mu$L of a WST-1 reagent (4-[3-(4-iodophenyl)-2-(4-nitrophenyl)-2H-5-tetrazolio]-1,3-benzenedisulfonate sodium salt) (Roche Diagnostics, catalog No. 11644807) diluted with the above-mentioned medium to 50% was added to each well, followed by incubation at 37°C for 2 hours. The absorbance at 450 nm (reference wavelength: 690 nm) of each well was measured with a microplate spectrophotometer SPECTRA max 340PC (Molecular Devices) to calculate the growth inhibition ratio by the following Equation 3:

Equation 3

$$\text{Growth inhibition ratio (\%)} = 100 - \frac{C - B}{A - B} \times 100$$

A: absorbance of well after culturing for 72 hours in the presence of OCUM-2MD3 cell, DMEM medium, and DMSO
B: absorbance of well after culturing for 72 hours in the presence of DMEM medium and DMSO
C: absorbance of well after culturing for 72 hours in the presence of OCUM-2MD3 cell, DMEM medium, and test compound (DMSO solution)

The growth inhibition ratios were determined by three experiments, and the average thereof was calculated. The concentrations of a test compound were plotted on the x-axis, and the average values of inhibition ratios at various concentrations of the test compound on the y-axis were plotted. These plots were approximated with Model 205 equation of Xlfit3 ver.4.2.1 (ID Business Solutions Ltd., Surrey, UK) to calculate the $IC_{50}$ value as the concentration showing a

growth inhibition ratio of 50%.

As a result, Compounds 22, 24, 26, 37 to 44, and 47 showed $IC_{50}$ values smaller than 30 nmol/L. Accordingly, it is believed that Compound (I) or a pharmaceutically acceptable salt thereof shows a growth inhibition activity of OCUM-2MD3 cells and is useful as a therapeutic and/or preventive agent for gastric cancer.

Furthermore, some compounds of Compound (I) or a pharmaceutically acceptable salt thereof were actually orally active.

Compound (I) or a pharmaceutically acceptable salt thereof can be administered alone. Compound (I) or a pharmaceutically acceptable salt thereof is usually preferably provided as various pharmaceutical formulations. Further, such pharmaceutical formulations are used in animals or humans.

The pharmaceutical formulation according to the present invention may contain Compound (I) or a pharmaceutically acceptable salt thereof alone as an active ingredient or as a mixture thereof with an optional active ingredient for any other therapy. Further, the pharmaceutical formulation according to the present invention is prepared by mixing the active ingredient with one or more pharmaceutically acceptable carriers (for example, a diluent, a solvent, an excipient, and the like) and subjecting the mixture to an optional method well-known in the technical field of pharmaceutics.

Regarding the administration route, it is preferable that the most effective route is selected for therapy. Examples of the administration route include oral and parenteral, such as intravenous, administration.

Examples of the dosage form include a tablet, an injection, and the like.

For example, a tablet, and the like suitable for oral administration can be produced using, for example, an excipient such as lactose, a disintegrator such as starch, a lubricant such as magnesium stearate, a binder such as hydroxypropyl cellulose, and the like.

For example, an injection, and the like suitable for parenteral administration can be produced using a diluent or a solvent such as a salt solution, a glucose solution, or a solution mixture of brine and a glucose solution.

The dose and frequency of administration of Compound (I) or a pharmaceutically acceptable salt thereof may vary depending on the dosage form, the age and the body weight of a patient, a property or seriousness of the symptom to be treated, and the like. In oral administration, in general, a dose of 0.01 to 1000 mg, preferably 0.05 to 100 mg, is administered to an adult once to several times a day. In parenteral administration, such as intravenous administration, a dose of 0.001 to 1000 mg, preferably 0.01 to 100 mg, is administered to an adult once to several times a day. However, the dose and frequency of administration vary depending on the various conditions mentioned above.

Hereinafter, the present invention will be described more specifically with reference to examples and reference examples. However, the scope of the present invention is not limited to these examples.

Note that proton nuclear magnetic resonance spectra ($^1H$ NMR) used in Examples and Reference Examples were determined at 270 MHz, 300 MHz, or 400 MHz, and exchangeable protons are not clearly observed in some compounds and measurement conditions. Further, for the descriptions of the multiplicity of signals, those generally applied are used, and the symbol "br" represents an apparently broad signal.

Each compound is named using ChemBioDraw Ultra Ver. 11.0 (CambridgeSoft Corporation).

Example 1

1-[4-(6,7-Dimethoxyquinolin-4-yloxy)phenyl]-3-(1-isopropyl-1H-imidazol-4-yl)urea (Compound 1)

Compound A2 (66 mg) obtained according to Reference Example 1 was dissolved in chloroform (5 mL), and triethylamine (66 mg), diphenylphosphoryl azide (179 mg), and 4-(6,7-dimethoxyquinolin-4-yloxy)aniline (127 mg) obtained according to Example 49 of WO 97/17329 were added to the resulting solution. The mixture was stirred with heating under reflux overnight. Water was added to the resulting reaction mixture, and extraction with chloroform was performed. The organic phase was dried over anhydrous sodium sulfate and was filtered. The solvent was distilled off from the filtrate under reduced pressure, and the obtained residue was purified by silica gel column chromatography using chloroform and methanol as the eluents to give the titled compound 1 (24 mg, yield: 13%).

ESI-MS: *m/z* 446$[M-H]^-$, 470$[M+H]^+$

Example 2

1-(2-tert-Butyl-1-methyl-4-phenyl-1H-imidazol-5-yl)-3-[4-(6,7-dimethoxyquinolin-4-yloxy)phenyl]urea (Compound 2)

Compound A6 (30 mg) obtained according to Reference Example 2 was dissolved in chloroform (2 mL), and triethylamine (17.6 mg) and diphenylphosphoryl azide (48 mg) were added to the resulting solution. The mixture was stirred under reflux for 1 hour. 4-(6,7-Dimethoxyquinolin-4-yloxy)aniline (31 mg) obtained according to Example 49 of WO 97/17329 was further added to the resulting reaction mixture, and the mixture was stirred under reflux overnight. Water was added to the resulting reaction mixture, and extraction with chloroform was performed. The organic phase

was dried over anhydrous sodium sulfate and was filtered. The solvent was distilled off from the filtrate under reduced pressure, and the residue obtained was purified by silica gel column chromatography using chloroform and methanol as the eluents to give the titled compound 2 (38 mg, yield: 66%).
$^1$H-NMR ($CDCl_3$) $\delta$: 8.45 (1H, d, J = 5.4 Hz), 7.92 (2H, d, J = 7.3 Hz), 7.52 (1H, s), 7.38 (3H, dd, J = 12.8, 5.2 Hz), 7.30-7.23 (3H, m), 7.06 (2H, dt, J = 9.6, 2.7 Hz), 6.79 (1H, s), 6.40 (2H, d, J = 5.1 Hz), 4.03 (6H, s), 3.67 (3H, s), 1.51 (9H, s).
ESI-MS: *m*/*z* 552$[M+H]^+$

Example 3

1-[4-(6,7-Dimethoxyquinolin-4-yloxy)phenyl]-3-[4-(3-fluorophenyl)-2-isopropyl-1-methyl-1H-imidazol-5-yl]urea

(Compound 3)

The titled compound 3 was obtained according to Example 1 (provided that toluene was used instead of chloroform as the solvent) from 4-(3-fluorophenyl)-2-isopropyl-1-methyl-1H-imidazole-5-carboxylic acid (Compound A6-1) obtained according to Reference Example 2 and 4-(6,7-dimethoxyquinolin-4-yloxy)aniline obtained according to Example 49 of WO 97/17329.
$^1$H-NMR ($CDCl_3$) $\delta$: 8.45 (1H, d, J = 5.4 Hz), 7.67 (2H, d, J = 7.6 Hz), 7.52 (1H, s), 7.41 (1H, s), 7.34 (3H, dt, J = 11.0, 4.9 Hz), 7.09 (2H, dd, J = 6.8, 2.2 Hz), 6.96 (1H, dt, J = 14.1, 5.0 Hz), 6.63 (1H, s), 6.42 (1H, d, J = 5.4 Hz), 6.14 (1H, s), 4.04 (3H, s), 4.03 (3H, s), 3.55 (3H, s), 3.09-3.02 (1H, m), 1.41 (6H, d, J = 7.1 Hz).
ESI-MS: *m*/*z* 556$[M+H]^+$

Example 4

1-[4-(6,7-Dimethoxyquinolin-4-yloxy)-3-fluorophenyl]-3-[4-(3-fluorophenyl)-2-isopropyl-1-methyl-1H-imidazol-5-yl]urea (Compound 4)

The titled compound 4 was obtained according to Example 3 from 4-(3-fluorophenyl)-2-isopropyl-1-methyl-1H-imidazole-5-carboxylic acid (Compound A6-1) obtained according to Reference Example 2 and 4-(6,7-dimethoxyquinolin-4-yloxy)-3-fluoroaniline obtained according to Production Example 2 of WO 01/47890.
$^1$H-NMR ($CDCl_3$) $\delta$: 8.46 (1H, d, J = 5.1 Hz), 7.66 (2H, d, J = 7.6 Hz), 7.55 (1H, s), 7.49 (1H, dd, J = 12.4, 10.0 Hz), 7.41 (1H, s), 7.34 (1H, dd, J = 14.0, 7.7 Hz), 7.12 (1H, t, J = 8.7 Hz), 6.96 (2H, dt, J = 15.7, 6.7 Hz), 6.76 (1H, s), 6.36 (1H, d, J = 5.4 Hz), 6.26 (1H, s), 4.05 (3H, s), 4.03 (3H, s), 3.54 (3H, s), 3.09-3.02 (1H, m), 1.41 (6H, d, J = 6.8 Hz).
ESI-MS: *m*/*z* 574$[M+H]^+$

Example 5

1-(2-tert-Butyl-1-methyl-4-phenyl-1H-imidazol-5-yl)-3-[4-(6,7-dimethoxyquinolin-4-yloxy)-2-fluorophenyl]urea (Compound 5)

The titled compound 5 was obtained according to Example 3 from Compound A6 obtained according to Reference Example 2 and 4-(6,7-dimethoxyquinolin-4-yloxy)-2-fluoroaniline obtained according to Journal of Medicinal Chemistry, 2005, 48(5), 1359-1366.
$^1$H-NMR ($CDCl_3$) $\delta$: 8.49 (1H, d, J = 5.4 Hz), 8.06 (1H, t, J = 8.9 Hz), 7.91 (2H, d, J = 7.1 Hz), 7.47-7.37 (4H, m), 7.26 (1H, s), 7.00-6.86 (2H, m), 6.66 (1H, s), 6.47 (1H, d, J = 5.4 Hz), 6.13 (1H, s), 4.04 (3H, s), 4.03 (3H, s), 3.70 (3H, s), 1.52 (9H, s).
ESI-MS: *m*/*z* 570$[M+H]^+$

Example 6

1-[2-tert-Butyl-4-(3-fluorophenyl)-1-methyl-1H-imidazol-5-yl]-3-[4-(6,7-dimethoxyquinolin-4-yloxy)-2-fluorophenyl]urea (Compound 6)

The titled compound 6 was obtained according to Example 3 from 2-tert-butyl-4-(3-fluorophenyl)-1-methyl-1H-imidazole-5-carboxylic acid (Compound A6-2) obtained according to Reference Example 2 and 4-(6,7-dimethoxyquinolin-4-yloxy)-2-fluoroaniline obtained according to Journal of Medicinal Chemistry, 2005, 48(5), 1359-1366.
$^1$H-NMR ($CDCl_3$) $\delta$: 8.49 (1H, d, J = 5.4 Hz), 8.10-8.04 (1H, m), 7.67 (2H, t, J = 6.3 Hz), 7.44 (2H, d, J = 20.5 Hz), 7.36-7.31 (1H, m), 6.98-6.87 (3H, m), 6.70 (1H, s), 6.47 (1H, d, J = 5.4 Hz), 6.35 (1H, s), 4.04 (3H, s), 4.03 (3H, s), 3.70

(3H, s), 1.51 (9H, s).
ESI-MS: *m/z* 588 $[M+H]^+$

Example 7

1-[2-tert-Butyl-4-(2-fluorophenyl)-1-methyl-1H-imidazol-5-yl]-3-[4-(6,7-dimethoxyquinolin-4-yloxy)-2-fluorophenyl]urea (Compound 7)
The titled compound 7 was obtained according to Example 3 from 2-tert-butyl-4-(2-fluorophenyl)-1-methyl-1H-imidazole-5-carboxylic acid (Compound A6-3) obtained according to Reference Example 2 and 4-(6,7-dimethoxyquinolin-4-yloxy)-2-fluoroaniline obtained according to Journal of Medicinal Chemistry, 2005, 48(5), 1359-1366.
$^1$H-NMR ($CDCl_3$) δ: 8.49 (1H, d, J = 5.1 Hz), 8.09 (1H, td, J = 9.0, 4.2 Hz), 7.81 (1H, td, J = 7.5, 1.8 Hz), 7.48 (1H, s), 7.42 (1H, s), 7.30-7.20 (2H, m), 7.11-7.06 (1H, m), 7.00-6.89 (2H, m), 6.62 (1H, s), 6.46 (1H, d, J = 5.1 Hz), 6.25 (1H, s), 4.04 (3H, s), 4.03 (3H, s), 3.72 (3H, s), 1.50 (9H, s).
ESI-MS: *m/z* 588$[M+H]^+$

Example 8

1-[2-tert-Butyl-4-(2-fluorophenyl)-1-methyl-1H-imidazol-5-yl]-3-[4-(6,7-dimethoxyquinolin-4-yloxy)-3-fluorophenyl]urea (Compound 8)

The titled compound 8 was obtained according to Example 3 from 2-tert-butyl-4-(2-fluorophenyl)-1-methyl-1H-imidazole-5-carboxylic acid (Compound A6-3) obtained according to Reference Example 2 and 4-(6,7-dimethoxyquinolin-4-yloxy)-3-fluoroaniline obtained according to Production Example 2 of WO 01/47890. $^1$H-NMR ($CDCl_3$) δ: 8.46 (1H, d, J = 5.1 Hz), 7.80 (1H, td, J = 7.6, 1.9 Hz), 7.56 (1H, s), 7.47-7.42 (2H, m), 7.31-7.21 (4H, m), 7.06 (3H, tt, J = 30.2, 8.3 Hz), 6.37 (1H, d, J = 5.1 Hz), 4.05 (3H, s), 4.04 (3H, s), 3.72 (3H, s), 1.52 (9H, s). ESI-MS: *m/z* 588$[M+H]^+$

Example 9

1-[4-(6,7-Dimethoxyquinolin-4-yloxy)-3-fluorophenyl]-3-(2-isopropyl-1-methyl-4-phenyl-1H-imidazol-5-yl)urea (Compound 9)

The titled compound 9 was obtained (yield: 73%) according to Example 3 from 2-isopropyl-1-methyl-4-phenyl-1H-imidazole-5-calboxylic acid (Compound A6-4) obtained according to Reference Example 2 and 4-(6,7-dimethoxyquinolin-4-yloxy)-3-fluoroaniline obtained according to Production Example 2 of WO 01/47890. ESI-MS: *m/z* 554$[M-H]^-$, 578$[M+H]^+$

Example 10

1-[4-(6,7-Dimethoxyquinolin-4-yloxy)-3-fluorophenyl]-3-[4-(4-fluorophenyl)-2-isopropyl-1-methyl-1H-imidazol-5-yl]urea (Compound 10)

The titled compound 10 was obtained (yield: 64%) according to Example 3 from 4-(4-fluorophenyl)-2-isopropyl-1-methyl-1H-imidazole-5-carboxylic acid (Compound A6-5) obtained according to Reference Example 2 and 4-(6,7-dimethoxyquinolin-4-yloxy)-3-fluoroaniline obtained according to Production Example 2 of WO 01/47890. ESI-MS: *m/z* 574$[M-H]^-$

Example 11

1-[2-tert-Butyl-4-(4-fluorophenyl)-1-methyl-1H-imidazol-5-yl]-3-[4-(6,7-dimethoxyquinolin-4-yloxy)phenyl]urea (Compound 11)

The titled compound 11 was obtained (yield: 72%) according to Example 3 from 2-tert-butyl-4-(4-fluorophenyl)-1-methyl-1H-imidazole-5-carboxylic acid (Compound A6-6) obtained according to Reference Example 2 and 4-(6,7-dimethoxyquinolin-4-yloxy)aniline obtained according to Example 49 of WO 97/17329.
ESI-MS: *m/z* 570$[M+H]^+$

Example 12

1-[2-tert-Butyl-4-(4-fluorophenyl)-1-methyl-1H-imidazol-5-yl]-3-[4-(6,7-dimethoxyquinolin-4-yloxy)-3-fluorophenyl]urea (Compound 12)

The titled compound 12 was obtained (yield: 46%) according to Example 3 from 2-tert-butyl-4-(4-fluorophenyl)-1-methyl-1H-imidazole-5-carboxylic acid (Compound A6-6) obtained according to Reference Example 2 and 4-(6,7-dimethoxyquinolin-4-yloxy)-3-fluoroaniline obtained according to Production Example 2 of WO 01/47890. ESI-MS: *m/z* 588$[M+H]^+$

Example 13

1-[4-(6,7-Dimethoxyquinolin-4-yloxy)-3-fluorophenyl]-3-[4-(2-fluorophenyl)-2-isopropyl-1-methyl-1H-imidazol-5-yl]urea (Compound 13)

The titled compound 13 was obtained (yield: 50%) according to Example 3 from 4-(2-fluorophenyl)-2-isopropyl-1-methyl-1H-imidazole-5-carboxylic acid (Compound A6-7) obtained according to Reference Example 7 and 4-(6,7-dimethoxyquinolin-4-yloxy)-3-fluoroaniline obtained according to Production Example 2 of WO 01/47890. ESI-MS: *m/z* 574$[M+H]^+$

Example 14

1-[4-(6,7-Dimethoxyquinolin-4-yloxy)-2-fluorophenyl]-3-[4-(2-fluorophenyl)-2-isopropyl-1-methyl-1H-imidazol-5-yl]urea (Compound 14)

The titled compound 14 was obtained (yield: 64%) according to Example 3 from 4-(2-fluorophenyl)-2-isopropyl-1-methyl-1H-imidazole-5-carboxylic acid (Compound A6-7) obtained according to Reference Example 7 and 4-(6,7-dimethoxyquinolin-4-yloxy)-2-fluoroaniline obtained according to Journal of Medicinal Chemistry, 2005, 48(5), 1359-1366.
ESI-MS: *m/z* 574$[M+H]^+$

Example 15

1-(2-tert-Butyl-1-methyl-4-phenyl-1H-imidazol-5-yl)-3-[4-(6,7-dimethoxyquinolin-4-yloxy)-3-fluorophenyl]urea

(Compound 15)

The titled compound 15 was obtained (yield: 55%) according to Example 3 from Compound A6 obtained according to Reference Example 2 and 4-(6,7-dimethoxyquinolin-4-yloxy)-3-fluoroaniline obtained according to Production Example 2 of WO 01/47890.
ESI-MS: *m/z* 570$[M+H]^+$

Example 16

1-[4-(6,7-Dimethoxyquinolin-4-yloxy)-2-fluorophenyl]-3-[4-(3-fluorophenyl)-2-isopropyl-1-methyl-1H-imidazol-5-yl]urea (Compound 16)

The titled compound 16 was obtained (yield: 55%) according to Example 3 from 4-(3-fluorophenyl)-2-isopropyl-1-methyl-1H-imidazole-5-carboxylic acid (Compound A6-1) obtained according to Reference Example 2 and 4-(6,7-dimethoxyquinolin-4-yloxy)-2-fluoroaniline obtained according to Journal of Medicinal Chemistry, 2005, 48(5), 1359-1366.
ESI-MS: *m/z* 574$[M+H]^+$

Example 17

1-[2-tert-Butyl-4-(3-fluorophenyl)-1-methyl-1H-imidazol-5-yl]-3-[4-(6,7-dimethoxyquinolin-4-yloxy)phenyl]urea (Compound 17)

The titled compound 17 was obtained (yield: 79%) according to Example 3 from 2-tert-butyl-4-(3-fluorophenyl)-1-methyl-1H-imidazole-5-carboxylic acid (Compound A6-2) obtained according to Reference Example 2 and 4-(6,7-dimethoxyquinolin-4-yloxy)aniline obtained according to Example 49 of WO 97/17329.
ESI-MS: *m*/*z* 570$[M+H]^+$

Example 18

1-[2-tert-Butyl-4-(3-fluorophenyl)-1-methyl-1H-imidazol-5-yl]-3-[4-(6,7-dimethoxyquinolin-4-yloxy)-3-fluorophenyl]urea (Compound 18)

The titled compound 18 was obtained (yield: 66%) according to Example 3 from 2-tert-butyl-4-(3-fluorophenyl)-1-methyl-1H-imidazole-5-carboxylic acid (Compound A6-2) obtained according to Reference Example 2 and 4-(6,7-dimethoxyquinolin-4-yloxy)-3-fluoroaniline obtained according to Production Example 2 of WO 01/47890. ESI-MS: *m*/*z* 588$[M+H]^+$

Example 19

1-[2-tert-Butyl-4-(2-fluorophenyl)-1-methyl-1H-imidazol-5-yl]-3-[4-(6,7-dimethoxyquinolin-4-yloxy)phenyl]urea (Compound 19)

The titled compound 19 was obtained (yield: 88%) according to Example 3 from 2-tert-butyl-4-(2-fluorophenyl)-1-methyl-1H-imidazole-5-carboxylic acid (Compound A6-3) obtained according to Reference Example 2 and 4-(6,7-dimethoxyquinolin-4-yloxy)aniline obtained according to Example 49 of WO 97/17329.
ESI-MS: *m*/*z* 570$[M+H]^+$

Example 20

1-[2-tert-Butyl-4-(1-methyl-1H-pyrazol-4-yl)-1H-imidazol-5-yl]-3-[4-(6,7-dimethoxyquinolin-4-yloxy)phenyl]urea (Compound 20)

Compound A13 (41.7 mg) obtained according to Reference Example 3 was dissolved in 1,4-dioxane (2 mL), and triethylamine (0.047 mL) and diphenylphosphoryl azide (0.072 mL) were added to the resulting solution. The mixture was stirred at 60°C for 1 hour. 4-(6,7-Dimethoxyquinolin-4-yloxy)aniline (42.0 mg, 0.142 mmol) obtained according to Example 49 of WO 97/17329 was added to the resulting reaction mixture at room temperature, followed by stirring at 100°C for 10 hours. A sodium bicarbonate aqueous solution was added to the resulting reaction mixture, and extraction with ethyl acetate was performed. The organic phase was washed with a saturated brine solution, dried over anhydrous magnesium sulfate, and filtered. The solvent was distilled off from the filtrate under reduced pressure, and the residue was purified by silica gel column chromatography (ethyl acetate : methanol = 95:5 to 80:20) to give the titled compound 20 (12.5 mg, yield: 16%).
$^1$H-NMR ($CDCl_3$, 270 MHz) $\delta$: 11.02 (s, 1H), 8.47 (d, J = 5.3 Hz, 1H), 7.68-7.62 (m, 2H), 7.62-7.54 (m, 3H), 7.49 (s, 1H), 7.18-7.12 (m, 2H), 6.49 (d, J = 5.3 Hz, 1H), 4.07 (s, 3H), 4.06 (s, 3H), 3.97 (s, 3H), 1.46 (s, 9H).
ESI-MS: *m*/*z* 542$[M+H]^+$

Example 21

1-[2-tert-Butyl-4-(pyridin-4-yl)-1H-imidazol-5-yl]-3-[4-(6,7-dimethoxyquinolin-4-yloxy)phenyl]urea (Compound 21)

The titled compound 21 was obtained (yield: 44%) according to Example 3 from 2-tert-butyl-4-(pyridin-4-yl)-1H-imidazole-5-carboxylic acid (Compound A13-1) obtained according to Reference Example 3 and 4-(6,7-dimethoxyquinolin-4-yloxy)aniline obtained according to Example 49 of WO 97/17329.
$^1$H-NMR ($CDCl_3$, 270 MHz) $\delta$: 11.31 (s, 1H), 9.27 (s, 1H), 8.65-8.50 (m, 2H), 8.47 (d, J = 5.3 Hz, 1H), 7.68-7.60 (m, 2H), 7.59 (s, 1H), 7.42 (s, 1H), 7.40-7.25 (m, 2H), 7.20-7.12 (m, 2H), 6.47 (d, J = 5.3 Hz, 1H), 4.07 (s, 3H), 4.05 (s, 3H), 1.49 (s, 9H).

ESI-MS: *m/z* 539[M+H]$^+$

Example 22

1-[2-tert-Butyl-4-(1-methyl-1H-pyrazol-5-yl)-1H-imidazol-5-yl]-3-[4-(6,7-dimethoxyquinolin-4-yloxy)phenyl]urea (Compound 22)

The titled compound 22 was obtained (yield: 44%) according to Example 3 from 2-tert-butyl-4-(1-methyl-1H-pyrazol-5-yl)-1H-imidazole-5-carboxylic acid (Compound A13-2) obtained according to Reference Example 3 and 4-(6,7-dimethoxyquinolin-4-yloxy)aniline obtained according to Example 49 of WO 97/17329.
$^1$H-NMR ($CDCl_3$, 300 MHz) $\delta$: 11.28 (s, 1H), 8.91 (s, 1H), 8.47 (d, J = 5.1 Hz, 1H), 7.70-7.62 (m, 2H), 7.59 (s, 1H), 7.58 (d, J = 1.7 Hz, 1H), 7.43 (s, 1H), 7.20-7.12 (m, 2H), 6.47 (d, J = 5. 1 Hz, 1H), 6.38 (d, J = 1. 7 Hz, 1H), 6.34 (s, 1H), 4.06 (s, 3H), 4.05 (s, 3H), 3.78 (s, 3H), 1.49 (s, 9H).
ESI-MS: *m/z* 542[M+H]$^+$

Example 23

1-[2-tert-Butyl-4-(1-methyl-1H-pyrazol-5-yl)-1H-imidazol-5-yl]-3-[4-(6,7-dimethoxyquinolin-4-yloxy)-3-fluorophenyl]urea (Compound 23)

The titled compound 23 was obtained (yield: 28%) according to Example 3 from 2-tert-butyl-4-(1-methyl-1H-pyrazol-5-yl)-1H-imidazole-5-carboxylic acid (Compound A13-2) obtained according to Reference Example 3 and 4-(6,7-dimethoxyquinolin-4-yloxy)-3-fluoroaniline obtained according to Production Example 2 of WO 01/47890. ESI-MS: *m/z* 560 [M+H]$^+$

Example 24

1-[2-tert-Butyl-4-(3,5-dimethylisoxazol-4-yl)-1H-imidazol-5-yl]-3-[4-(6,7-dimethoxyquinolin-4-yloxy)phenyl]urea (Compound 24)

The titled compound 24 was obtained (yield: 43%) according to Example 3 from 2-tert-butyl-4-(3,5-dimethylisoxazol-4-yl)-1H-imidazole-5-carboxylic acid (Compound A13-3) obtained according to Reference Example 3 and 4-(6,7-dimethoxyquinolin-4-yloxy)aniline obtained according to Example 49 of WO 97/17329.
$^1$H-NMR (DMSO-$d_6$, 300 MHz) $\delta$: 11.51 (s, 1H), 10.10 (s, 1H), 8.45 (d, J = 5.5 Hz, 1H), 8.08 (s, 1H), 7.58-7.48 (m, 3H), 7.38 (s, 1H), 7.18-7.08 (m, 2H), 6.48 (d, J = 4.8 Hz, 1H), 3.95 (s, 3H), 3.93 (s, 3H), 2.28 (s, 3H), 2.12 (s, 3H), 1.36 (s, 9H).
ESI-MS: *m/z* 557 [M+H]$^+$

Example 25

1-[2-tert-Butyl-4-(pyridin-3-yl)-1H-imidazol-5-yl]-3-[4-(6,7-dimethoxyquinolin-4-yloxy)phenyl]urea (Compound 25)

The titled compound 25 was obtained (yield: 74%) according to Example 3 from 2-tert-butyl-4-(pyridin-3-yl)-1H-imidazole-5-carboxylic acid (Compound A13-4) obtained according to Reference Example 3 and 4-(6,7-dimethoxyquinolin-4-yloxy)aniline obtained according to Example 49 of WO 97/17329.
$^1$H-NMR (DMSO-$d_6$, 300 MHz) $\delta$: 11.65 (s, 1H), 8.84 (s, 1H), 8.50-8.30 (m, 2H), 8.20-7.90 (m, 2H), 7.65-7.43 (m, 3H), 7.45-7.25 (m, 2H), 7.20-7.06 (m, 2H), 6.48 (m, 1H), 3.95 (s, 3H), 3.93 (s, 3H), 1.38 (s, 9H).
ESI-MS: *m/z* 539[M+H]$^+$

Example 26

1-[2-tert-Butyl-4-(2-chloropyridin-3-yl)-1H-imidazol-5-yl]-3-[4-(6,7-dimethoxyquinolin-4-yloxy)phenyl]urea (Compound 26)

The titled compound 26 was obtained (yield: 65%) according to Example 3 from 2-tert-butyl-4-(2-chloropyridin-3-yl)-1H-imidazole-5-carboxylic acid (Compound A13-5) obtained according to Reference Example 3 and 4-(6,7-dimethoxyquinolin-4-yloxy)aniline obtained according to Example 49 of WO 97/17329.
$^1$H-NMR (DMSO-$d_6$, 300 MHz) $\delta$: 11.68 (s, 1H), 9.95 (s, 1H), 8.45 (d, J = 5.1 Hz, 1H), 8.34 (s, 1H), 8.16 (s, 1H), 7.95-7.82 (m, 2H), 7.56-7.46 (m, 3H), 7.43 (dd, J = 4.2, 7.5 Hz, 1H), 7.38 (s, 1H), 7.20-7.10 (m, 2H), 6.48 (d, J = 5.1 Hz, 1H), 3.94

(s, 3H), 3.92 (s, 3H), 1.38 (s, 9H). ESI-MS: *m/z* 573[M+H]+

Example 27

Ethyl 2-[4-(4-{3.-[2-tert-butyl-4-(1-methyl-1H-pyrazol-5-yl)-lH-imidazol-5-yl]ureido}phenoxy)-6-methoxyquinolin-7-yloxy] acetate (Compound 27)

2-tert-Butyl-4-(1-methyl-1H-pyrazol-5-yl)-1H-imidazole-5-carboxylic acid (Compound A13-2, 30 mg) obtained according to Reference Example 3 was dissolved in chloroform (3 mL), and triethylamine (0.016 mL) and diphenylphosphoryl azide (0.026 mL) were added to the solution, followed by stirring at 50°C for 1 hour. Compound A15 (30 mg) obtained according to Reference Example 4 was added to the resulting reaction mixture, followed by stirring at 50°C overnight. Water was added to the resulting reaction mixture, and extraction with chloroform was performed. The organic phase was washed with water and saturated brine, dried over anhydrous magnesium sulfate, and filtered. The solvent was distilled off from the filtrate under reduced pressure, and the resulting residue was purified by silica gel column chromatography (chloroform : methanol = 97:3 to 93:7) to give the titled compound 27 (8 mg, yield: 16%).

Example 28

1-[2-tert-Butyl-4-(1-methyl-1H-pyrazol-5-yl)-1H-imidazol-5-yl]-3-{4-[7-(2-hydroxyethoxy)-6-methoxyquinolin-4-yloxy] phenyl}urea (Compound 28)

Sodium borohydride (0.8 mg) was suspended in THF (1 mL), and Compound 27 (8 mg) obtained according to Example 27 was added to the suspension, followed by stirring at room temperature overnight. Water (0.1 mL) and sodium borohydride (0.8 mg) were further added to the reaction mixture, followed by stirring at room temperature. Water was added to the resulting reaction mixture, and extraction with ethyl acetate was performed. The organic phase was washed with saturated brine, dried over anhydrous magnesium sulfate, and filtered. The solvent was distilled off from the filtrate under reduced pressure, and the resulting residue was purified by silica gel column chromatography (chloroform : methanol = 20:1) to give the titled compound 28 (1.3 mg, yield: 18%).
$^1$H-NMR ($CDCl_3$, 300 MHz) δ:11.32 (s, 1H), 9.25 (s, 1H), 8.46 (d, J = 5.3 Hz, 1H), 7.61 (dd, J = 17.3, 7.1 Hz, 4H), 7.43 (s, 1H), 7.16 (dd, J = 6.8, 2.1 Hz, 2H), 6.47 (d, J = 5.3 Hz, 2H), 6.37 (s, 1H), 4.30 (t, J = 4.5 Hz, 2H), 4.09 (t, J = 4.6 Hz, 2H), 4.04 (s, 3H), 3.75 (s, 3H), 1.48 (s, 9H), 1.25 (s, 3H)
ESI-MS: *m/z* 572[M+H]+

Example 29

1-[2-tert-Butyl-4-(4-fluorophenyl)-1-methyl-1H-imidazol-5-yl]-3-[5-(6,7-dimethoxyquinolin-4-yloxy)pyridin-2-yl]urea (Compound 29)

The titled compound 29 was obtained (yield: 23%) according to Example 3 from Compound A17 obtained according to Reference Example 5 and 2-tert-butyl-4-(4-fluorophenyl)-1-methyl-1H-imidazole-5-carboxylic acid (Compound A6-6) obtained according to Reference Example 2.
ESI-MS: *m/z* 571[M+H]+

Example 30

1-[2-tert-Butyl-4-(3-fluorophenyl)-1-methyl-1H-imidazol-5-yl]-3-[5-(6,7-dimethoxyquinolin-4-yloxy)pyridin-2-yl]urea (Compound 30)

The titled compound 30 was obtained (yield: 50%) according to Example 3 from Compound A17 obtained according to Reference Example 5 and 2-tert-butyl-4-(3-fluorophenyl)-1-methyl-1H-imidazole-5-carboxylic acid (Compound A6-2) obtained according to Reference Example 2.
ESI-MS: *m/z* 571[M+H]+

Example 31

1-(2-tert-Butyl-1-methyl-4-phenyl-1H-imidazol-5-yl)-3-[5-(6,7-dimethoxyquinolin-4-yloxy)pyridin-2-yl]urea

(Compound 31)

The titled compound 31 was obtained (yield: 34%) according to Example 3 from Compound A17 obtained according to Reference Example 5 and Compound A6 obtained according to Reference Example 2.
ESI-MS: *m*/*z* 553[M+H]$^+$

Example 32

1-[2-tert-Butyl-4-(2-fluorophenyl)-1-methyl-1H-imidazol-5-yl]-3-[5-(6,7-dimethoxyquinolin-4-yloxy)pyridin-2-yl]urea (Compound 32)

The titled compound 32 was obtained (yield: 84%) according to Example 3 from Compound A17 obtained according to Reference Example 5 and 2-tert-butyl-4-(2-fluorophenyl)-1-methyl-1H-imidazole-5-carboxylic acid (Compound A6-3) obtained according to Reference Example 2.
ESI-MS: *m*/*z* 571[M+H]$^+$

Example 33

(E)-1-{2-tert-Butyl-4-[2-(pyridin-4-yl)vinyl]-1H-imidazol-5-yl}-3-[4-(6,7-dimethoxyquinolin-4-yloxy)phenyl]urea (Compound 33)

Compound A20 (38.0 mg) obtained according to Reference Example 7 and 4-(6,7-dimethoxyquinolin-4-yloxy) aniline obtained according to Example 49 of WO 97/17329 (62.9 mg) were suspended in chloroform (1.5 mL), followed by stirring at 50°C overnight. The reaction mixture was cooled to room temperature, and the solvent was distilled off under reduced pressure. The residue was purified by preparative thin-layer chromatography using chloroform and methanol as the eluents to give the titled compound 33 (28.3 mg, yield: 35%). $^1$H-NMR (DMSO-$d_6$, 270 MHz): $\delta$ 11.92 (s, 1H), 11.48 (s, 1H), 9.40 (s, 1H), 8.52 - 8.42 (m, 3H), 7.81 (d, J = 16.5 Hz, 1H), 7.69 - 7.61 (m, 2H), 7.53 (s, 1H), 7.43 - 7.38 (m, 3H), 7.30 - 7.22 (m, 2H), 6.78 (d, J = 16.1 Hz, 1H), 6.46 (dd, J = 1.0, 5.3 Hz, 1H), 3.95 (s, 3H), 3.95 (s, 3H), 1.41 (s, 9H) ESI-MS: *m*/*z* 565[M+H]$^+$

Example 34

1-[2-tert-Butyl-4-(phenylethynyl)-1H-imidazol-4-yl]-3-[4-(6,7-dimethoxyquinolin-5-yloxy)phenyl]urea (Compound 34)

Compound A23 (64.5 mg) obtained according to Reference Example 8 and 4-(6,7-dimethoxyquinolin-4-yloxy) aniline obtained according to Example 49 of WO 97/17329 (86 mg) were suspended in chloroform (1.4 mL), followed by stirring at 50°C overnight. The reaction mixture was cooled to room temperature, and the solvent was distilled off under reduced pressure. The residue was purified by preparative thin-layer chromatography using hexane and acetone as the eluents to give the titled compound 34 (15.1 mg, yield: 11%). $^1$H-NMR ($CDCl_3$, 300 MHz): $\delta$ 11.34 (br s, 1H), 9.90 - 9.60 (m, 1H), 8.48 (d, J = 5.5 Hz, 1H), 7.71 - 7.63 (m, 2H), 7.60 (s, 1H), 7.43 (s, 1H), 7.42 - 7.26 (m, 5H), 7.19 - 7.10 (m, 2H), 6.88 (br s, 1H), 6.47 (d, J = 5.1 Hz, 1H), 4.06 (s, 3H), 4.04 (s, 3H), 1.45 (s, 9H)
ESI-MS: *m*/*z* 562[M+H]$^+$

Example 35

1-[2-tert-Butyl-4-(4-methylthiazol-5-yl)-1H-imidazol-5-yl]-3-[4-(6,7-dimethoxyquinolin-4-yloxy)phenyl]urea

(Compound 35)

The titled compound 35 was obtained (yield: 34%) according to Example 3 from 2-tert-butyl-5-(4-methylthiazol-5-yl)-1H-imidazole-4-carboxylic acid hydrochloride (Compound A26-1) obtained according to Reference Example 9 and 4-(6,7-dimethoxyquinolin-4-yloxy)aniline obtained according to Example 49 of WO 97/17329.
$^1$H-NMR ($CDCl_3$, 300 MHz): $\delta$ 11.30 (s, 1H), 8.87 (br s, 1H), 8.82 (s, 1H), 7.47 (d, J = 5.1 Hz, 1H), 7.70 - 7.62 (m, 2H), 7.59 (s, 1H), 7.41 (s, 1H), 7.19 - 7.12 (m, 2H), 6.47 (d, J = 5.5 Hz, 1H), 6.28 (s, 1H), 4.06 (s, 3H), 4.05 (s, 3H), 2.45 (s,

3H), 1.48 (s, 9H)
ESI-MS: m/z 559[M+H]$^+$

Example 36

1-[2-tert-Butyl-4-(2,6-difluoropyridin-3-yl)-1H-imidazol-5-yl]-3-[4-(6,7-dimethoxyquinolin-4-yloxy)phenyl]urea (Compound 36)

The titled compound 36 was obtained (yield: 33%) according to Example 3 from 2-tert-butyl-5-(2,6-difluoropyridin-3-yl)-1H-imidazole-4-carboxylic acid (Compound A13-6) obtained according to Reference Example 3 and 4-(6,7-dimethoxyquinolin-4-yloxy)aniline obtained according to Example 49 of WO 97/17329.
$^1$H-NMR ($CDCl_3$, 270 MHz): δ 11.10 (br s, 1H), 9.09 (br s, 1H), 8.48 (d, J = 5.3 Hz, 1H), 8.18 - 8.04 (m, 1H), 7.63 - 7.54 (m, 3H), 7.42 (s, 1H), 7.20 - 7.11 (m, 2H), 7.06 (br s, 1H), 6.95 (dd, J = 2.6, 8.2 Hz, 1H), 6.47 (d, J = 4.9 Hz, 1H), 4.06 (s, 3H), 4.05 (s, 3H), 1.48 (s, 9H)
ESI-MS: *m/z* 575[M+H]$^+$

Example 37

1-[2-tert-Butyl-4-(2,6-dichloropyridin-3-yl)-1H-imidazol-5-yl]-3-[4-(6,7-dimethoxyquinolin-4-yloxy)phenyl]urea (Compound 37)

The titled compound 37 was obtained (yield: 11%) according to Example 3 from 2-tert-butyl-5-(2,6-dichloropyridin-3-yl)-1H-imidazole-4-carboxylic acid (Compound A13-7) obtained according to Reference Example 3 and 4-(6,7-dimethoxyquinolin-4-yloxy)aniline obtained according to Example 49 of WO 97/17329.
$^1$H-NMR ($CDCl_3$, 270 MHz): δ 11.23 (br s, 1H), 9.08 (br s, 1H), 8.48 (d, J = 5.1 Hz, 1H), 7.82 (d, J = 8.1 Hz, 1H), 7.65 - 7.57 (m, 3H), 7.42 (s, 1H), 7.37 (d, J = 8.1 Hz, 1H), 7.21 - 7.12 (m, 2H), 6.70 (br s, 1H), 6.47 (d, J = 5.1 Hz, 1H), 4.06 (s, 3H), 4.05 (s, 3H), 1.49 (s, 9H)
ESI-MS: *m/z* 607[M+H]$^+$

Example 38

1-[2-tert-Butyl-4-(2-chloro-5-methylpyridin-3-yl)-1H-imidazol-5-yl]-3-[4-(6,7-dimethoxyquinolin-4-yloxy)phenyl]urea (Compound 38)

The titled compound 38 was obtained (yield: 18%) according to Example 3 from 2-tert-butyl-5-(2-chloro-5-methylpyridin-3-yl)-1H-imidazole-4-carboxylic acid (Compound A13-8) obtained according to Reference Example 3 and 4-(6,7-dimethoxyquinolin-4-yloxy)aniline obtained according to Example 49 of WO 97/17329.
$^1$H-NMR ($CDCl_3$, 270 MHz): δ 11.35 (br s, 1H), 9.08 (br s, 1H), 8.48 (d, J = 5.3 Hz, 1H), 8.19 (s, 1H), 7.69 - 7.56 (m, 4H), 7.42 (s, 1H), 7.20 - 7.11 (m, 2H), 6.59 (br s, 1H), 6.48 (d, J = 5.3 Hz, 1H), 4.07 (s, 3H), 4.05 (s, 3H), 2.38 (s, 3H), 1.49 (s, 9H)
ESI-MS: *m/z* 587[M+H]$^+$

Example 39

1-[2-tert-Butyl-4-(2-chloro-6-methylpyridin-3-yl)-1H-imidazol-5-yl]-3-[4-(6,7-dimethoxyquinolin-4-yloxy)phenyl]urea (Compound 39)

The titled compound 39 was obtained (yield: 36%) according to Example 3 from 2-tert-butyl-5-(2-chloro-6-methylpyridin-3-yl)-1H-imidazole-4-carboxylic acid (Compound A13-9) obtained according to Reference Example 3 and 4-(6,7-dimethoxyquinolin-4-yloxy)aniline obtained according to Example 49 of WO 97/17329.
$^1$H-NMR ($CDCl_3$, 270 MHz): δ 11.29 (br s, 1H), 9.09 (br s, 1H), 8.48 (d, J = 5.3 Hz, 1H), 7.72 (d, J = 7.9 Hz, 1H), 7.68 - 7.56 (m, 3H), 7.42 (s, 1H), 7.23 - 7.11 (m, 3H), 6.54 (s, 1H), 6.47 (d, J = 5.3 Hz, 1H), 4.06 (s, 3H), 4.05 (s, 3H), 2.58 (s, 3H), 1.48 (s, 9H)
ESI-MS: *m/z* 587[M+H]$^+$

Example 40

1-[2-tert-Butyl-4-(2-fluoropyridin-3-yl)-1H-imidazol-5-yl]-3-[4-(6,7-dimethoxyquinolin-4-yloxy)phenyl]urea (Compound 40)

The titled compound 40 was obtained (yield: 54%) according to Example 3 from 2-tert-butyl-5-(2-fluoropyridin-3-yl)-1H-imidazole-4-carboxylic acid (Compound A13-10) obtained according to Reference Example 3 and 4-(6,7-dimethoxyquinolin-4-yloxy)aniline obtained according to Example 49 of WO 97/17329.
$^1$H-NMR ($CDCl_3$, 270 MHz): δ 11.19 (br s, 1H), 9.12 (br s, 1H), 8.48 (d, J = 5.3 Hz, 1H), 8.16 - 7.92 (m, 2H), 7.68 - 7.57 (m, 3H), 7.43 (s, 1H), 7.35 - 7.25 (m, 1H), 7.20 - 7.12 (m, 2H), 6.82 (br s, 1H), 6.47 (d, J = 5.3 Hz, 1H), 4.06 (s, 3H), 4.05 (s, 3H), 1.49 (s, 9H)
ESI-MS: *m*/*z* 557$[M+H]^+$

Example 41

1-[2-tert-Butyl-4-(2-chloropyridin-3-yl)-1H-imidazol-5-yl]-3-[4-(6,7-dimethoxyquinolin-4-yloxy)-3-fluorophenyl]urea (Compound 41)

The titled compound 41 was obtained (yield: 26%) according to Example 3 from 2-tert-butyl-4-(2-chloropyridin-3-yl)-1H-imidazole-5-carboxylic acid (Compound A13-5) obtained according to Reference Example 3 and 4-(6,7-dimethoxyquinolin-4-yloxy)-3-fluoroaniline obtained according to Production Example 2 of WO 01/47890. $^1$H-NMR ($CDCl_3$, 300MHz) δ: 11.46 (br s, 1H), 9.09 (br s, 1H), 8.49 (d, J = 5.1 Hz, 1H), 8.39 (dd, J = 4.6, 2.0 Hz, 1H), 7.83 (dd, J = 7.5, 2.0 Hz, 1H), 7.72 (dd, J = 12.5, 2.2 Hz, 1H), 7.61 (s, 1H), 7.43 (s, 1H), 7.37 (dd, J = 7.7, 4.8 Hz, 1H), 7.25-7.20 (m, 2H), 6.60 (br s, 1H), 6.44 (dd, J = 5.1, 1.1 Hz, 1H), 4.08 (s, 3H), 4.05 (s, 3H), 1.50 (s, 9H). ESI-MS: *m*/*z* 591$[M+H]^+$

Example 42

1-[2-tert-Butyl-4-(3-methylisoxazol-4-yl)-1H-imidazol-5-yl]-3-[4-(6,7-dimethoxyquinolin-4-yloxy)phenyl]urea (Compound 42)

The titled compound 42 was obtained (yield: 22%) according to Example 3 from 2-tert-butyl-5-(3-methylisoxazol-4-yl)-1H-imidazole-4-carboxylic acid hydrochloride (Compound A26-2) obtained according to Reference Example 9 and 4-(6,7-dimethoxyquinolin-4-yloxy)aniline obtained according to Example 49 of WO 97/17329.
$^1$H-NMR ($CDCl_3$, 270 MHz): δ 11. 20 (br s, 1H), 8.99 (br s, 1H), 8.50 - 8.43 (m, 2H), 7.63 - 7.54 (m, 3H), 7.40 (s, 1H), 7.20- 7.11 (m, 2H), 6. 94 - 6.76 (m, 1H), 6.47 (d, J = 5.3 Hz, 1H), 4.06 (s, 3H), 4.04 (s, 3H), 2.30 (s, 3H), 1.46 (s, 3H)
ESI-MS: *m*/*z* 543$[M+H]^+$

Example 43

1-[2-tert-Butyl-4-(6-fluoropyridin-3-yl)-1H-imidazol-5-yl]-3-[4-(6,7-dimethoxyquinolin-4-yloxy)phenyl]urea (Compound 43)

The titled compound 43 was obtained (yield: 26%) according to Example 3 from 2-tert-butyl-5-(6-fluoropyridin-3-yl)-1H-imidazole-4-carboxylic acid (Compound A13-11) obtained according to Reference Example 3 and 4-(6,7-dimethoxyquinolin-4-yloxy)aniline obtained according to Example 49 of WO 97/17329.
$^1$H-NMR ($CDCl_3$, 270 MHz): δ 11.19 (br s, 1H), 9.88 (br s, 1H), 8.44 (d, J = 5.3 Hz, 1H), 8.28 (s, 1H), 7.98 - 7.85 (m, 1H), 7.62 - 7.34 (m, 5H), 7.16 - 7.08 (m, 2H), 6.90 (dd, J =2.6, 8.2 Hz, 1H), 6.44 (d, J = 5.3 Hz, 1H), 4.05 (s, 3H), 4.01 (s, 3H), 1.47 (s, 9H)
ESI-MS: *m*/*z* 557$[M+H]^+$

Example 44

1-[2-tert-Butyl-4-(2-chloropyridin-3-yl)-1H-imidazol-5-yl]-3-[2-chloro-4-(6,7-dimethoxyquinolin-4-yloxy)phenyl]urea (Compound 44)

The titled compound 44 was obtained (yield: 5%) according to Example 3 from 2-tert-butyl-4-(2-chloropyridin-3-yl)-1H-imidazole-5-carboxylic acid (Compound A13-5) obtained according to Reference Example 3 and 2-chloro-4-(6,7-dimethoxyquinolin-4-yloxy)aniline obtained according to Production Example 1 of WO 00/43366.

$^1$H-NMR ($CDCl_3$) δ: 11.16 (br s, 1H), 9.07 (br s, 1H), 8.51 (d, J = 5.5 Hz, 1H), 8.44 - 8.38 (m, 2H), 7.86 (dd, J = 7.7, 1.8 Hz, 1H), 7.54 (s, 1H), 7.46 (s, 1H), 7.37 (dd, J = 7.7, 4.8 Hz, 1H), 7.27 (m, 1H), 7.13 (dd, J = 9.0, 2.7 Hz, 1H), 6.62 (s, 1H), 6.53 (d, J = 5.5 Hz, 1H), 4.06 (s, 6H), 1.48 (s, 9H).
ESI-MS: *m/z* 607[M+H]$^+$

Example 45

1-[2-tert-Butyl-4-(3,5-dimethylisoxazol-4-yl)-1H-imidazol-5-yl]-3-[4-(6,7-dimethoxyquinolin-4-yloxy)-2-methylphenyl] urea (Compound 45)

The titled compound 45 was obtained (yield: 28%) according to Example 3 from 2-tert-butyl-4-(3,5-dimethylisoxazol-4-yl)-1H-imidazole-5-carboxylic acid (Compound A13-3) obtained according to Reference Example 3 and 4-(6,7-dimethoxyquinolin-4-yloxy)-2-methylaniline obtained according to Production Example 6 of JP-A-11-158149. $^1$H-NMR ($CDCl_3$) δ: 10.60 (br s, 1H), 8.83 (br s, 1H), 8.47 (d, J = 5.1 Hz, 1H), 8.12 (d, J = 8.4 Hz, 1H), 7.58 (s, 1H), 7.42 (s, 1H), 7.11 - 7.03 (m, 2H), 6.50 (d, J = 5.1 Hz, 1H), 6.26 (s, 1H), 4.06 (s, 3H), 4.05 (s, 3H), 2.48 (s, 3H), 2.38 (s, 3H), 2.20 (s, 3H), 1.44 (s, 9H).
ESI-MS: *m/z* 572[M+H]$^+$

Example 46

1-[2-tert-Butyl-4-(3,5-dimethylisoxazol-4-yl)-1H-imidazol-5-yl]-3-[3-chloro-4-(6,7-dimethoxyquinolin-4-yloxy)phenyl] urea (Compound 46)

The titled compound 46 was obtained (yield: 9%) according to Example 3 from 2-tert-butyl-4-(3,5-dimethylisoxazol-4-yl)-1H-imidazole-5-carboxylic acid (Compound A13-3) obtained according to Reference Example 3 and Compound A18 obtained according to Reference Example 6. $^1$H-NMR ($CDCl_3$) δ: 11.43 (br s, 1H), 8.47 (d, J = 5.6 Hz, 1H), 7.89 (d, J = 2.6 Hz, 1H), 7.64 (s, 1H), 7.56 (s, 1H), 7.50 (dd, J = 8.8, 2.5 Hz, 1H), 7.29 - 7.20 (m, 2H), 6.40 (d, J = 5.6 Hz, 1H), 6.27 (br s, 1H), 4.09 (s, 3H), 4.07 (s, 3H), 2.38 (s, 3H), 2.20 (s, 3H), 1.47 (s, 9H).
ESI-MS: *m/z* 591[M+H]$^+$

Example 47

1-[2-tert-Butyl-4-(3,5-dimethylisoxazol-4-yl)-1H-imidazol-5-yl]-3-[4-(6,7-dimethoxyquinolin-4-yloxy)-3-fluorophenyl]urea (Compound 47)

The titled compound 47 was obtained (yield: 15%) according to Example 3 from 2-tert-butyl-4-(3,5-dimethylisoxazol-4-yl)-1H-imidazole-5-carboxylic acid (Compound A13-3) obtained according to Reference Example 3 and 4-(6,7-dimethoxyquinolin-4-yloxy)-3-fluoroaniline obtained according to Production Example 2 of WO 01/47890. $^1$H-NMR ($CDCl_3$) δ: 8.47 (d, J = 5.6 Hz, 1H), 7.72 (dd, J = 12.2, 2.3 Hz, 1H), 7.63 (s, 1H), 7.59 (s, 1H), 7.31-7.22 (m, 2H), 6.53 (d, J = 5.6 Hz, 1H), 4.09 (s, 3H), 4.08 (s, 3H), 2.37 (s, 3H), 2.19 (s, 3H), 1.48 (s, 9H).
ESI-MS: *m/z* 575[M+H]$^+$

Example 48

1-[2-tert-Butyl-4-(3,5-dimethylisoxazol-4-yl)-1H-imidazol-5-yl]-3-[2-chloro-4-(6,7-dimethoxyquinolin-4-yloxy)phenyl] urea (Compound 48)

The titled compound 48 was obtained (yield: 6%) according to Example 3 from 2-tert-butyl-4-(3,5-dimethylisoxazol-4-yl)-1H-imidazole-5-carboxylic acid (Compound A13-3) obtained according to Reference Example 3 and 2-chloro-4-(6,7-dimethoxyquinolin-4-yloxy)aniline obtained according to Production Example 1 of WO 00/43366. $^1$H-NMR ($CDCl_3$) δ: 11.14 (br s, 1H), 8.71 (br s, 1H), 8.51 (d, J = 5.1 Hz, 1H), 8.42 (d, J = 8.8 Hz, 1H), 7.54 (s, 1H), 7.43 (s, 1H), 7.27-7.26 (m, 1H), 7.13 (dd, J = 8.8, 2.6 Hz, 1H), 6.51 (d, J = 5.1 Hz, 1H), 6.35 (br s, 1H), 4.05 (s, 6H), 2.38 (s, 3H), 2.20 (s, 3H), 1.46 (s, 9H).
ESI-MS: *m/z* 591[M+H]$^+$

Example 49

1-[2-tert-Butyl-4-(3,5-dimethylisoxazol-4-yl)-1H-imidazol-5-yl]-3-[4-(6,7-dimethoxyquinolin-4-yloxy)-2-fluorophenyl]urea (Compound 49)

The titled compound 49 was obtained (yield: 6%) according to Example 3 from 2-tert-butyl-4-(3,5-dimethylisoxazol-4-yl)-1H-imidazole-5-carboxylic acid (Compound A13-3) obtained according to Reference Example 3 and 4-(6,7-dimethoxyquinolin-4-yloxy)-2-fluoroaniline obtained according to J. Med. Chem., 2005, 48(5), 1359-1366. $^1$H-NMR ($CDCl_3$) $\delta$: 11.56 (br s, 1H), 8.77 (br s, 1H), 8.50 (d, J = 5.3 Hz, 1H), 8.41 (t, J = 9.3 Hz, 1H), 7.55 (s, 1H), 7.44 (s, 1H), 7.04-6.96 (m, 2H), 6.54 (br s, 1H), 6.52 (d, J = 5.3 Hz, 1H), 4.06 (s, 3H), 4.05 (s, 3H), 2.37 (s, 3H), 2.19 (s, 3H), 1.45 (s, 9H). ESI-MS: *m*/*z* 575$[M+H]^+$

Example 50

1-[2-tert-Butyl-4-(2-methylthiazol-5-yl)-1H-imidazol-5-yl]-3-[4-(6,7-dimethoxyquinolin-4-yloxy)phenyl]urea (Compound 50)

The titled compound 50 was obtained (yield: 15%) according to Example 3 from 2-tert-butyl-5-(2-methylthiazol-5-yl)-1H-imidazole-4-carboxylic acid hydrochloride (Compound A26-3) obtained according to Reference Example 9 and 4-(6,7-dimethoxyquinolin-4-yloxy)aniline obtained according to Example 49 of WO 97/17329.
$^1$H-NMR ($CDCl_3$, 300 MHz): $\delta$11.11 (br s, 1H), 9.75 - 9.25 (m, 1H), 8.47 (d, J = 5.5 Hz, 1H), 7.67 - 7.55 (m, 4H), 7.41 (s, 1H), 7.18 - 7.11 (m, 2H), 6.65 (br s, 1H), 6.47 (d, J = 5.1 Hz, 1H), 4.06 (s, 3H), 4.04 (s, 3H), 2.71 (s, 3H), 1.45 (s, 9H)
ESI-MS: *m*/*z* 559$[M+H]^+$

Example 51

1-[2-tert-Butyl-4-(thiazol-5-yl)-1H-imidazol-5-yl]-3-[4-(6,7-dimethoxyquinolin-4-yloxy)phenyl]urea (Compound 51)

Hydrochloric acid (3 mol/L) was added to Compound A26 (33.0 mg) obtained according to Reference Example 9, followed by stirring at 70°C for 1 hour. The reaction mixture was cooled to room temperature, and the solvent was distilled off under reduced pressure. The resulting residue was dissolved in anhydrous DMF (2 mL). Triethylamine (27.1 mg) and diphenylphosphoryl azide (73.6 mg) were added to the solution, followed by stirring at 50°C for 1 hour under an argon atmosphere. 4-(6,7-Dimethoxyquinolin-4-yloxy)aniline (47.6 mg) obtained according to Example 49 of WO 97/17329 was added to the mixture, followed by further stirring at 50°C overnight. The reaction mixture was cooled to room temperature, and water was added thereto. After extraction with ethyl acetate, washing with water and saturated brine, drying over anhydrous sodium sulfate, and filtration was performed. The solvent was distilled off from the filtrate under reduced pressure, and the resulting residue was purified by preparative thin-layer chromatography using chloroform and methanol as the eluents. The resulting crude purified product was dissolved in ethyl acetate and extracted with a 10% citric aqueous solution. The aqueous phase was washed with ethyl acetate and was made to be basic with sodium carbonate, followed by extraction with ethyl acetate, washing with saturated brine, drying over anhydrous sodium sulfate, and filtration. The solvent was distilled off from the filtrate under reduced pressure to give the titled compound 51 (9.8 mg, 2-step yield: 17 %). $^1$H-NMR ($CDCl_3$, 300 MHz): $\delta$10.22 (br s, 1H), 8.74 (br s, 1H), 8.46 (d, J = 5.5 Hz, 1H), 7.87 (s, 1H), 7.65 - 7.56 (m, 3H), 7.39 (s, 1H), 7.19 - 7.11 (m, 2H), 7.00 - 6.60 (m, 1H), 6.46 (d, J = 5.1 Hz, 1H), 4.05 (s, 3H), 4.02 (s, 3H), 1.46 (s, 9H)
ESI-MS: *m*/*z* 545$[M+H]^+$

Example 52

1-[2-tert-Butyl-4-(4-methyloxazol-5-yl)-1H-imidazol-5-yl]-3-[4-(6,7-dimethoxyquinolin-4-yloxy)phenyl]urea (Compound 52)

The titled compound 52 was obtained (yield: 19%) according to Example 3 from 2-tert-butyl-5-(4-methyloxazol-5-yl)-1H-imidazole-4-carboxylic acid hydrochloride (Compound A26-4) obtained according to Reference Example 9 and 4-(6,7-dimethoxyquinolin-4-yloxy)aniline obtained according to Example 49 of WO 97/17329.
$^1$H-NMR ($CDCl_3$, 270 MHz): $\delta$11.35 (br s, 1H), 9.51 (br s, 1H), 8.46 (d, J = 5.3 Hz, 1H), 7.80 (br s, 1H), 7.70 - 7.56 (m, 3H), 7.40 (s, 1H), 7.21 - 7.12 (m, 2H), 6.78 (br s, 1H), 6.47 (d, J = 5.3 Hz, 1H), 4.06 (s, 3H), 4.03 (s, 3H), 2.29 (s, 3H), 1.47 (s, 9H)
ESI-MS: *m*/*z* 543$[M+H]^+$

Example 53

1-[2-tert-Butyl-4-(5-chloropyridin-3-yl)-1H-imidazol-5-yl]-3-[4-(6,7-dimethoxyquinolin-4-yloxy)phenyl]urea (Compound 53)

The titled compound 53 was obtained (yield: 22%) according to Example 3 from 2-tert-butyl-4-(5-chloropyridin-3-yl)-1H-imidazole-5-carboxylic acid (Compound A13-12) obtained according to Reference Example 3 and 4-(6,7-dimethoxyquinolin-4-yloxy)aniline obtained according to Example 49 of WO 97/17329.
$^1$H-NMR ($CDCl_3$) δ: 11.23 (br s, 1H), 8.95 (br s, 1H), 8.60 (s, 1H), 8.48 (m, 2H), 7.74 (s, 1H), 7.65-7.59 (m, 3H), 7.45 (s, 1H), 7.16 (d, J = 8.8 Hz, 2H), 6.79 (br s, 1H), 6.48 (d, J = 5.5 Hz, 1H), 4.06 (s, 3H), 4.05 (s, 3H), 1.49 (s, 9H).
ESI-MS: *m*/*z* 607[M+H]$^+$

Example 54

1-[2-tert-Butyl-4-(2-methylpyridin-4-yl)-1H-imidazol-5-yl]-3-[4-(6,7-dimethoxyquinolin-4-yloxy)phenyl]urea (Compound 54)

The titled compound 54 was obtained (yield: 4%) according to Example 3 from 2-tert-butyl-4-(2-methylpyridin-4-yl)-1H-imidazole-5-carboxylic acid (Compound A13-13) obtained according to Reference Example 3 and 4-(6,7-dimethoxyquinolin-4-yloxy)aniline obtained according to Example 49 of WO 97/17329.
$^1$H-NMR ($CDCl_3$) δ: 11.33 (br s, 1H), 8.91 (br s, 1H), 8.52-8.47 (m, 2H), 7.65 (d, J = 8.9 Hz, 2H), 7.59 (s, 1H), 7.43 (s, 1H), 7.18-7.09 (m, 4H), 6.80 (br s, 1H), 6.48 (d, J = 5.3 Hz, 1H), 4.06 (s, 3H), 4.05 (s, 3H), 2.60 (s, 3H), 1.49 (s, 9H).
ESI-MS: *m*/*z* 573 [M+H]$^+$

Example 55

1-[2-tert-Butyl-4-(3-chloropyridin-4-yl)-1H-imidazol-5-yl]-3-[4-(6,7-dimethoxyquinolin-4-yloxy)phenyl]urea (Compound 55)

The titled compound 55 was obtained (yield: 32%) according to Example 3 from 2-tert-buty-5-(3-chloropyridin-4-yl)-1H-imidazole-4-carboxylic acid (Compound A13-14) obtained according to Reference Example 3 and 4-(6,7-dimethoxyquinolin-4-yloxy)aniline obtained according to Example 49 of WO 97/17329.
$^1$H-NMR ($CDCl_3$, 300 MHz): δ11.39 (br s, 1H), 9.75 - 9.35 (m, 1H), 8.68 - 8.59 (m, 1H), 8.54 - 8.43 (m, 2H), 7.66 - 7.56 (m, 3H), 7.44 - 7.38 (m, 1H), 7.20 - 6.92 (m, 3H), 6.47 (d, J = 5.5 Hz, 1H), 4.07 (s, 3H), 4.04 (s, 3H), 1.49 (s, 9H). ESI-MS: *m*/*z* 573[M+H]$^+$

Example 56

1-[2-tert-Butyl-4-(6-chloropyridin-3-yl)-1H-imidazol-5-yl]-3-[4-(6,7-dimethoxyquinolin-4-yloxy)phenyl]urea (Compound 56)

The titled compound 56 was obtained (yield: 26%) according to Example 3 from 2-tert-butyl-4-(6-chloropyridin-3-yl)-1H-imidazole-5-carboxylic acid (Compound A13-15) obtained according to Reference Example 3 and 4-(6,7-dimethoxyquinolin-4-yloxy)aniline obtained according to Example 49 of WO 97/17329.
$^1$H-NMR ($CDCl_3$) δ: 11.17 (br s, 1H), 9.18 (br s, 1H), 8.47 (d, J = 5.5 Hz, 2H), 7.75 (br s, 1H), 7.61-7.58 (m, 3H), 7.41-7.35 (m, 2H), 7.15 (d, J = 8.8 Hz, 2H), 6.96 (br s, 1H), 6.46 (d, J = 5.5 Hz, 1H), 4.06 (s, 3H), 4.04 (s, 3H), 1.48 (s, 9H).
ESI-MS: *m*/*z* 573[M+H]$^+$

Example 57

1-{2-tert-Butyl-4-[2-(dimethylamino)pyrimidin-5-yl]-1H-imidazol-5-yl}-3-[4-(6,7-dimethoxyquinolin-4-yloxy)phenyl]urea (Compound 57)

The titled compound 57 was obtained (yield: 31%) according to Example 3 from 2-tert-butyl-4-[2-(dimethylamino)pyrimidin-5-yl]-1H-imidazole-5-carboxylic acid (Compound A13-16) obtained according to Reference Example 3 and 4-(6,7-dimethoxyquinolin-4-yloxy)aniline obtained according to Example 49 of WO 97/17329. ESI-MS: *m*/*z* 583 [M+H]$^+$

Example 58

1-[2-tert-Butyl-4-(2-chloropyridin-3-yl)-1H-imidazol-5-yl]-3-[3-chloro-4-(6,7-dimethoxyquinolin-4-yloxy)phenyl]urea (Compound 58)

The titled compound 58 was obtained (yield: 8%) according to Example 3 from 2-tert-butyl-4-(2-chloropyridin-3-yl)-1H-imidazole-5-carboxylic acid (Compound A13-5) obtained according to Reference Example 3 and Compound A18 obtained according to Reference Example 6.
$^1$H-NMR (DMSO-$d_6$) δ: 11.93 (br s, 1H), 8.61 (br s, 1H), 8.46 (d, J = 5.1 Hz, 1H), 8.37 (dd, J = 4.8, 1.8 Hz, 1H), 7.90 (dd, J = 7.5, 2.0 Hz, 2H), 7.53 (s, 1H), 7.48 (dd, J = 7.7, 4.8 Hz, 1H), 7.40 (s, 1H), 7.36-7.35 (m, 2H), 6.34 (d, J = 5.5 Hz, 1H), 3.95 (s, 3H), 3.95 (s, 3H), 1.37 (s, 9H). ESI-MS: *m*/*z* 607[M+H]$^+$

Example 59

1-[2-tert-Butyl-4-(2-chloropyridin-4-yl)-1H-imidazol-5-yl]-3-[4-(6,7-dimethoxyquinolin-4-yloxy)phenyl]urea (Compound 59)

The titled compound 59 was obtained (yield: 26%) according to Example 3 from 2-tert-butyl-4-(2-chloropyridin-4-yl)-1H-imidazole-5-carboxylic acid (Compound A13-17) obtained according to Reference Example 3 and 4-(6,7-dimethoxyquinolin-4-yloxy)aniline obtained according to Example 49 of WO 97/17329.
$^1$H-NMR ($CDCl_3$, 300 MHz) δ: 11.06 (s, 1H), 8.74 (s, 1H), 8.26 (d, J = 5.5 Hz, 1H), 8.18 (d, J = 5.5 Hz, 1H), 7.43-7.38 (m, 3H), 7.20 (s, 1H), 7.06-6.94 (m, 2H), 6.60 (s, 1H), 6.25 (d, J = 5.5 Hz, 1H), 3.84 (s, 3H), 3.83 (s, 3H), 1.28 (s, 9H). ESI-MS: *m*/*z* 573[M+H]$^+$

Example 60

1-[2-tert-Butyl-4-(2-chloropyridin-3-yl)-1H-imidazol-5-yl]-3-[4-(6,7-dimethoxyquinolin-4-yloxy)-2-methoxyphenyl]urea (Compound 60)

The titled compound 60 was obtained (yield: 18%) according to Example 3 from 2-tert-butyl-4-(2-chloropyridin-3-yl)-1H-imidazole-5-carboxylic acid (Compound A13-5) obtained according to Reference Example 3 and 4-(6,7-dimethoxyquinolin-4-yloxy)-2-methoxyaniline obtained according to Production Example 5 of JP-A-11-158149. $^1$H-NMR (DMSO-$d_6$) δ: 11.78 (br s, 1H), 8.54 (br s, 1H), 8.46 (d, J = 5.3 Hz, 1H), 8.36 (dd, J = 4.8, 1.8 Hz, 1H), 8.27 (s, 1H), 7.88 (dd, J = 7.6, 2.0 Hz, 1H), 7.53 (s, 1H), 7.45 (dd, J = 7.6, 4.6 Hz, 1H), 7.38 (s, 1H), 6.97 (d, J = 2.6 Hz, 1H), 6.76 (dd, J = 8.9, 2.6 Hz, 1H), 6.51 (d, J = 5.3 Hz, 1H), 3.95 (s, 3H), 3.94 (s, 3H), 3.86 (s, 3H), 1.39 (s, 9H). ESI-MS: *m*/*z* 603[M+H]$^+$

Example 61

1-[2-tert-Butyl-4-(2-chloropyridin-3-yl)-1H-imidazol-5-yl]-3-[4-(6,7-dimethoxyquinolin-4-yloxy)-2-methylphenyl]urea (Compound 61)

The titled compound 61 was obtained (yield: 26%) according to Example 3 from 2-tert-butyl-4-(2-chloropyridin-3-yl)-1H-imidazole-5-carboxylic acid (Compound A13-5) obtained according to Reference Example 3 and 4-(6,7-dimethoxyquinolin-4-yloxy)-2-methylaniline obtained according to Production Example 6 of JP-A-11-158149. $^1$H-NMR (DMSO-$d_6$) δ: 11.92 (br s, 1H), 8.73 (br s, 1H), 8.46 (d, J = 5.1 Hz, 1H), 8.37 (dd, J = 4.8, 1.8 Hz, 1H), 8.01 (d, J = 8.4 Hz, 1H), 7.90 (dd, J = 7.3, 1.8 Hz, 1H), 7.51-7.45 (m, 2H), 7.39 (s, 1H), 7.13 (d, J = 2.6 Hz, 1H), 7.05 (dd, J = 8.4, 2.6 Hz, 1H), 6.46 (d, J = 5.1 Hz, 1H), 3.94 (s, 3H), 3.93 (s, 3H), 2.33 (s, 3H), 1.36 (s, 9H).
ESI-MS: *m*/*z* 587[M+H]$^+$

Example 62

1-[2-tert-Butyl-4-(2-chloropyridin-3-yl)-1H-imidazol-5-yl]-3-[4-(6,7-dimethoxyquinolin-4-yloxy)-2-fluorophenyl]urea (Compound 62)

The titled compound 62 was obtained (yield: 29%) according to Example 3 from 2-tert-butyl-4-(2-chloropyridin-3-yl)-1H-imidazole-5-carboxylic acid (Compound A13-5) obtained according to Reference Example 3 and 4-(6,7-dimethoxyquinolin-4-yloxy)-2-fluoroaniline obtained according to J. Med. Chem., 2005, 48(5), 1359-1366. $^1$H-NMR (DMSO-$d_6$) δ: 11.98 (br s, 1H), 8.91 (br s, 1H), 8.48 (d, J = 5.5 Hz, 1H), 8.40-8.37 (m, 2H), 7.90 (d, J = 7.3 Hz, 1H), 7.50-7.46 (m,

2H), 7.40-7.33 (m, 2H), 7.07 (d, J = 9.2 Hz, 1H), 6.53 (d, J = 5.1 Hz, 1H), 3.95 (s, 3H), 3.94 (s, 3H), 1.37 (s, 9H).
ESI-MS: *m/z* 591[M+H]$^+$

Example 63

1-[2-tert-Butyl-4-(2,6-dimethylpyridin-4-yl)-1H-imidazol-5-yl]-3-[4-(6,7-dimethoxyquinolin-4-yloxy)phenyl]urea (Compound 63)

The titled compound 63 was obtained (yield: 5%) according to Example 3 from 2-tert-butyl-4-(2,6-dimethylpyridin-4-yl)-1H-imidazole-5-carboxylic acid (Compound A13-18) obtained according to Reference Example 3 and 4-(6,7-dimethoxyquinolin-4-yloxy)aniline obtained according to Example 49 of WO 97/17329.
$^1$H-NMR ($CDCl_3$, 300 MHz) δ: 11.41 (s, 1H), 9.21 (s, 1H), 8.49 (d, J = 5.1 Hz, 1H), 7.67 (d, J = 8.8 Hz, 2H), 7.60 (s, 1H), 7.42 (s, 1H), 7.17 (d, J = 8.8 Hz, 2H), 6.95 (s, 2H), 6.81 (s, 1H), 6.48 (d, J = 5.1 Hz, 1H), 4.08 (s, 3H), 4.05 (s, 3H), 2.54 (s, 6H), 1.50 (s, 9H).
ESI-MS: *m/z* 567[M+H]$^+$

Example 64

1-[2-tert-Butyl-4-(2,4-difluorophenyl)-1H-imidazol-5-yl]-3-[4-(6,7-dimethoxyquinolin-4-yloxy)phenyl]urea (Compound 64)

The titled compound 64 was obtained (yield: 23%) according to Example 3 from 2-tert-butyl-4-(2,4-difluorophenyl)-1H-imidazole-5-carboxylic acid (Compound A13-19) obtained according to Reference Example 3 and 4-(6,7-dimethoxyquinolin-4-yloxy)aniline obtained according to Example 49 of WO 97/17329.
$^1$H-NMR ($CDCl_3$, 270 MHz) δ: 11.25 (s, 1H), 8.83 (s, 1H), 8.48 (d, J = 5.3 Hz, 1H), 7.71-7.62 (m, 2H), 7.60 (s, 1H), 7.44 (s, 1H), 7.34 (s, 1H), 7.20-7.12 (m, 3H), 7.01 (m, 1H), 6.51 (s, 1H), 6.48 (d, J = 5.3 Hz, 1H), 4.07 (s, 3H), 4.06 (s, 3H), 1.48 (s, 9H).
ESI-MS: *m/z* 574[M+H]$^+$

Example 65

1-[2-tert-Butyl-4-(2-fluorophenyl)-1H-imidazol-5-yl]-3-[4-(6,7-dimethoxyquinolin-4-yloxy)phenyl]urea (Compound 65)

The titled compound 65 was obtained (yield: 49%) according to Example 3 from 2-tert-butyl-4-(2-fluorophenyl)-1H-imidazole-5-carboxylic acid (Compound A13-20) obtained according to Reference Example 3 and 4-(6,7-dimethoxyquinolin-4-yloxy)aniline obtained according to Example 49 of WO 97/17329.
$^1$H-NMR ($CDCl_3$, 270 MHz) δ: 11.32 (s, 1H), 8.93 (s, 1H), 8.48 (d, J = 5.3 Hz, 1H), 7.70-7.63 (m, 2H), 7.60 (s, 1H), 7.53-7.42 (m, 2H), 7.34 (s, 1H), 7.29 (m, 1H), 7.22-7.12 (m, 3H), 6.62 (s, 1H), 6.48 (d, J = 5.3 Hz, 1H), 4.07 (s, 3H), 4.06 (s, 3H), 1.49 (s, 9H).
ESI-MS: *m/z* 556[M+H]$^+$

Example 66

1-(2-tert-Butyl-1H-imidazol-5-yl)-3-[4-(6,7-dimethoxyquinolin-4-yloxy)phenyl]urea (Compound 66)

The titled compound 66 was obtained (yield: 27%) according to Example 3 from 2-tert-butyl-1H-imidazole-5-carboxylic acid (Compound A10-1) obtained by the usual hydrolysis of methyl 2-tert-butyl-1H-imidazole-5-carboxylate (Compound A10) obtained according to Step 4 of Reference Example 3 and 4-(6,7-dimethoxyquinolin-4-yloxy)aniline obtained according to Example 49 of WO 97/17329. $^1$H-NMR ($CDCl_3$, 300 MHz) δ: 10.85 (s, 1H), 8.47 (d, J = 5.5 Hz, 1H), 7.68-7.60 (m, 2H), 7.59 (s, 1H), 7.43 (s, 1H), 7.18-7.10 (m, 2H), 6.79 (s, 1H), 6.51 (s, 1H), 6.47 (d, J = 5.5 Hz, 1H), 4.06 (s, 3H), 4.05 (s, 3H), 1.42 (s, 9H).
ESI-MS: *m/z* 462[M+H]$^+$

Example 67

1-[2-tert-Butyl-4-(2-chloropyridin-3-yl)-1H-imidazol-5-yl]-3-[4-(6,7-dimethoxyquinolin-4-yloxy)-2,5-dimethylphenyl]urea (Compound 67)

The titled compound 67 was obtained (yield: 74%) according to Example 3 from 2-tert-butyl-4-(2-chloropyridin-3-yl)-1H-imzdazole-5-carboxylic acid (Compound A13-5) obtained according to Reference Example 3 and 4-(6,7-dimethoxyquinolin-4-yloxy)-2,5-dimethylaniline obtained according to Production Example 3 of WO 00/43366. $^1$H-NMR ($CDCl_3$, 300 MHz) δ: 10.54 (s, 1H), 9.08 (s, 1H), 8.45 (d, J = 5.5 Hz, 1H), 8.40 (dd, J = 4.8, 1.8 Hz, 1H), 8.01 (s, 1H), 7.88 (dd, J = 5.5, 1.8 Hz, 1H), 7.62 (s, 1H), 7.43 (s, 1H), 7.41-7.38 (m, 1H), 6.95 (s, 1H), 6.50 (s, 1H), 6.34 (d, J = 5.1 Hz, 1H), 4.07 (s, 3H), 4.06 (s, 3H), 2.43 (s, 3H), 2.17 (s, 3H), 1.46 (s, 9H).
ESI-MS: *m/z* 601$[M+H]^+$

Example 68

1-[2-tert-Butyl-4-(2-chloropyridin-3-yl)-1H-imidazol-5-yl]-3-[4-(6,7-dimethoxyquinolin-4-yloxy)-2,3-dimethylphenyl]urea (Compound 68)

The titled compound 68 was obtained (yield: 72%) according to Example 3 from 2-tert-butyl-4-(2-chloropyridin-3-yl)-1H-imidazole-5-carboxylic acid (Compound A13-5) obtained according to Reference Example 3 and 4-(6,7-dimethoxyquinolin-4-yloxy)-2,3-dimethylaniline obtained according to Production Example 2 of WO 00/43366.
$^1$H-NMR ($CDCl_3$, 300 MHz) δ: 10.65 (s, 1H), 9.08 (s, 1H), 8.44 (d, J = 5.1 Hz, 1H), 8.39 (dd, J = 4.6, 2.0 Hz, 1H), 7.88-7.87 (m, 2H), 7.64 (s, 1H), 7.44 (s, 1H), 7.38 (dd, J = 8.1, 5.1 Hz, 1H), 7.02 (d, J = 9.2 Hz, 1H), 6.51 (s, 1H), 6.32 (d, J = 5.1 Hz, 1H), 4.08 (s, 3H), 4.06 (s, 3H), 2.43 (s, 3H), 2.15 (s, 3H), 1.47 (s, 9H).
ESI-MS: *m/z* 601$[M+H]^+$

Example 69

2-tert-Butyl-5-{3-[4-(6,7-dimethoxyquinolin-4-yloxy)phenyl]ureido}-N-(pyridin-3-yl)-1H-imidazole-4-carboxamide (Compound 69)

The titled compound 69 was obtained (yield: 13%) according to Example 3 from 2-tert-butyl-4-(pyridin-3-ylcarbamoyl)-1H-imidazole-5-carboxylic acid (Compound A29) obtained according to Reference Example 10 and 4-(6,7-dimethoxyquinolin-4-yloxy)aniline obtained according to Example 49 of WO 97/17329.
$^1$H-NMR ($CDCl_3$, 300 MHz) δ: 11.00 (s, 1H), 9.51 (s, 1H), 8.89 (s, 1H), 8.78 (s, 1H), 8.49 (d, J = 5.1 Hz, 1H), 8.33-8.24 (m, 2H), 8.04 (d, J = 7.3 Hz, 1H), 7.57 (s, 1H), 7.46 (d, J = 8.8 Hz, 2H), 7.43 (s, 1H), 7.15 (d, J = 8.8 Hz, 2H), 6.47 (d, J = 5.1 Hz, 1H), 4.05 (s, 3H), 4.05 (s, 3H), 1.40 (s, 9H).
ESI-MS: *m/z* 582$[M+H]^+$

Example 70

2-tert-Butyl-5-{3-[4-(6,7-dimethoxyquinolin-4-yloxy)phenyl]ureido}-N-(pyridin-2-yl)-1H-imidazole-4-carboxamide (Compound 70)

The titled compound 70 was obtained (yield: 13%) according to Example 3 from 2-tert-butyl-4-(pyridin-2-ylcarbamoyl)-1H-imidazole-5-carboxylic acid (Compound A29-1) obtained according to Reference Example 10 and 4-(6,7-dimethoxyquinolin-4-yloxy)aniline obtained according to Example 49 of WO 97/17329.
$^1$H-NMR ($CDCl_3$, 300 MHz) δ: 10.99 (s, 1H), 9.57 (s, 1H), 9.36 (s, 1H), 8.47 (d, J = 5.1 Hz, 1H), 8.36-8.31 (m, 2H), 8.16 (d, J = 8.4 Hz, 1H), 7.57 (s, 1H), 7.45 (d, J = 8.8 Hz, 2H), 7.43 (s, 1H), 7.14 (d, J = 8.8 Hz, 2H), 6.44 (d, J = 5.1 Hz, 1H), 4.05 (s, 3H), 4.05 (s, 3H), 1.38 (s, 9H). ESI-MS: *m/z* 582 $[M+H]^+$

Example 71

1-[2-tert-Butyl-4-(3,5-dimethylisoxazol-4-yl)-1H-imidazol-5-yl]-3-[4-(7-chloroquinolin-4-yloxy)phenyl]urea (Compound 71)

The titled compound 71 was obtained (yield: 44%) according to Example 3 from 2-tert-butyl-4-(3,5-dimethylisoxazol-4-yl)-1H-imidazole-5-carboxylic acid (Compound A13-3) obtained according to Reference Example 3 and 4-(7-chloroquinolin-4-yloxy)aniline obtained according to Eur. J. Med. Chem., 1986, 21, 5-8.
$^1$H-NMR (DMSO-$d_6$, 300 MHz) δ: 11.73 (s, 1H), 8.71 (d, J = 5.5 Hz, 1H), 8.38 (s, 1H), 8.36 (d, J = 8.8 Hz, 1H), 8.08 (d, J = 2.2 Hz, 1H), 7.69 (dd, J = 8.8, 2.2 Hz, 1H), 7.58 (d, J = 9.2 Hz, 2H), 7.24 (d, J = 9.2 Hz, 2H), 6.61 (d, J = 5.5 Hz, 1H), 2.29 (s, 3H), 2.13 (s, 3H), 1.36 (s, 9H). ESI-MS: *m*/*z* 531[M+H]$^+$

Example 72

4-(4-{3-[2-tert-Butyl-4-(3,5-dimethylisoxazol-4-yl)-1H-imidazol-5-yl]ureido}-3-chlorophenoxy)-7-methoxyquinoline-6-carboxamide (Compound 72)

The titled compound 72 was obtained (yield: 26%) according to Example 3 from 2-tert-butyl-4-(3,5-dimethylisoxazol-4-yl)-1H-imidazole-5-carboxylic acid (Compound A13-3) obtained according to Reference Example 3 and 4-(4-amino-3-chlorophenoxy)-7-methoxyquinoline-6-carboxamide obtained according to the production method 366-1 of WO 02/32872.
$^1$H-NMR ($CDCl_3$, 300 MHz) δ: 8.62 (d, J = 5.5 Hz, 1H), 8.34 (d, J = 9.2 Hz, 1H), 7.51 (s, 1H), 7.30 (d, J = 2.9 Hz, 1H), 7.26 (s, 1H), 7.14 (dd, J = 9.2, 2.9 Hz, 1H), 6.57 (d, J = 5.5 Hz, 1H), 4.16 (s, 3H), 2.40 (s, 3H), 2.24 (s, 3H), 1.43 (s, 9H). ESI-MS: *m*/*z* 604[M+H]$^+$

Example 73

Methyl 4-(4-{3-[2-tert-butyl-4-(3,5-dimethylisoxazol-4-yl)-1H-imidazol-5-yl]ureido}-2-fluorophenoxy)-7-methoxyquinoline-6-carboxylate (Compound 73)
The titled compound 73 was obtained (yield: 75%) according to Example 3 from 2-tert-butyl-4-(3,5-dimethylisoxazol-4-yl)-1H-imidazole-5-carboxylic acid (Compound A13-3) obtained according to Reference Example 3 and methyl 4-(4-amino-2-fluorophenoxy)-7-methoxyquinoline-6-carboxylate obtained according to Example 39b of WO 2009/125597.
$^1$H-NMR (DMSO-$d_6$, 300 MHz) δ: 11.76 (s, 1H), 8.70 (d, J = 5.1 Hz, 1H), 8.61 (s, 1H), 8.50 (s, 1H), 7.77 (dd, J = 8.8, 2.4 Hz, 1H), 7.55 (s, 1H), 7.42 (t, J = 8.8 Hz, 1H), 7.20 (d, J = 8.8 Hz, 1H), 6.53 (dd, J = 5.1 Hz, 1H), 3.99 (s, 3H), 3.88 (s, 3H), 2.30 (s, 3H), 2.14 (s, 3H), 1.36 (s, 9H).
ESI-MS: *m*/*z* 603[M+H]$^+$

Example 74

4-(4-{3-[2-tert-Butyl-4-(3,5-dimethylisoxazol-4-yl)-1H-imidazol-5-yl]ureido}-2-fluorophenoxy)-7-methoxy-N-(2-morpholinoethyl)quinoline-6-carboxamide (Compound 74)

Compound 73 (30 mg) obtained according to Example 73 was dissolved in a solvent mixture of THF (0.66 mL) and water (0.33 mL), and potassium hydroxide (97 mg) was added thereto, followed by stirring at 80°C overnight. Ethyl acetate was added to the reaction mixture. After extraction with water, 2 mol/L of hydrochloric acid was added to the aqueous phase to give a pH of 4. The solvent was distilled off from the resulting aqueous solution under reduced pressure. The resulting residue was purified by silica gel column chromatography (chloroform : methanol = 8:2). To the resulting residue, THF (1 mL), 2-morpholinoethaneamine (14 mg), 4-(4,6-dimethoxy-1,3,5-triazin-2-yl)-4-methylmorpholin-4-ium hydrochloride (18 mg), and methanol (0.5 mL) were added, followed by stirring at room temperature for 2 hours. The solvent was distilled off from the reaction mixture under reduced pressure. Water was added to the resulting residue, and the precipitated solid was collected by filtration. Isopropyl alcohol (1 mL) was added to the resulting solid to reslurry for purification. The titled compound 74 (12 mg, 2-step yield: 30%) was obtained. $^1$H-NMR ($CDCl_3$:$CD_3OD$ = 2:1, 270 MHz) δ: 9.19 (s, 1H), 8.61 (d, J = 5.6 Hz, 1H), 7.71 (d, J = 12.5 Hz, 1H), 7.51 (s, 1H), 7.22 (m, 2H), 6.51 (d, J = 5.6Hz, 1H), 4.17 (s, 3H), 3.80 (t, J = 4.3 Hz, 4H), 3.65 (t, J = 6.3 Hz, 2H), 2.69 (t, J = 6.3 Hz, 2H), 2.60 (t, J = 4.3 Hz, 4H), 2.39 (s, 3H), 2.24 (s, 3H), 1.44 (s, 9H).
ESI-MS: *m*/*z* 701[M+H]$^+$

Example 75

1-[2-tert-Butyl-4-(3,5-dimethylisoxazol-4-yl)-1H-imidazol-5-yl]-3-{4-[3-(1-methyl-1H-pyrazol-4-yl)pyridin-4-yloxy]phenyl} urea (Compound 75)

The titled compound 75 was obtained (yield: 53%) according to Example 3 from 2-tert-butyl-4-(3,5-dimethylisoxazol-4-yl)-1H-imidazole-5-carboxylic acid (Compound A13-3) obtained according to Reference Example 3 and 4-[3-(1-methyl-1H-pyrazol-4-yl)pyridin-4-yloxy]aniline (Compound A31) obtained according to Reference Example

11. $^1$H-NMR ($CDCl_3$, 300 MHz) δ: 11.35 (s, 1H), 9.24 (s, 1H), 8.75 (s, 1H), 8.22 (d, J = 5.9 Hz, 1H), 7.97 (s, 1H), 7.94 (s, 1H), 7.59 (d, J = 8.8 Hz, 2H), 7.09 (d, J = 8.8 Hz, 2H), 6.66 (d, J = 5.9 Hz, 1H), 6.55 (s, 1H), 3.97 (s, 3H), 2.37 (s, 3H), 2.16 (s, 3H), 1.47 (s, 9H).
ESI-MS: *m*/*z* 527$[M+H]^+$

Example 76

4-(4-{3-[2-tert-Butyl-4-(3,5-dimethylisoxazol-4-yl)-1H-imidazol-5-yl]ureido}phenoxy)-N-(pyridin-4-yl)picolinamide (Compound 76)

The titled compound 76 was obtained (yield: 65%) according to Example 3 from 2-tert-butyl-4-(3,5-dimethylisoxazol-4-yl)-1H-imidazole-5-carboxylic acid (Compound A13-3) obtained according to Reference Example 3 and 4-(4-aminophenoxy)-N-(pyridin-4-yl)picolinamide (Compound A33) obtained according to Reference Example 12. $^1$H-NMR (DMSO-$d_6$, 300 MHz) δ: 11.74 (s, 1H), 11.03 (s, 1H), 8.63 (d, J = 5.5 Hz, 1H), 8.48 (d, J = 6.6 Hz, 2H), 7.92 (d, J = 6.6 Hz, 2H), 7.57 (d, J = 9.2 Hz, 2H), 7.52 (d, J = 2.9 Hz, 1H), 7.25 (dd, J = 5.5, 2.9 Hz, 1H), 7.19 (d, J = 9.2 Hz, 2H), 2.30 (s, 3H), 2.14 (s, 3H), 1.36 (s, 9H). ESI-MS: *m*/*z* 567$[M+H]^+$

Example 77

4-(4-{3-[2-tert-Butyl-4-(3,5-dimethylisoxazol-4-yl)-1H-imidazol-5-yl]ureido}-2-fluorophenoxy)-N-methylpicolinamide (Compound 77)

The titled compound 77 was obtained (yield: 15%) according to Example 3 from 2-tert-butyl-4-(3,5-dimethylisoxazol-4-yl)-1H-imidazole-5-carboxylic acid (Compound A13-3) obtained according to Reference Example 3 and 4-(4-amino-2-fluorophenoxy)-N-methylpicolinamide obtained according to Example A1 of US 2008/0090856. $^1$H-NMR ($CDCl_3$, 270 MHz) δ: 11.43 (s, 1H), 8.68 (s, 1H), 8.39 (d, J = 5.6 Hz, 1H), 8.02 (s, 1H), 7.66 (t, J = 4.6 Hz, 2H), 7.22 (s, 1H), 7.12 (t, J = 8.7 Hz, 1H), 6.99-6.97 (m, 1H), 6.16 (s, 1H), 3.00 (d, J = 5.3 Hz, 3H), 2.38 (s, 3H), 2.20 (s, 3H), 1.46 (s, 9H).
ESI-MS: *m*/*z* 522$[M+H]^+$

Example 78

1-[2-tert-Butyl-4-(3,5-dimethylisoxazol-4-yl)-1H-imidazol-5-yl]-3-(4-{2-(1-methyl-1H-imidazol-2-yl)thieno[3,2-b]pyridin-7-yloxy}phenyl)urea (Compound 78)

The titled compound 78 was obtained (yield: 40%) according to Example 3 from 2-tert-butyl-4-(3,5-dimethylisoxazol-4-yl)-1H-imidazole-5-carboxylic acid (Compound A13-3) obtained according to Reference Example 3 and 4-{2-(1-methyl-1H-imidazol-2-yl)thieno[3,2-b]pyridin-7-yloxy}aniline obtained according to Example 194 (Compound 219) of WO 2006/010264.
$^1$H-NMR (DMSO-$d_6$, 300 MHz) δ: 11.73 (s, 1H), 8.50 (d, J = 5.3 Hz, 1H), 8.39 (s, 1H), 7.88 (s, 1H), 7.56 (d, J = 8.9 Hz, 2H), 7.41 (s, 1H), 7.23 (d, J = 8.9 Hz, 2H), 7.05 (s, 1H), 6.65 (d, J = 5.3 Hz, 1H), 3.99 (s, 3H), 2.29 (s, 3H), 2.13 (s, 3H), 1.36 (s, 9H).
ESI-MS: *m*/*z* 583$[M+H]^+$

Example 79

(R)-1-{2-tert-Butyl-4-(3,5-dimethylisoxazol-4-yl)-1H-imidazol-5-yl}-3-(4-[2-{3-(dimethylamino)pyrrolidine-1-carbonyl}thieno[3,2-b]pyridin-7-yloxy]phenyl)urea (Compound 79)

The titled compound 79 was obtained (yield: 30%) according to Example 3 from 2-tert-butyl-4-(3,5-dimethylisoxazol-4-yl)-1H-imidazole-5-carboxylic acid (Compound A13-3) obtained according to Reference Example 3 and (R)-{7-(4-aminophenoxy)thieno[3,2-b]pyridin-2-yl}{3-(dimethylamino)pyrrolidin-1-yl}methanone obtained according to Example 7 (Compound 26) of US 2007/0004675. $^1$H-NMR (DMSO-$d_6$, 300 MHz) δ: 11.73 (s, 1H), 8.57 (d, J = 5. 1 Hz, 1H), 8.39 (s, 1H), 7.56 (d, J = 8.8 Hz, 2H), 7.23 (d, J = 8.8 Hz, 2H), 6.70 (d, J = 5.1 Hz, 1H), 4.06-3.45 (m, 4H), 2.83-2.71 (m, 1H), 2.29 (s, 3H), 2.20 (s, 3H), 2.19 (s, 3H), 2.13 (s, 3H), 1.91-1.72 (m, 2H), 1.35 (s, 9H). ESI-MS: *m*/*z* 643$[M+H]^+$

Example 80

1-{2-tert-Butyl-4-(3,5-dimethylisoxazol-4-yl)-1H-imidazol-5-yl}-3-[4-{2-(1-methyl-1H-imidazol-4-yl)thieno[3,2-b]pyridin-7-yloxy}phenyl]urea (Compound 80)

The titled compound 80 was obtained (yield: 44%) according to Example 3 from 2-tert-butyl-4-(3,5-dimethylisoxazol-4-yl)-1H-imidazole-5-carboxylic acid (Compound A13-3) obtained according to Reference Example 3 and 4-{2-(1-methyl-1H-imidazol-4-yl)thieno[3,2-b]pyridin-7-yloxy}aniline obtained according to Example 201 (Compound 226) of WO 2006/010264.
$^1$H-NMR (DMSO-D6, 300 MHz) $\delta$: 8.41 (d, J = 5.5 Hz, 1H), 8.38 (s, 1H), 7.85 (s, 1H), 7.72 (s, 1H), 7.66 (s, 1H), 7.55 (d, J = 8.8 Hz, 2H), 7.21 (d, J = 8.8 Hz, 2H), 6.54 (d, J = 5.5 Hz, 1H), 3.73 (s, 3H), 2.30 (s, 3H), 2.14 (s, 3H), 1.35 (s, 9H).
ESI-MS: *m*/*z* 583$[M+H]^+$

Example 81

1-{2-tert-Butyl-4-(3,5-dimethylisoxazol-4-yl)-1H-imidazol-5-yl}-3-[4-{2-(4-methoxyphenyl)thieno[3,2-b]pyridin-7-yloxy}phenyl]urea (Compound 81)

The titled compound 81 was obtained (yield: 79%) according to Example 3 from 2-tert-butyl-4-(3,5-dimethylisoxazol-4-yl)-1H-imidazole-5-carboxylic acid (Compound A13-3) obtained according to Reference Example 3 and 4-{2-(4-methoxyphenyl)thieno[3,2-b]pyridin-7-yloxy}aniline obtained according to Example 109 (Compound 154) of WO 2006/010264.
$^1$H-NMR (DMSO-D6, 400 MHz) $\delta$: 11.73 (s, 1H), 8.45 (d, J = 5.9 Hz, 1H), 8.39 (s, 1H), 7.90 (s, 1H), 7.83 (d, J = 8.8 Hz, 2H), 7.56 (d, J = 8.8 Hz, 2H), 7.23 (d, J = 8.8 Hz, 2H), 7.07 (d, J = 8.8 Hz, 2H), 6.57 (d, J = 5.9 Hz, 1H), 3.83 (s, 3H), 2.29 (s, 3H), 2.13 (s, 3H), 1.35 (s, 9H).
ESI-MS: *m*/*z* 609$[M+H]^+$

Example 82

1-{2-tert-Butyl-4-(3,5-dimethylisoxazol-4-yl)-1H-imidazol-5-yl}-3-[4-{2-(6-methoxypyridin-3-yl)thieno[3,2-b]pyridin-7-yloxy}phenyl]urea (Compound 82)

The titled compound 82 was obtained (yield: 44%) according to Example 3 from 2-tert-butyl-4-(3,5-dimethylisoxazol-4-yl)-1H-imidazole-5-carboxylic acid (Compound A13-3) obtained according to Reference Example 3 and 4-{2-(6-methoxypyridin-3-yl)thieno[3,2-b]pyridin-7-yloxy}aniline obtained according to Example 49 (Compound 46) of WO 2006/010264.
$^1$H-NMR (DMSO-D6, 400 MHz) $\delta$: 11.73 (s, 1H), 8.72 (d, J = 2.9 Hz, 1H), 8.49 (d, J = 4.9 Hz, 1H), 8.39 (s, 1H), 8.23 (dd, J = 8.8, 2.9 Hz, 1H), 8.03 (s, 1H), 7.56 (d, J = 8.8 Hz, 2H), 7.24 (d, J = 8.8 Hz, 2H), 6.99 (d, J = 8.8 Hz, 1H), 6.59 (d, J = 4.9 Hz, 1H), 3.93 (s, 3H), 2.29 (s, 3H), 2.13 (s, 3H), 1.36 (s, 9H).
ESI-MS: *m*/*z* 610$[M+H]^+$

Example 83

4-(4-{3-[2-tert-Butyl-4-(2-chloropyridin-3-yl)-1H-imidazol-5-yl]ureido}-3-chlorophenoxy)-7-methoxyquinoline-6-carboxamide (Compound 83)

The titled compound 83 was obtained (yield: 35%) according to Example 3 from 2-tert-butyl-4-(2-chloropyridin-3-yl)-1H-imidazole-5-carboxylic acid (Compound A13-5) obtained according to Reference Example 3 and 4-(4-amino-3-chlorophenoxy)-7-methoxyquinoline-6-carboxamide obtained according to Production Example 366-1 of WO 02/32872. $^1$H-NMR ($CDCl_3$, 270 MHz) $\delta$: 9.21 (s, 1H), 8.62 (d, J = 5.3 Hz, 1H), 8.34 (d, J = 8.6 Hz, 2H), 7.87 (s, 1H), 7.51 (s, 1H), 7.40-7.35 (m, 4H), 7.27 (d, J = 3.0 Hz, 1H), 7.12 (dd, J = 9.0, 2.8 Hz, 1H), 6.54 (d, J = 5.3 Hz, 1H), 4.15 (s, 2H), 3.38 (s, 3H), 1.45 (s, 9H).
ESI-MS: *m*/*z* 620$[M+H]^+$

Example 84

1-[2-tert-Butyl-4-(2-chloropyridin-3-yl)-1H-imidazol-5-yl]-3-(2-methoxy-4-{2-(1-methyl-1H-imidazol-2-yl)thieno[3,2-b]pyridin-7-yloxy}phenyl)urea (Compound 84)

The titled compound 84 was obtained (yield: 51%) according to Example 3 from 2-tert-butyl-4-(2-chloropyridin-3-yl)-1H-imidazole-5-carboxylic acid (Compound A13-5) obtained according to Reference Example 3 and 2-methoxy-4-{2-(1-methyl-1H-imidazol-2-yl)thieno[3,2-b]pyridin-7-yloxy}aniline obtained according to Example 194 (Compound 219) of WO 2006/10264.
$^1$H-NMR ($CDCl_3$, 270 MHz) δ: 10.99 (s, 1H), 9.10 (s, 1H), 8.48 (d, J = 5.3 Hz, 1H), 8.40-8.37 (m, 2H), 7.87 (dd, J = 7.6, 2.0 Hz, 1H), 7.70 (s, 1H), 7.36-7.34 (m, 1H), 7.17 (d, J = 1.0 Hz, 1H), 7.04 (s, 1H), 6.80-6.79 (m, 2H), 6.62 (d, J = 5.6 Hz, 1H), 6.50 (s, 1H), 3.97 (s, 3H), 3.92 (s, 3H), 1.51 (s, 9H).
ESI-MS: *m*/*z* 629$[M+H]^+$

Example 85

1-[2-tert-butyl-4-(2-chloropyridin-3-yl)-1H-imidazol-5-yl]-3-{3-fluoro-4-[6-methoxy-7-(3-morpholinopropoxy)quinolin-4-yloxy]phenyl}urea (Compound 85)

The titled compound 85 was obtained (yield: 39%) according to Example 3 from 2-tert-butyl-4-(2-chloropyridin-3-yl)-1H-imidazole-5-carboxylic acid (Compound A13-5) obtained according to Reference Example 3 and 3-fluoro-4-{6-methoxy-7-(3-morpholinopropoxy)quinolin-4-yloxy}aniline obtained according to Production Example 12 of WO 03/000660. $^1$H-NMR ($CDCl_3$, 270 MHz) δ: 11.47 (s, 1H), 9.07 (s, 1H), 8.48 (d, J = 5.3 Hz, 1H), 8.40 (dd, J = 4.6, 1.3 Hz, 1H), 7.83 (dd, J = 7. 7, 1.8 Hz, 1H), 7.73 (t, J = 6.3 Hz, 1H), 7.60 (s, 1H), 7.44 (s, 1H), 7.38 (dd, J = 7.6, 4.6 Hz, 1H), 6.54 (s, 1H), 6.42 (d, J = 4.9 Hz, 1H), 4.28 (t, J = 6.9 Hz, 2H), 4.05 (s, 3H), 3.74-3.72 (m, 4H), 2.58 (t, J = 7.1 Hz, 2H), 2.51-2.49 (m, 4H), 2.14 (dd, J = 11.8, 4.6 Hz, 2H), 1.50 (s, 9H).
ESI-MS: *m*/*z* 704$[M+H]^+$

Reference Example 1

Step 1

Methyl 1-isopropyl-1H-imidazole-4-carboxylate (Compound A1)

A mixture of methyl 2-isocyanoacetate (198 mg) and 1,1-dimethoxy-N,N-dimethylmethanamide (286 mg) was stirred at 0°C for 1 hour. Isopropylamine (591 mg) was added to the mixture, followed by stirring under reflux for 2 hours. After the completion of the reaction, the solvent was distilled off from the resulting reaction mixture under reduced pressure. The resulting residue was purified by silica gel column chromatography using chloroform and methanol as the eluents to give the titled compound A1 (210 mg, yield: 63%).

Step 2

1-Isopropyl-1H-imidazole-4-carboxylic acid (Compound A2)

Compound A1 (168 mg) obtained according to Step 1 was dissolved in THF (5 mL), and water (5 mL) and lithium hydroxide monohydrate (84 mg) were added thereto, followed by stirring at room temperature overnight. After the completion of the reaction, the solvent was distilled off from the resulting reaction mixture under reduced pressure. The resulting residue was purified by silica gel column chromatography using chloroform and methanol as the eluents to give the titled compound A2 (66 mg, yield: 43%). Reference Example 2

Step 1

Ethyl 2,3-dioxo-3-phenylpropanoate (Compound A3)

Ethyl 3-oxo-3-phenylpropanoate (1.9 g) was dissolved in dichloromethane (100 mL), and pyridine (2.4 g) and Dess-Martin periodinane (6.4 g) were added thereto, followed by stirring at room temperature overnight. To the resulting reaction mixture was added a solution mixture (volume ratio = 1:1) of a saturated sodium bicarbonate aqueous solution and a saturated sodium thiosulfate aqueous solution, followed by stirring at room temperature for 1 hour. After extraction

with chloroform, the organic phase was washed with water and saturated brine, dried over anhydrous sodium sulfate, and filtered. The solvent was distilled off from the filtrate under reduced pressure, and the residue was purified by silica gel column chromatography using hexane and ethyl acetate as the eluents to give the titled compound A3 (1.3 g, yield: 65%).

Step 2

Ethyl 2-tert-butyl-5-phenyl-1H-imidazole-4-carboxylate (Compound A4)

Ammonium acetate (770 mg), acetic acid (4 mL), Compound A3 (206 mg) obtained according to Step 1, and pivalaldehyde (130 mg) were placed in a reaction vessel for a microwave reaction apparatus (Emrys Optimizer (registered trademark), manufactured by Personal Chemistry) and were stirred under microwave irradiation at 150°C for 15 minutes. The solvent was distilled off from the reaction mixture under reduced pressure, and ethyl acetate was added to the residue. The organic phase was washed with water and saturated brine, dried over anhydrous sodium sulfate, and filtered. The solvent was distilled off from the filtrate under reduced pressure. The resulting residue was purified by silica gel column chromatography using hexane and ethyl acetate as the eluents to give the titled compound A4 (469 mg, yield: 57%).

Step 3

Ethyl 2-tert-butyl-1-methyl-4-phenyl-1H-imidazole-5-carboxylate (Compound A5)

DMF (50 mL) and Compound A4 (469 mg) obtained according to Step 2 were added to sodium hydride (82 mg), followed by stirring at room temperature for 10 minutes. Methyl iodide (292 mg) was further added to the mixture, followed by stirring at room temperature overnight. The reaction was stopped by adding water to the reaction mixture, and extraction with ethyl acetate was performed. The organic phase was washed with water and saturated brine, dried over anhydrous sodium sulfate, and filtered. The solvent was distilled off from the filtrate under reduced pressure. The resulting residue was purified by silica gel column chromatography using hexane and ethyl acetate as the eluents to give the titled compound A5 (267 mg, yield: 54%).

Step 4

2-tert-Butyl-1-methyl-4-phenyl-1H-imidazole-5-carboxylic acid (Compound A6)

Compound A5 (267 mg) obtained according to Step 3 was dissolved in THF (1.6 mL), water (0.8 mL), and methanol (0.4 mL), and lithium hydroxide monohydrate (79 mg) was added thereto, followed by stirring at 50°C overnight. The reaction mixture was neutralized with 1 mol/L of hydrochloric acid, and the solvent was distilled off under reduced pressure. The resulting residue was purified by silica gel column chromatography using chloroform and methanol as the eluents to give the titled compound A6 (199 mg, yield: 82%).

Reference Example 3

Step 1

Methyl pivalimidate hydrochloride (Compound A7)

Methanol (29.0 mL) and pivalonitrile (15.0 g) were added to a 4 mol/L hydrogen chloride-dioxane solution (200 mL), followed by stirring at room temperature for 26 hours. The solvent was distilled off from the reaction mixture under reduced pressure. The residue was slurried for purification using hexane to give the titled compound A7 (13.87 g, yield: 51%).

Step 2

Methyl 2,3-diaminopropionate dihydrochloride (Compound A8)

2,3-Diaminopropionic acid hydrochloride (10.5 g) was suspended in methanol, and thionyl chloride (6.50 mL) was added to the suspension at 0°C, followed by stirring under reflux for 15 hours. The resulting reaction mixture was cooled with ice, and the precipitated solid was collected by filtration to give the titled compound A8 (10.41 g, yield: 73%).

Step 3

Methyl 2-tert-butyl-4,5-dihydro-1H-imidazole-5-carboxylate (Compound A9)

Compound A7 (500 mg) obtained according to Step 1 and Compound A8 (694 mg) obtained according to Step 2 were suspended in acetonitrile (20 mL), and triethylamine (1.52 mL) was added thereto, followed by stirring at 80°C for 3 hours. The solvent was distilled off from the resulting reaction mixture under reduced pressure. A sodium bicarbonate aqueous solution was added to the residue, followed by extraction with chloroform. The organic phase was washed with a saturated brine solution, dried over anhydrous magnesium sulfate, and filtered. The solvent was distilled off from the filtrate under reduced pressure to give the titled compound A9 (448 mg, yield: 74%).

Step 4

Methyl 2-tert-butyl-1H-imidazole-5-carboxylate (Compound A10)

Compound A9 (448 mg) obtained according to Step 3 was dissolved in acetonitrile (10 mL), and DBU (0.509 mL) was added to the solution. 1,3,5-Trichloro-1,3,5-triazine-2,4,6-trione (226 mg) was further added thereto at 0°C, followed by stirring at 0°C for 45 minutes. The resulting reaction mixture was filtered, and the solvent was distilled off from the filtrate under reduced pressure. The residue was purified by silica gel column chromatography (hexane : ethyl acetate = 70:30 to 50:50) to give the titled compound A10 (325 mg, yield: 73%).

Step 5

Methyl 2-tert-butyl-4-iodo-1H-imidazole-5-carboxylate (Compound A11)

Compound A10 (325 mg) obtained according to Step 4 was dissolved in acetic acid (5 mL), and trifluoroacetic acid (0.140 mL) and N-iodosuccinimide (602 mg) were added thereto, followed by stirring at room temperature for 30 hours. To the water was added the resulting reaction mixture, and the pH was adjusted to 9 with sodium carbonate. Extraction with ethyl acetate was performed. The organic phase was washed with a saturated sodium bicarbonate aqueous solution, a saturated sodium thiosulfate aqueous solution, and a saturated brine solution, was dried over anhydrous magnesium sulfate, and was filtered. The solvent was distilled off from the filtrate under reduced pressure. The residue was purified by silica gel column chromatography (hexane : ethyl acetate = 90:10 to 70:30) to give the titled compound A11 (390 mg, yield: 71%).

Step 6

Methyl 2-tert-butyl-4-(1-methyl-1H-pyrazol-4-yl)-1H-imidazole-5-carboxylate (Compound A12)

Compound A11 (100 mg) obtained according to Step 5 and 1-methyl-4-(4,4,5,5-tetramethyl-1,3,2-dioxaborolan-2-yl)-1H-pyrazole (135 mg) were dissolved in DME (3 mL), and water (0.5 mL), potassium carbonate (90.0 mg), and [bis(diphenylphosphino)ferrocene]dichloropalladium (27.0 mg) were added to the solution, followed by stirring at 90°C for 4 hours under a nitrogen atmosphere. Water was added to the resulting reaction mixture, and extraction with ethyl acetate was performed. The organic phase was washed with a saturated brine solution, dried over anhydrous magnesium sulfate, and filtered. The solvent was distilled off from the filtrate under reduced pressure, the residue was purified by silica gel column chromatography (hexane : ethyl acetate = 80:20 to 20:80) to give the titled compound A12 (76.0 mg, yield: 89%).

Step 7

2-tert-Butyl-4-(1-methyl-1H-pyrazol-4-yl)-1H-imidazole-5-carboxylic acid (Compound A13)

Compound A12 (75.0 mg) obtained according to Step 6 was dissolved in THF (1 mL), and a 2 mol/L potassium hydroxide aqueous solution (1.0 mL) was added to the solution, followed by stirring at 60°C for 7 hours. The resulting reaction mixture was adjusted to a pH of 7 with 1 mol/L of hydrochloric acid, and the solvent was distilled off under reduced pressure. The resulting residue was purified by HP-20 resin column chromatography (water : methanol = 100:0 to 0:100) to give the titled compound A13 (70.4 mg, yield: 99%). Reference Example 4

Step 1

4-(4-Aminophenoxy)-6-methoxyquinolin-7-ol (Compound A14)

Trifluoroacetic acid (200 mL) and methanesulfonic acid (10 mL) were added to 4-(7-benzyloxy-6-methoxyquinolin-4-yloxy)aniline (20 g) obtained according to Production Example 1 of WO 03/033472, followed by stirring under reflux for 1.5 hours. The solvent was distilled off from the resulting reaction mixture under reduced pressure, and the resulting residue was adjusted to a pH of 9 with a saturated sodium bicarbonate aqueous solution. The precipitated solid was collected by filtration and was dried under reduced pressure to give the titled compound A14 (7.2 g, yield: 47%).

Step 2

Ethyl 2-[4-(4-aminophenoxy)-6-methoxyquinolin-7-yloxy]acetate (Compound A15)

Sodium hydride (106 mg) was suspended in DMF (17 mL), and Compound A14 (500 mg) obtained according to Step 1 was added thereto, followed by stirring at room temperature for 30 minutes. Bromoacetic acid ethyl ester (0.295 mL) was further added to the mixture, followed by stirring at room temperature for 1 hour. Water was added to the resulting reaction mixture, and extraction with ethyl acetate was performed. The organic phase was washed with water and saturated brine, dried over anhydrous magnesium sulfate, and filtered. The solvent was distilled off from the filtrate under reduced pressure, and the resulting residue was purified by preparative thin-layer chromatography (chloroform : methanol = 100:0 to 93:7) to give the titled compound A15 (165 mg, yield: 25%).

Reference Example 5

Step 1

4-(6-Bromopyridin-3-yloxy)-6,7-dimethoxyquinoline (Compound A16)

4-Chloro-6,7-dimethoxyquinoline (1.12 g) obtained according to J. Med. Chem., 2006, 49, 2186-2192 was dissolved in chlorobenzene (50 mL), and 6-bromopyridin-3-ol (1.74 g) and dimethylaminopyridine (1.83 g) were added thereto, followed by stirring at 100°C overnight. After the completion of the reaction, the solvent was distilled off from the resulting reaction mixture under reduced pressure. The resulting residue was purified by silica gel column chromatography using chloroform and methanol as the eluents to give the titled compound A16 (1.57 g, yield: 87%).

Step 2

5-(6,7-Dimethoxyquinolin-4-yloxy)pyridine-2-amine (Compound A17)

Lithium hexamethyldisilazide (1.0 mol/L THF solution, 6.5 mL) was added to Compound A16 (1.57 g) obtained according to Step 1, palladium dibenzylideneacetone (201 mg), and diphenyl-2-yl dicyclohexylphosphine (182 mg), followed by stirring at 65°C for 2 hours. The reaction was stopped by adding 1 mol/L hydrochloric acid (25 mL) to the resulting reaction mixture and stirring the mixture at room temperature for 5 minutes. Subsequently, a 1 mol/L sodium hydroxide aqueous solution was added to neutralize, and extraction with chloroform was performed. The organic phase was dried over anhydrous sodium sulfate and filtered. The solvent was distilled off from the filtrate under reduced pressure. The resulting residue was purified by silica gel column chromatography using chloroform and methanol as the eluents to give the titled compound A17 (1.29 g, yield: 100%).

Reference Example 6

3-Chloro-4-(6,7-dimethoxyquinolin-4-yloxy)aniline (Compound A18)

Sodium hydride (60 wt%, 0.72 g) was added to DMSO (10 mL), followed by stirring at 50°C for 20 minutes. 4-Amino-2-chlorophenol hydrochloride (1.61 g) was added to the mixture, followed by stirring at room temperature for 10 minutes. 4-Chloro-6,7-dimethoxyquinoline (1.00 g) was added to the resulting reaction mixture, followed by stirring at 100°C overnight. Water was added to the mixture, and extraction with chloroform was performed. The organic phase was washed with a saturated sodium bicarbonate aqueous solution, dried over anhydrous sodium sulfate, and filtered. The solvent was distilled off from the filtrate under reduced pressure. Methanol was added to the resulting residue, and the precipitated solid was collected by filtration to give the titled compound A18 (0.80 g, yield: 60%).

ESI-MS: $m/z$ 332 $[M+H]^+$

Reference Example 7

Step 1

Ethyl (E)-2-tert-butyl-4-styryl-1H-imidazole-5-carboxylate (Compound A19)

Ethyl 2-tert-butyl-4-iodo-1H-imidazole-5-carboxylate (200 mg), palladium(II) acetate (4.2 mg), and tri-o-toluylphosphine (11.3 mg) were dissolved in DMF (6 mL), and 4-vinylpyridine (98.0 mg) and triethylamine (94.0 mg) were further added thereto, followed by stirring under an argon atmosphere at 120°C for 3 hours. The mixture was cooled to room temperature. Water was added to the mixture, and extraction with ethyl acetate, washing with saturated brine, and drying over anhydrous sodium sulfate were performed. After filtration, the solvent was distilled off from the filtrate. The resulting residue was purified by silica gel column chromatography using hexane and ethyl acetate as the eluents to give the titled compound A19 (68.7 mg, yield: 37 %).

Step 2

(E)-4-(2-(2-tert-Butyl-5-isocyanato-1H-imidazol-4-yl)vinyl)pyridine (Compound A20)

Compound A19 (65.0 mg) obtained according to Step 1 was dissolved in THF (2 mL), and an aqueous solution obtained by dissolving sodium hydroxide (87.0 mg) in water (2 mL) was added thereto, followed by stirring at 60°C overnight. The mixture was cooled to room temperature and was adjusted to a pH of 4 with 3 mol/L of hydrochloric acid. The solvent was distilled off from the mixture under reduced pressure. The resulting residue was suspended in a solvent mixture of chloroform : methanol = 10:1 (about 20 mL), and insoluble substance was removed by filtration. The solvent was distilled off from the filtrate under reduced pressure. The resulting residue was dissolved in anhydrous DME (2.5 mL), and N,N-diisopropylethylamine (97.0 mg) and diphenylphosphoryl azide (138 mg) were added thereto, followed by stirring at 50°C for 2 hours. The mixture was cooled to room temperature, and the solvent was distilled off under reduced pressure. The resulting residue was purified by silica gel column chromatography using hexane and ethyl acetate as the eluents to give the titled compound A20 (38.3 mg, 2-step yield: 69 %).

Reference Example 8

Step 1

Ethyl 2-tert-butyl-5-(phenylethynyl)-1H-imidazole-4-carboxylate (Compound A21)

Ethyl 2-tert-butyl-4-iodo-1H-imidazole-5-carboxylate (200 mg) was dissolved in THF (6 mL), and ethynylbenzene (317 g), bis(triphenylphosphi.ne)palladium(II) dichloride (43.6 mg), and copper(I) iodide (11.8 mg) were added thereto, followed by stirring under an argon atmosphere at room temperature for 5 hours. The mixture was cooled to room temperature, and saturated brine was added to the mixture. Extraction with ethyl acetate, drying over anhydrous sodium sulfate, and filtration were performed. The solvent was distilled off from the filtrate under reduced pressure. The resulting residue was purified by silica gel column chromatography using hexane and ethyl acetate as the eluents to give the titled compound A21 (139.4 mg, yield: 76 %).

Step 2

2-tert-Butyl-5-(phenylethynyl)-1H-imidazole-4-carboxylic acid (Compound A22)

Sodium hydroxide (147 mg), 2-propanol (2 mL), and Compound A21 (109 mg) obtained according to Step 1 were added to water (4 mL), followed by stirring at 70°C for 8 hours. The mixture was cooled to room temperature, and water was added to the mixture. After washing with ethyl acetate, the aqueous phase was adjusted to a pH of 4 with 3 mol/L of hydrochloric acid and was extracted with ethyl acetate. The solvent was distilled off under reduced pressure to give the titled compound A22 (76.0 mg, yield: 77%).

Step 3

2-tert-Butyl-4-isocyanato-5-(phenylethynyl)-1H-imidazole (Compound A23)

Compound A22 (76.0 mg) obtained according to Step 2 was suspended in DME (3 mL), and N,N-diisopropylethylamine (110 mg) and diphenylphosphoryl azide (156 mg) were added thereto, followed by stirring at 50°C for 2 hours. The mixture was cooled to room temperature, and the solvent was distilled off under reduced pressure. The resulting residue was purified by silica gel column chromatography using hexane and ethyl acetate as the eluents to give the titled compound A23 (64.9 mg, yield: 86 %).

Reference Example 9

Step 1

tert-Butyl 3-oxo-3-(thiazol-5-yl)propanoate (Compound A24)

Thiazole-5-carboxylic acid (500 mg) was suspended in THF (4 mL), and carbodiimidazole (816 mg) was added thereto, followed by stirring at room temperature for 1 hour to prepare an active carbonyl intermediate. Barium oxide (890 mg) was suspended in THF (1 mL) in another reaction vessel, and tert-butyl acetate (1.84 g) was dropwise added to the suspension at room temperature, followed by stirring for 1 hour. The THF solution containing the above active carbonyl intermediate was dropwise added to the mixture, followed by stirring at room temperature for 5 hours. Methanol (2 mL) was added thereto, followed by stirring at room temperature overnight. A 10% citric acid aqueous solution was added to the reaction mixture to be a pH of 4, and insoluble substance was filtered. Extraction with chloroform, washing with a saturated sodium bicarbonate aqueous solution and brine in this order, and drying over anhydrous sodium sulfate were performed, followed by filtration. The solvent was distilled off from the filtrate under reduced pressure. The resulting residue was purified by silica gel column chromatography using hexane and ethyl acetate as the eluents and further purified by aminopropylsilica gel column chromatography using hexane and ethyl acetate as the eluents to give the titled compound A24 (338 mg, yield: 38 %).

Step 2

tert-Butyl 2-bromo-3-oxo-3-(thiazol-5-yl)propanoate (Compound A25)

Compound A24 (300 mg) obtained according to Step 1 was dissolved in THF (6 mL), and barium oxide (304 mg) was added thereto at 0°C, followed by stirring at room temperature for 30 minutes. The mixture was cooled to 0°C, and N-bromosuccinimide (247 mg) was added thereto, followed by stirring at room temperature for 1 hour. The mixture was cooled to 0°C and was adjusted to a pH of 4 with 1 mol/L of hydrochloric acid. After filtration of insoluble substance, extraction with dichloromethane, washing with a saturated sodium bicarbonate aqueous solution and a saturated brine in this order, drying over anhydrous sodium sulfate, and filtration were performed. The solvent was distilled off from the filtrate under reduced pressure to give the titled compound A25 (297 mg, yield: 74 %).

Step 3

tert-Butyl 2-tert-butyl-5-(thiazol-5-yl)-1H-imidazole-4-carboxylate (Compound A26)

tert-Butyl carbamidine hydrochloride (178 mg) was dissolved in DMF (1 mL), and sodium bicarbonate (110 mg) was added thereto, followed by stirring at 100°C for 30 minutes. The mixture was cooled to 50°C, and Compound A25 (200 mg) obtained according to Step 2 was added to the mixture, followed by stirring at 50°C for 2 hours. The mixture was cooled to room temperature, and ethyl acetate was added to the mixture. Extraction with 1 mol/L of hydrochloric acid was performed, and the aqueous phase was washed with ethyl acetate. Sodium carbonate was added to the resulting aqueous phase to be basic. Extraction with ethyl acetate, washing with saturated brine, drying over anhydrous sodium sulfate, and filtration were performed. The solvent was distilled off from the filtrate under reduced pressure. The resulting residue was purified by silica gel column chromatography using hexane and ethyl acetate as the eluents to give the titled compound A26 (33.0 mg, yield: 16 %). Reference Example 10

Step 1

2-tert-Butyl-5-(ethoxycarbonyl)-1H-imidazole-4-carboxylic acid (Compound A27)

Ethyl 2-tert-butyl-4-iodo-1H-imidazole-5-carboxylate (2.00 g, 6.21 mmol) was dissolved in DMF (25 mL), and water (6.2 mL), triethylamine (3.14 g, 31.0 mmol), [1,1'-bis(diphenylphosphino)ferrocene]dichloropalladium(II) dichloromethane complex (254 mg, 0.31 mmol), and 1,1'-bis(diphenylphosphino)ferrocene (344 mg, 0.621 mmol) were added thereto, followed by stirring under a carbon monoxide atmosphere, at 60°C for 14 hours. The mixture was cooled to room temperature, and a saturated sodium bicarbonate aqueous solution and ethyl acetate were added thereto, followed by filtration through celite. The filtrate was extracted with water, and 1 mol/L of hydrochloric acid was added to the aqueous phase to be a pH of 2. Extraction with ethyl acetate, drying over anhydrous sodium sulfate, and filtration were performed. The solvent was distilled off from the filtrate under reduced pressure to give the titled compound A27 (1.14 g, yield: 76 %).

Step 2

Ethyl 2-tert-butyl-4-(pyridin-3-ylcarbamoyl)-1H-imidazole-5-carboxylate (Compound A28)

Compound A27 (75 mg) obtained according to Step 1 was dissolved in DMF (1.5 mL), $N^1$-{(ethylimino)methylene}-$N^3$,$N^3$-dimethylpropane-1,3-diamine hydrochloride (90 mg), 1H-benzo[d][1,2,3]triazol-1-ol (72 mg), and 3-aminopyridine (44 mg) were added thereto, followed by stirring at 50°C for 14 hours. The mixture was cooled to room temperature, and water and ethyl acetate were added to the mixture. Extraction with ethyl acetate, washing with saturated brine, drying over anhydrous sodium sulfate, and filtration were performed. The solvent was distilled off from the filtrate under reduced pressure. The residue was purified by silica gel column chromatography (hexane : ethyl acetate = 80:20 to 60:40) to give the titled compound A28 (80 mg, yield: 81 %).

Step 3

2-tert-Butyl-4-(pyridin-3-ylcarbamoyl)-1H-imidazole-5-carboxylic acid (Compound A29)

Compound A28 (81 mg) obtained according to Step 2 was suspended in THF (1.3 mL) and water (1.3 mL), and potassium hydroxide (284 mg) was added thereto, followed by stirring at 60°C for 2 hours. The mixture was cooled to room temperature and was adjusted to a pH of 2 with ethyl acetate, water, and 1 mol/L of hydrochloric acid, followed by extraction with water. Water was distilled off from the mixture under reduced pressure. A solvent mixture of chloroform : methanol = 4:1 was added to the dried solid, and insoluble substance was removed by filtration, and the solvent was distilled off from the filtrate under reduced pressure to give the titled compound A29 (75 mg, yield: 100%).

Reference Example 11

Step 1

4-Chloro-3-(1-methyl-1H-pyrazol-4-yl)pyridine (Compound A30)

3-Bromo-4-chloropyridine (962 mg) was dissolved in toluene (40 mL) and ethanol (10 mL), and a tetrakis(triphenylphosphine)palladium (578 mg), 1-methyl-4-(4,4,5,5-tetramethyl-1,3,2-dioxaborolan-2-yl)-1H-pyrazole (1.2 g), and sodium carbonate (3.7 g) dissolved in water (20 mL) were added thereto, followed by heating under reflux for 4 hours. Water was added to the mixture, and extraction with ethyl acetate, washing with water and saturated brine in this order, drying over anhydrous sodium sulfate, and filtration were performed. The solvent was distilled off from the filtrate under reduced pressure. The resulting residue was purified by silica gel column chromatography (chloroform : acetone = 98:2 to 84:16) to give the titled compound A30 (443 mg, yield: 46%).

Step 2

4-{3-(1-Methyl-1H-pyrazol-4-yl)pyridin-4-yloxy}aniline (Compound A31)

Sixty percent by weight of sodium hydride (182 g) was suspended in DMSO (30 mL), and 4-aminophenol (499 mg) was added thereto, followed by stirring at room temperature for 10 minutes. Compound A30 (443 mg) obtained according to Step 1 was added to the mixture, followed by stirring at 100°C overnight. Water was added to the mixture.

Extraction with chloroform, washing with a 1 mol/L sodium hydroxide aqueous solution, water, and saturated brine, drying over anhydrous sodium sulfate, and filtration were performed. The solvent was distilled off from the filtrate under reduced pressure. The resulting residue was purified by preparative thin-layer chromatography (chloroform : acetone = 1:1) to give the titled compound A31 (223 mg, yield: 37%). Reference Example 12

Step 1

4-Chloro-N-(pyridin-4-yl)picolinamide (Compound A32)

4-Chloropicolinic acid (1.58 g) was dissolved in DMF (100 mL), and 1-(3-dimethylaminopropyl)-3-ethylcarbodiimide hydrochloride (2.88 g) and 1H-benzo[d][1,2,3]triazol-1-ol monohydrate (2.3 g) were added thereto, followed by stirring at room temperature for 30 minutes. Triethylamine (1.52 g) and pyridine-4-amine (1.41 g) were added thereto, followed by stirring at 50°C overnight. Water was added to the mixture, and extraction with ethyl acetate, washing with water and saturated brine, drying over anhydrous sodium sulfate, and filtration were performed. The solvent was distilled off from the filtrate under reduced pressure. A solvent mixture of methanol (about 5 mL) and ether (about 20 mL) was added to the resulting residue, and the precipitated solid was collected by filtration and was dried under reduced pressure to give the titled compound A32 (1.06 g, yield: 45%).

Step 2

4-(4-Aminophenoxy)-N-(pyridin-4-yl)picolinamide (Compound A33)

Sixty percent by weight of sodium hydride (368 mg) was suspended in DMSO (50 mL), 4-aminophenol (1.0 g) was added thereto, followed by stirring at room temperature for 10 minutes. Compound A32 (1.07 g) obtained according to Step 1 was added thereto, followed by stirring at 100°C overnight. Water was added to the mixture, extraction with chloroform, washing with a 1 mol/L sodium hydroxide aqueous solution, water, and saturated brine, drying over anhydrous sodium sulfate, and filtration were performed. The solvent was distilled off from the filtrate under reduced pressure. A solvent mixture of ethyl acetate (about 10 mL) and hexane (about 30 mL) was added to the resulting residue, followed by stirring. The precipitated solid was collected by filtration and was dried under reduced pressure to give the titled compound A33 (873 mg, yield: 62%).

Industrial Applicability

The present invention provides, for example, a nitrogen-containing aromatic heterocyclic derivative, a pharmaceutically acceptable salt thereof having an activity inhibiting FGFR, or the like, and being effective for treating and/or preventing a disease in which FGFR is involved (for example, gastric cancer, endometrial cancer, pituitary tumor, myeloproliferative disorder, renal cancer, bladder cancer, colon cancer, head and neck cancer, skin cancer, non-Hodgkin's lymphoma, brain tumor, breast cancer, ovarian cancer, multiple myeloma, osteosarcoma, virus infection, an autoimmune disease such as systemic lupus erythematosus, rheumatoid arthritis, hepatitis, a renal disease, or the like), or the like.

## Claims

1. A nitrogen-containing aromatic heterocyclic derivative represented by Formula (I):

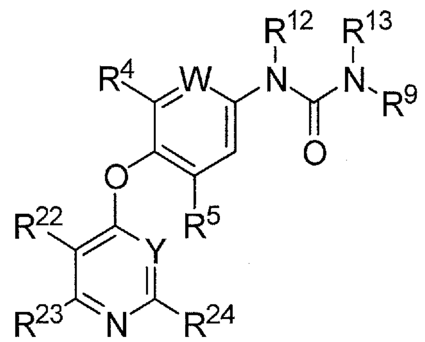

(I)

[wherein W represents $CR^3$ (wherein $R^3$ represents a hydrogen atom, lower alkyl, lower alkoxy, or halogen) or a nitrogen atom;
$R^4$ represents a hydrogen atom, lower alkyl, or halogen;
$R^5$ represents a hydrogen atom or lower alkyl;
$R^9$ represents substituted imidazolyl;
$R^{12}$ and $R^{13}$ may be the same or different and each represent a hydrogen atom or lower alkyl;
$R^{22}$ and $R^{23}$ may be the same or different and each represent a hydrogen atom, optionally substituted aryl, an optionally substituted aromatic heterocyclic group, or $-C(=O)NR^{26}R^{27}$ (wherein $R^{26}$ and $R^{27}$ may be the same or different and each represent a hydrogen atom, lower alkyl, or an aromatic heterocyclic group), or $R^{22}$ and $R^{23}$ are combined together with the respective adjacent carbon atoms to form an optionally substituted benzene ring or an optionally substituted thiophene ring;
$R^{24}$ represents a hydrogen atom or lower alkyl; and
Y represents $C-R^{25}$ (wherein $R^{25}$ represents a hydrogen atom or lower alkyl) or a nitrogen atom] or a pharmaceutically acceptable salt thereof.

**2.** The nitrogen-containing aromatic heterocyclic derivative or the pharmaceutically acceptable salt thereof according to Claim 1, wherein in Formula (I),

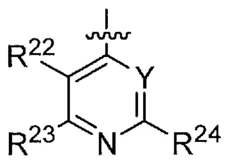

is

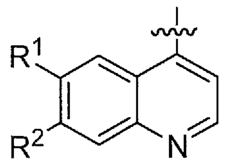

[wherein $R^1$ and $R^2$ may be the same or different and each represent a hydrogen atom, halogen, hydroxy, optionally substituted lower alkylsulfonyloxy, optionally substituted lower alkoxy, $-NR^{31}R^{32}$ (wherein $R^{31}$ and $R^{32}$ may be the same or different and each represent a hydrogen atom or optionally substituted lower alkyl), $-C(=O)NR^{33}R^{34}$ (wherein $R^{33}$ and $R^{34}$ may be the same or different and each represent a hydrogen atom or optionally substituted lower alkyl), or optionally substituted lower alkoxycarbonyl].

**3.** The nitrogen-containing aromatic heterocyclic derivative or the pharmaceutically acceptable salt thereof according to Claim 2, wherein $R^1$ and $R^2$ may be the same or different and each are a hydrogen atom, hydroxy, optionally substituted lower alkylsulfonyloxy, optionally substituted lower alkoxy, or $-NR^{31}R^{32}$ (wherein $R^{31}$ and $R^{32}$ may be the same or different and each represent a hydrogen atom or optionally substituted lower alkyl).

**4.** The nitrogen-containing aromatic heterocyclic derivative or the pharmaceutically acceptable salt thereof according to Claim 2, wherein $R^1$ and $R^2$ may be the same or different and each are optionally substituted lower alkoxy.

**5.** The nitrogen-containing aromatic heterocyclic derivative or the pharmaceutically acceptable salt thereof according to Claim 1, wherein in Formula (I),

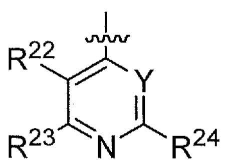

is

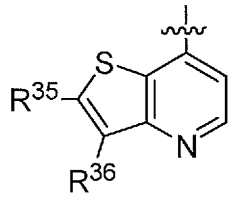

[wherein $R^{35}$ and $R^{36}$ may be the same or different and each represent a hydrogen atom, halogen, optionally substituted aryl, an optionally substituted aromatic heterocyclic group, or -(C=O)$R^{37}$ (wherein $R^{37}$ represents an optionally substituted aliphatic heterocyclic group)].

**6.** The nitrogen-containing aromatic heterocyclic derivative or the pharmaceutically acceptable salt thereof according to Claim 5, wherein $R^{35}$ is optionally substituted aryl or an optionally substituted aromatic heterocyclic group, and $R^{36}$ is a hydrogen atom.

**7.** The nitrogen-containing aromatic heterocyclic derivative or the pharmaceutically acceptable salt thereof according to any one of Claims 1 to 6, wherein W is $CR^{3A}$ (wherein $R^{3A}$ represents a hydrogen atom, lower alkoxy, or halogen) or a nitrogen atom.

**8.** The nitrogen-containing aromatic heterocyclic derivative or the pharmaceutically acceptable salt thereof according to any one of Claims 1 to 6, wherein W is $CR^{3A}$ (wherein $R^{3A}$ has the same meaning as defined above).

**9.** The nitrogen-containing aromatic heterocyclic derivative or the pharmaceutically acceptable salt thereof according to any one of Claims 1 to 8, wherein $R^4$ is a hydrogen atom or halogen.

**10.** The nitrogen-containing aromatic heterocyclic derivative or the pharmaceutically acceptable salt thereof according to any one of Claims 1 to 9, wherein $R^5$ is a hydrogen atom.

**11.** The nitrogen-containing aromatic heterocyclic derivative or the pharmaceutically acceptable salt thereof according to any one of Claims 1 to 10, wherein $R^9$ is

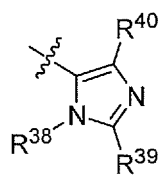

[wherein $R^{38}$ represents a hydrogen atom or lower alkyl; $R^{39}$ represents lower alkyl; and $R^{40}$ represents a hydrogen atom, optionally substituted lower alkyl, optionally substituted lower alkenyl, optionally substituted lower alkynyl, optionally substituted aryl, an optionally substituted aromatic heterocyclic group, or -C(=O)$NR^{41}R^{42}$ (wherein $R^{41}$ and $R^{42}$ may be the same or different and each represent a hydrogen atom or an aromatic heterocyclic group)].

**12.** The nitrogen-containing aromatic heterocyclic derivative or the pharmaceutically acceptable salt thereof according to Claim 11, wherein $R^{40}$ is optionally substituted aryl or an optionally substituted aromatic heterocyclic group.

**13.** The nitrogen-containing aromatic heterocyclic derivative or the pharmaceutically acceptable salt thereof according to any one of Claims 1 to 10, wherein $R^9$ is

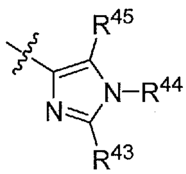

(wherein $R^{43}$ represents a hydrogen atom or lower alkyl, $R^{44}$ represents lower alkyl, and $R^{45}$ represents a hydrogen atom or lower alkyl).

**14.** The nitrogen-containing aromatic heterocyclic derivative or the pharmaceutically acceptable salt thereof according to Claim 13, wherein $R^{43}$ is a hydrogen atom, $R^{44}$ is lower alkyl, and $R^{45}$ is a hydrogen atom.

**15.** The nitrogen-containing aromatic heterocyclic derivative or the pharmaceutically acceptable salt thereof according to any one of Claims 1 to 14, wherein $R^{12}$ and $R^{13}$ are hydrogen atoms.

**16.** A pharmaceutical composition comprising, as an active ingredient, the nitrogen-containing aromatic heterocyclic derivative or the pharmaceutically acceptable salt thereof according to any one of Claims 1 to 15.

**17.** A fibroblast growth factor receptor inhibitor comprising, as an active ingredient, the nitrogen-containing aromatic heterocyclic derivative or the pharmaceutically acceptable salt thereof according to any one of Claims 1 to 15.

**18.** A therapeutic and/or preventive agent for a disease in which a fibroblast growth factor receptor is involved, comprising, as an active ingredient, the nitrogen-containing aromatic heterocyclic derivative or the pharmaceutically acceptable salt thereof according to any one of Claims 1 to 15.

**19.** An antitumor agent comprising, as an active ingredient, the nitrogen-containing aromatic heterocyclic derivative or the pharmaceutically acceptable salt thereof according to any one of Claims 1 to 15.

**20.** A therapeutic and/or preventive agent for gastric cancer comprising, as an active ingredient, the nitrogen-containing aromatic heterocyclic derivative or the pharmaceutically acceptable salt thereof according to any one of Claims 1 to 15.

**21.** A method for inhibiting a fibroblast growth factor receptor, comprising a step of administering an effective amount of the nitrogen-containing aromatic heterocyclic derivative or the pharmaceutically acceptable salt thereof according to any one of Claims 1 to 15.

**22.** A method for treating and/or preventing a disease in which a fibroblast growth factor receptor is involved, comprising a step of administering an effective amount of the nitrogen-containing aromatic heterocyclic derivative or the pharmaceutically acceptable salt thereof according to any one of Claims 1 to 15.

**23.** A method for treating and/or preventing cancer, comprising a step of administering an effective amount of the nitrogen-containing aromatic heterocyclic derivative or the pharmaceutically acceptable salt thereof according to any one of Claims 1 to 15.

**24.** A method for treating and/or preventing gastric cancer, comprising a step of administering an effective amount of the nitrogen-containing aromatic heterocyclic derivative or the pharmaceutically acceptable salt thereof according to any one of Claims 1 to 15.

**25.** Use of the nitrogen-containing aromatic heterocyclic derivative or the pharmaceutically acceptable salt thereof according to any one of Claims 1 to 15 for the manufacture of a fibroblast growth factor receptor inhibitor.

**26.** Use of the nitrogen-containing aromatic heterocyclic derivative or the pharmaceutically acceptable salt thereof according to any one of Claims 1 to 15 for the manufacture of a therapeutic and/or preventive agent for a disease in which a fibroblast growth factor receptor is involved.

**27.** Use of the nitrogen-containing aromatic heterocyclic derivative or the pharmaceutically acceptable salt thereof according to any one of Claims 1 to 15 for the manufacture of an antitumor agent.

**28.** Use of the nitrogen-containing aromatic heterocyclic derivative or the pharmaceutically acceptable salt thereof according to any one of Claims 1 to 15 for the manufacture of a therapeutic and/or preventive agent for gastric cancer.

**29.** The nitrogen-containing aromatic heterocyclic derivative or the pharmaceutically acceptable salt thereof according to any one of Claims 1 to 15 for use in inhibiting a fibroblast growth factor receptor.

**30.** The nitrogen-containing aromatic heterocyclic derivative or the pharmaceutically acceptable salt thereof according to any one of Claims 1 to 15 for use in treating and/or preventing a disease in which a fibroblast growth factor receptor is involved.

**31.** The nitrogen-containing aromatic heterocyclic derivative or the pharmaceutically acceptable salt thereof according to any one of Claims 1 to 15 for use in treating and/or preventing cancer.

**32.** The nitrogen-containing aromatic heterocyclic derivative or the pharmaceutically acceptable salt thereof according to any one of Claims 1 to 15 for use in treating and/or preventing gastric cancer.

**INTERNATIONAL SEARCH REPORT**

International application No.
PCT/JP2011/066185

A. CLASSIFICATION OF SUBJECT MATTER
*C07D401/12*(2006.01)i, *A61K31/4709*(2006.01)i, *A61P35/00*(2006.01)i, *A61P43/00*(2006.01)i, *C07D401/14*(2006.01)i, *C07D413/14*(2006.01)i, *C07D417/14*(2006.01)i, *C07D495/04*(2006.01)i

According to International Patent Classification (IPC) or to both national classification and IPC

B. FIELDS SEARCHED

Minimum documentation searched (classification system followed by classification symbols)
C07D401/12, A61K31/4709, A61P35/00, A61P43/00, C07D401/14, C07D413/14, C07D417/14, C07D495/04

Documentation searched other than minimum documentation to the extent that such documents are included in the fields searched

| | | | |
|---|---|---|---|
| Jitsuyo Shinan Koho | 1922-1996 | Jitsuyo Shinan Toroku Koho | 1996-2011 |
| Kokai Jitsuyo Shinan Koho | 1971-2011 | Toroku Jitsuyo Shinan Koho | 1994-2011 |

Electronic data base consulted during the international search (name of data base and, where practicable, search terms used)
CAplus(STN), REGISTRY(STN)

C. DOCUMENTS CONSIDERED TO BE RELEVANT

| Category* | Citation of document, with indication, where appropriate, of the relevant passages | Relevant to claim No. |
|---|---|---|
| X | JP 2010-506850 A (Deciphera Pharmaceuticals, L.L.C.), 04 March 2010 (04.03.2010), claims 1, 51, 52; page 119, lines 31 to 33; page 120, lines 7 to 10; page 120, lines 28 to 30 & US 2008/0090856 A1 & EP 2073811 A & WO 2008/046003 A2 & CA 2666563 A & CN 101553233 A & IL 197933 D & KR 10-2009-0080517 A | 1,16,19,27, 29-32 |
| X<br>Y | WO 2003/033472 A1 (Kirin Brewery Co., Ltd.), 24 April 2003 (24.04.2003), compound 377; claims 1, 15 to 17; page 5, line 27 to page 6, line 8 & JP 4383870 B & EP 1447405 A1 | 1-4,7-20, 25-32<br>1,5-20,25-32 |

☒ Further documents are listed in the continuation of Box C. ☐ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| 17 August, 2011 (17.08.11) | 30 August, 2011 (30.08.11) |

| Name and mailing address of the ISA/ | Authorized officer |
|---|---|
| Japanese Patent Office | |
| Facsimile No. | Telephone No. |

Form PCT/ISA/210 (second sheet) (July 2009)

**INTERNATIONAL SEARCH REPORT**

International application No.
PCT/JP2011/066185

C (Continuation). DOCUMENTS CONSIDERED TO BE RELEVANT

| Category* | Citation of document, with indication, where appropriate, of the relevant passages | Relevant to claim No. |
|---|---|---|
| X<br><br>Y | WO 2008/093855 A1 (Eisai R & D Management Co., Ltd.),<br>07 August 2008 (07.08.2008),<br>claims 1, 8, 10; page 125, lines 1 to 6; page 133, lines 4 to 10; page 134, line 10 to page 135, line 4; page 120, lines 14 to 17<br>& EP 2119707 A1 & WO 2008/093855 A1<br>& CA 2676796 A & CN 101600694 A<br>& IL 200090 D | 1-4,7-20,<br>25-32<br>1,5-20,25-32 |
| Y | WO 2006/104161 A1 (Kirin Brewery Co., Ltd.),<br>05 October 2006 (05.10.2006),<br>claims 1, 25, 57 to 59; paragraph [0001]; compound of examples<br>& US 2009/0270391 A1 & EP 1870414 A1<br>& CA 2603125 A & KR 10-2008-0007443 A<br>& CN 101189241 A | 1,5-20,25-32 |
| Y | JP 2008-539275 A (Amgen Inc.),<br>13 November 2008 (13.11.2008),<br>claims 1, 2, 52, 53; examples 131 to 153<br>& US 2008/0312232 A1 & EP 1881976 A<br>& WO 2006/116713 A1 & UY 29503 A<br>& CA 2605680 A & KR 10-2008-0004617 A<br>& NO 20076093 A & AR 54262 A<br>& CN 101248059 A & GT 200600181 A<br>& ZA 200708775 A & EA 200702339 A<br>& BRA PI0608097 & IL 186526 D<br>& PE 14362006 A & NZ 562595 A | 1,5-20,25-32 |

Form PCT/ISA/210 (continuation of second sheet) (July 2009)

**INTERNATIONAL SEARCH REPORT**

International application No.
PCT/JP2011/066185

**Box No. II Observations where certain claims were found unsearchable (Continuation of item 2 of first sheet)**

This international search report has not been established in respect of certain claims under Article 17(2)(a) for the following reasons:

1. [x] Claims Nos.: 21-24
because they relate to subject matter not required to be searched by this Authority, namely:
Claims 21 to 24 involve "methods for treatment of the human body or animal body by surgery or therapy" and thus relate to a subject matter which this International Searching Authority is not required, under the provisions of PCT Article 17(2)(a)(i) and PCT Rule 39.1(iv), to search.

2. [ ] Claims Nos.:
because they relate to parts of the international application that do not comply with the prescribed requirements to such an extent that no meaningful international search can be carried out, specifically:

3. [ ] Claims Nos.:
because they are dependent claims and are not drafted in accordance with the second and third sentences of Rule 6.4(a).

**Box No. III Observations where unity of invention is lacking (Continuation of item 3 of first sheet)**

This International Searching Authority found multiple inventions in this international application, as follows:

1. [ ] As all required additional search fees were timely paid by the applicant, this international search report covers all searchable claims.
2. [ ] As all searchable claims could be searched without effort justifying additional fees, this Authority did not invite payment of additional fees.
3. [ ] As only some of the required additional search fees were timely paid by the applicant, this international search report covers only those claims for which fees were paid, specifically claims Nos.:

4. [ ] No required additional search fees were timely paid by the applicant. Consequently, this international search report is restricted to the invention first mentioned in the claims; it is covered by claims Nos.:

**Remark on Protest**

[ ] The additional search fees were accompanied by the applicant's protest and, where applicable, the payment of a protest fee.

[ ] The additional search fees were accompanied by the applicant's protest but the applicable protest fee was not paid within the time limit specified in the invitation.

[ ] No protest accompanied the payment of additional search fees.

Form PCT/ISA/210 (continuation of first sheet (2)) (July 2009)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 9717329 A
- WO 0043366 A
- WO 02088110 A
- US 7329670 B
- WO 9932106 A
- WO 9932455 A
- WO 0232872 A
- WO 2008026748 A
- WO 2007061127 A
- WO 97017329 A
- JP 11158149 A
- WO 9924440 A
- WO 0041698 A
- WO 0042012 A
- WO 0147890 A
- WO 03000194 A
- WO 03000660 A
- WO 03033472 A
- WO 2004018430 A
- WO 2004039782 A
- WO 2006010264 A
- US 5773449 A
- US 20080312232 A
- WO 2005121125 A
- US 20100048545 A
- WO 1999032106 A
- WO 2009125597 A
- US 20080090856 A
- US 20070004675 A
- WO 200610264 A


### Non-patent literature cited in the description


- *Nature Reviews Cancer,* 2010, vol. 10, 116-129
- *Cancer Res.,* 1997, vol. 57, 4360-4367
- *Clin. Cancer Res.,* 2007, vol. 13, 3051-3057
- *Proc. Natl. Acad. Sci. USA,* 2008, vol. 105, 8713-8717
- *Nature Genetics,* 1999, vol. 23, 18-19
- *Cancer Res.,* 2002, vol. 62, 840-847
- *Expert Opinion on Therapeutic Targets,* 2002, vol. 6, 469-482
- *Nature,* 2001, vol. 411, 355-365
- *Journal of Hepatology,* 2009, vol. 50, 118-127
- *The Journal of Biological Chemistry,* 1998, vol. 273 (50), 33533-33539
- *Protein, Nucleic acid and Enzyme,* 1993, vol. 38 (2), 102-108
- *Protein, Nucleic acid and Enzyme,* 1999, vol. 44 (10), 1477-1486
- #36 Azolyl Quinoline-Urea Derivatives: A Novel Series of Potent Orally Active VEGF Receptor Tyrosine Kinase Inhibitors. American Association for Cancer Research (AACR, 11 July 2003
- **T.W. GREENE.** Protective Groups in Organic Synthesis. John Wiley & Sons Inc, 1999
- *J. Med. Chem.,* 2005, vol. 48 (5), 1359-1366
- *J. Med. Chem.,* 2006, vol. 49 (8), 2440-2455
- Jikken Kagaku Koza. Yuki Gosei. Maruzen, 2001, vol. 20, 473
- *Synth. Commun.,* 1993, vol. 23 (3), 335
- Jikken Kagaku Koza. Synthesis of carboxylic acid. Maruzen, vol. 16, 1-34
- *J. Heterocyclic Chem.,* 1989, vol. 26 (5), 1389-1392
- *J. Org. Chem.,* 1977, vol. 42 (7), 1153-1159
- *Heterocycles,* 2006, vol. 68 (6), 1149-1162
- *Tetrahedron Lett.,* 1994, vol. 35 (11), 1635-1638
- *Journal of Medicinal Chemistry,* 2005, vol. 48 (5), 1359-1366
- *Eur. J. Med. Chem.,* 1986, vol. 21, 5-8
- *J. Med. Chem.,* 2006, vol. 49, 2186-2192